(19) Europäisches Patentamt
European Patent Office
Office européen des brevets

(11) EP 0 634 386 B1

(12) # EUROPÄISCHE PATENTSCHRIFT

(45) Veröffentlichungstag und Bekanntmachung des Hinweises auf die Patenterteilung:
**28.05.1997 Patentblatt 1997/22**

(51) Int. Cl.[6]: **C07C 68/00**, C07C 69/96

(21) Anmeldenummer: **94110391.3**

(22) Anmeldetag: **04.07.1994**

(54) **Verfahren zur Herstellung von Dimethylcarbonat**
Process for the preparation of dimethyle carbonate
Procédé de préparation du carbonate de diméthyle

(84) Benannte Vertragsstaaten:
**BE DE ES FR GB IT NL**

(30) Priorität: **15.07.1993 DE 4323682**
**15.07.1993 DE 4323675**

(43) Veröffentlichungstag der Anmeldung:
**18.01.1995 Patentblatt 1995/03**

(73) Patentinhaber: **BAYER AG**
**51368 Leverkusen (DE)**

(72) Erfinder:
- **Landscheidt, Heinz, Dr.**
**D-47057 Duisburg (DE)**
- **Wolters, Erich, Dr.**
**D-50939 Köln (DE)**
- **Wagner, Paul, Dr.**
**D-40597 Düsseldorf (DE)**
- **Klausener, Alexander, Dr.**
**D-50670 Köln (DE)**

(56) Entgegenhaltungen:
**EP-A- 0 523 508** **EP-A- 0 523 728**
- **DATABASE WPI Week 9422, Derwent Publications Ltd., London, GB; AN 94-183383 & WO-A-94 11335**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 634 386 B1

Printed by Rank Xerox (UK) Business Services
2.14.7/3.4

## Beschreibung

Gegenstand der Erfindung ist ein neues Verfahren zur kontinuierlichen Herstellung von Dimethylcarbonat (DMC), dadurch gekennzeichnet, daß man Kohlenmonoxid und Methylnitrit in der Gasphase in Gegenwart eines heterogenen Katalysators miteinander zur Reaktion bringt und das dabei gebildete Dimethylcarbonat in nachgeschalteten Verfahrensschritten isoliert. Das erfindungsgemäße Verfahren eignet sich insbesondere zur technischen Herstellung von Dimethylcarbonat.

Dimethylcarbonat ist ein wichtiges Ausgangsmaterial für die Herstellung aromatischer Polycarbonate. Es findet ferner Verwendung als Ausgangsmaterial für die Synthese aliphatischer und aromatischer Mono- und Diisocyanate, als Methylierungsmittel, als Ersatz für das giftige Phosgen bei der Herstellung pharmazeutischer und agrochemischer Produkte, als Lösungsmittel, Octanzahlverbesserer für Ottokraftstoffe und als Zwischenprodukt bei der Herstellung synthetischer Schmierstoffe.

Die Reaktion zwischen Kohlenmonoxid und Methylnitrit, die der Bildung von Dimethylcarbonat zugrundeliegt, läßt sich durch Reaktionsgleichung (1) beschreiben.

$$(1) \qquad CO + 2\ CH_3ONO \xrightarrow[\text{[Kat.]}]{} H_3CO\text{-}CO\text{-}OCH_3 + 2\ NO$$

Methylnitrit selbst kann dazu auf an sich bekannte Weise in einer vorgeschalteten Reaktion gemäß einer der Reaktionsgleichungen (2) bis (5) erzeugt werden.

$$4\ NO + O_2 + 4\ CH_3OH \rightarrow 4\ CH_3ONO + 2\ H_2O \qquad (2)$$

$$NO + NO_2 + 2\ CH_3OH \rightarrow 2\ CH_3ONO + H_2O \qquad (3)$$

$$N_2O_4 + CH_3OH \rightarrow CH_3ONO + HNO_3 \qquad (4)$$

$$2\ NaNO_2 + H_2SO_4 + 2\ CH_3OH \rightarrow 2\ CH_3ONO + Na_2SO_4 + 2\ H_2O \qquad (5)$$

Die Herstellung von Dimethylcarbonat durch Umsetzung von Kohlenmonoxid und Methylnitrit in der Gasphase in Gegenwart eines heterogenen Katalysators, bei dem es sich bevorzugt um einen trägerfixierten Platinmetall-Katalysator handelt, ist verschiedentlich beschrieben worden, beispielsweise in folgenden wissenschaftlichen Publikationen, Offenlegungsschriften und Patentschriften:
JP 60 181 051, X.-Z. Jiang et al.; Cuihua Xuebao 10(1) 75-78 (März 1989), EP 425 197, X.-Z. Jiang; Platinum Metals Rev. 34(4), 178-180 (1990), EP 464 460, EP 503 091, EP 501 507, EP 503 618, EP 523 508, EP 523 728 und EP 538 676, EP 559 001, EP 558 996, EP 559 212, EP 565 076 und EP 581 240.

Bis auf die genannte Europäische Patentanmeldung EP 523 728 lehrt keine der aufgeführten Publikationen einen Weg, der für die kontinuierliche technische Herstellung von Dimethylcarbonat geeignet wäre. So werden im allgemeinen als Reaktionsprodukte Gemische erhalten, in denen außer der gewünschten Zielverbindung Dimethylcarbonat weitere Stoffe vorliegen, wie beispielsweise Oxalsäuredimethylester, das im Zuge einer nach Reaktionsgleichung (6) ablaufenden Nebenreaktion entsteht, Ameisensäuremethylester, Formaldehyddimethylacetal, Wasser und insbesondere Methanol. Für viele der möglichen Verwendungszwecke des Dimethylcarbonats sind derartige Gemische aber völlig ungeeignet.

$$(6) \qquad 2\ CO + 2\ CH_3ONO \xrightarrow[\text{[Kat.]}]{} H_3COOC\text{-}COOCH_3 + 2\ NO$$

Eine unter industriellen Gesichtspunkten anzustrebende kontinuierliche Prozeßführung muß grundsätzlich dem in Fig. 1 wiedergegebenen Prinzip und somit einem Kreisprozeß entsprechen. Sie muß so angelegt sein, daß die im Zuge der nach Reaktionsgleichung (1) erfolgten Bildung des Dimethylcarbonats anfallenden Stickoxide sowie alle weiteren gasförmigen und kondensierten Produkte, Nebenprodukte und Hilfsstoffe entweder, soweit verwendbar oder die Ökonomie bzw. die technische Sicherheit des Gesamtverfahrens nicht negativ beeinträchtigend, vollständig oder zum größten Teil in den Prozeß zurückgeführt werden, oder, soweit diese Komponenten die Ökonomie bzw. die technische

Sicherheit des Gesamtverfahrens negativ beeinträchtigen, vollständig oder zum für den ungestörten kontinuierlichen Betrieb des Gesamtverfahrens erforderlichen Teil ausgeschleust werden.

In EP 523 728 wird ein Verfahren beschrieben, dessen Prinzip in Fig. 2 wiedergegeben ist und das die kontinuierliche Herstellung von Dimethylcarbonat durch Umsetzung von Methylnitrit mit Kohlenmonoxid in der Gasphase an einem heterogenen Katalysator, bei dem es sich vorzugsweise um einen trägerfixierten Platinmetallkontakt handelt, sowie die nachfolgende Isolierung des als Gemisch mit Methanol, Oxalsäuredimethylester und weiteren Verunreinigungen anfallenden Dimethylcarbonats im Zuge einer Extraktivdestillation, wobei als Extraktionsmittel Oxalsäuredimethylester verwendet wird, beinhaltet. Weiterhin beinhaltet EP 523 728 die Rückführung der im Zuge der Umsetzung von Methylnitrit mit Kohlenmonoxid freigesetzten Stickoxide samt den nicht umgesetzten gasförmigen Reaktionspartnern und dem zur Inertisierung erforderlichen zusätzlichen Gas, bevorzugt Stickstoff in einen dem eigentlichen Herstellungsprozeß des Dimethylcarbonats vorgeschalteten Verfahrensschritt, der der Reaktionsgleichung (2) entspricht und in dem durch Zuspeisung von Methanol und Sauerstoff und unter weitestgehender Entfernung des dabei freiwerdenden Wassers das für die Reaktion benötigte Methylnitrit erneut gebildet wird. Somit handelt es sich, bezogen auf die beteiligten gasförmigen Komponenten, namentlich bezüglich der Inertgase und Hilfsstoffe, der nicht umgesetzten gasförmigen Reaktanden, wie beispielsweise des nicht umgesetzten Methylnitrits und Kohlenmonoxids, sowie der beteiligten Stickoxide um einen Kreisprozeß.

Nachteilig bei dem in EP 523 728 beschriebenen Verfahren sind folgende Punkte, die seine industrielle Anwendbarkeit in ökonomischer und ökologischer Hinsicht in Frage stellen:

Zur Herstellung, Isolierung und Reinigung des Dimethylcarbonats müssen große Mengen von Hilfsstoffen im Kreis gefahren werden. Legt man beispielsweise die Angaben des Beispiels 1 der genannten Patentanmeldung zugrunde, so ergeben sich pro kg DMC folgende im Kreis zu führende Mengen:

| | |
|---|---|
| 4,8 kg | Methanol, |
| 6,0 kg | Dimethyloxalat, |
| 2,8 kg | Methylnitrit, |
| 1,6 kg | Kohlenmonoxid, |
| 1,2 kg | Stickstoffmonoxid und |
| 7,8 kg | Stickstoff |

Insbesondere wird durch den großen Überschuß an Methanol, der in den Methylnitrit-Reaktor 3 der Fig. 2 eingespeist wird und der ca. 500 % der stöchiometrisch benötigten Menge beträgt, ein ganz erheblicher Destillationsaufwand erforderlich, der zu hohen Energiekosten führt, wenn man den nicht umgesetzten Anteil Methanol aus dem Bodenablauf des Reaktors wiedergewinnen will.

Da die Rückführung dieser Hilfsstoffe bzw. Nebenkomponenten (Wasser, Salpetersäure, Ameisensäuremethylester, Formaldehyddimethylacetal), vor allem bezüglich Methanol und Dimethyloxalat, eine destillative Abtrennung vom gewünschten Reaktionsprodukt Dimethylcarbonat erfordert, ist dieses Verfahren äußerst energieaufwendig und daher nicht nur unter ökonomischen, sondern auch unter ökologischen Aspekten unattraktiv.

Grundsätzlich bildet Oxalsäuredimethylester durch Reaktion mit in Spuren vorhandenem Wasser, das der Herstellung des Methylnitrits im Methylnitritreaktor 3 der Fig. 2 entstammt und das aufgrund der dort niemals vollständig gelingenden Abtrennung typischerweise in Reaktandgasgemischen und somit auch in Produktgasgemischen der Dimethylcarbonat-Herstellung enthalten ist, gemäß den Reaktionsgleichungen (7) und (8) Oxalsäurehalbester oder Oxalsäure. Diese können aufgrund ihrer Acidität in der Extraktionskolonne 2 und insbesondere aufgrund der höheren Temperatur in der Methanolkolonne 4 der Fig. 2 das dort vorhandene Methanol gemäß Reaktionsgleichung (9) zu Dimethylether umsetzen. Dieser Prozeß ist autokatalytisch, da mit jedem Reaktionsereignis ein weiteres Äquivalent Wasser freigesetzt wird, das seinerseits wieder mit Oxalsäuredimethylester reagieren kann.

$$H_3COOC\text{-}COOCH_3 + H_2O \rightarrow H_3COOC\text{-}COOH + CH_3OH \quad (7)$$

$$H_3COOC\text{-}COOH + H_2O \rightarrow HOOC\text{-}COOH + CH_3OH \quad (8)$$

$$(9) \quad 2\ CH_3OH \xrightarrow[{[H^+]}]{} H_3C\text{-}O\text{-}CH_3 + H_2O$$

Außerdem kann der gemäß Reaktionsgleichung (7) gebildete Halbester der Oxalsäure unter Bildung von Ameisensäuremethylester gemäß Reaktionsgleichung (10) decarboxylieren.

$$H_3COOC\text{-}COOH \rightarrow HCOOCH_3 + CO_2 \qquad (10)$$

Im übrigen enthält technisch verfügbares Kohlenmonoxid auch nach aufwendiger Reinigung geringe Mengen unter den Bedingungen der Dimethylcarbonat-Herstellung inerter gasförmiger Verunreinigungen, wie beispielsweise Wasserstoff, Methan und Kohlendioxid.

Das unweigerliche Akkumulieren flüchtiger Nebenkomponenten im zurückgeführten Kreisgas, mag es sich nun um solche handeln, die im Verlaufe unerwünschter Nebenreaktionen gebildet werden, oder um solche, die als Verunreinigungen in den zum Einsatz kommenden Rohstoffen enthalten sind, erfordert die Ausschleusung eines entsprechenden Anteils des zirkulierenden Kreisgases (purge). Diese wird zwar grundsätzlich in der Verfahrensbeschreibung der genannten Patentanmeldung EP 523 728 erwähnt, jedoch werden über Umfang und Behandlung der ausgeschleusten Gasmengen keine Angaben gemacht. In jedem Fall ist zu erwarten, daß sowohl Ökonomie als auch Ökologie des Verfahrens hierdurch beeinträchtigt werden.

Über diese Betrachtungen hinaus ist die Beschreibung des Verfahrens beispielsweise an den folgenden Stellen inkonsistent oder fehlerhaft:

So wird in Spalte 11, Zeilen 40 bis 42 der Patentanmeldung EP 523 728, ein aus 6 Rohren bestehender Rohrbündelreaktor, dessen Rohre einen Durchmesser von jeweils 26,1 mm und eine Länge von jeweils 500 mm besitzen, beschrieben. Ein solcher Reaktor hat ein maximales Volumen von 1,6 l. Nach Zeile 43 der Patentanmeldung EP 523 728 wird dieser Reaktor aber mit 1,73 l Katalysator befüllt.

Nach Spalte 12, Zeile 15 der Patentanmeldung EP 523 728 werden am Boden der Dimethylcarbonat-Extraktionskolonne (vgl. Ziffer 2 in Fig. 2 hierin, entsprechend Ziffer 2 in Fig. 1 der Patentanmeldung EP 523 728) 2,8 kg/h einer Absorptionslösung entnommen und der Destillationskolonne (vgl. Ziffer 4 in Fig. 2 hierin, entsprechend Ziffer 4 in Fig. 1 der Patentanmeldung EP 523 728) zugeführt. Nach Spalte 12, Zeilen 51 bis 52 der Patentanmeldung EP 523 728 sind dies jedoch 3,5 kg/h.

Nach Spalte 13, Zeilen 2 bis 4 der Patentanmeldung EP 523 728 entnimmt man dem Sumpf der ersten Destillationskolonne (Methanol-Destillation, vgl. Ziffer 4 in Fig. 2 hierin, entsprechend Ziffer 4 in Fig. 1 der Patentanmeldung EP 523 728) ein Gemisch, das zu 14,3 % aus Dimethylcarbonat und zu 87,5 % aus Dimethyloxalat besteht. Dies ist jedoch rein rechnerisch nicht möglich.

Nach Spalte 13, Zeile 21 der Patentanmeldung EP 523 728 werden dem Sumpf der zweiten Destillationskolonne (Dimethylcarbonat-Destillation, vgl. Ziffer 5 in Fig. 2 hierin, entsprechend Ziffer 5 in Fig. 1 der Patentanmeldung EP 523 728) 4,69 kg/h Dimethyloxalat entnommen. Diese Menge ist aber um mindestens 0,6 kg größer, als sie nach den Angaben in Spalte 12, Zeilen 15 bis 16 oder in Spalte 12, Zeilen 51 und 55 der Patentanmeldung EP 523 728 sein kann.

Der Methanolanteil des Gases, das den Methylnitrit-Synthesereaktor (Ziffer 3 in Fig. 2 hierin, entsprechend Ziffer 3 in Fig. 1 der Patentanmeldung EP 523 728) verläßt und das nach Beimischung von Kohlenmonoxid in den Dimethylcarbonat-Synthesereaktor eingespeist wird (Ziffer 1 in Fig. 2 hierin, entsprechend Ziffer 1 in Fig. 1 der Patentanmeldung EP 523 728), wird bei vorgegebenem Druck und vorgegebenen Anteilen der übrigen im Gesamtgasgemisch vorhandenen gasförmigen Komponenten durch die Austrittstemperatur des am Kopf des Methylnitrit-Synthesereaktors befindlichen Kondensators bestimmt. Er stellt keine frei wählbare Größe dar, sondern entspricht dem sich unter diesen Bedingungen einstellenden Partialdampfdruck und beträgt für Beispiel 1 der genannten Patentanmeldung EP 523 728 zwischen 5,5 und 5,8 Vol.-%. Genannt werden in Beispiel 1, Spalte 11, Zeilen 51 bis 52 der besagten Patentanmeldung jedoch 1,8 Vol.-%.

Schließlich entsprechen die Angaben für die gebildete bzw. ausgeschleuste Menge des bei der Herstellung von Methylnitrit anfallenden Wassers (0,07 kg/h) nicht derjenigen Menge, die bei Zugrundelegung der in Beispiel 1 der genannten EP 523 728 beschriebenen Reaktionsausbeuten an Dimethylcarbonat und Oxalsäuredimethylester zu erwarten wäre, nämlich 0,14 kg/h.

Es bestand somit die Aufgabe, ein Verfahren zu finden, daß durch einen niedrigeren Rohstoff- und Energieaufwand, durch einen geringeren Anfall an Nebenprodukten sowie durch eine effektivere und möglichst einfachere Isolierung und Reinigung des gewünschten Dimethylcarbonats gekennzeichnet ist, als dies durch den Stand der Technik beschrieben wird. Diese Aufgabe wird durch das erfindungsgemäße Verfahren gelöst.

Für die gemäß Reaktionsgleichung (1) durchgeführte Herstellung von Dimethylcarbonat durch Umsetzung von Kohlenmonoxid mit Methylnitrit an heterogenen Platinmetall-Trägerkontakten in der Gasphase werden in der Literatur verschiedene Katalysatoren bzw. Katalysatortypen beschrieben.

So lassen sich etwa der wissenschaftlichen Publikation, Cuihua Xuebao 10(1), S. 75 bis 78 (März 1989) zufolge als Katalysatoren beispielsweise Palladium(II)-halogenide, bevorzugt Palladium(II)-chlorid und insbesondere auf Aktivkohle-Trägern fixiertes Palladium(II)-chlorid, das mit Eisen-, Lithium- und/oder Kupfer-Verbindungen dotiert bzw. modifiziert ist, verwenden, wobei hohe Selektivitäten und Raum-Zeit-Ausbeuten an gewünschtem Dimethylcarbonat erhalten. Ähnliche Katalysatorsysteme werden in den Patentanmeldungen EP 425 197, EP 464 460, EP 503 091, EP 503 618 und EP 523 728 beschrieben. Katalysatoren dieses Typs liefern im allgemeinen das gewünschte Dimethylcarbonat in einer nicht völlig zufriedenstellenden Selektivität. Als Nebenprodukt entsteht nach Reaktionsgleichung (6) der unerwünschte Oxalsäuredimethylester. Dies ist einerseits der anzustrebenden möglichst hohen Ausnutzung der eingesetzten Rohstoffe abträglich und erfordert andererseits einen zusätzlichen Trennaufwand im Zuge der Isolierung und Reinigung des gewünschten Dimethylcarbonats. Weiterhin unterliegen viele Katalysatoren des genannten Typs im Verlaufe längerer Betriebszeiten einem Austrag an Halogenid-, speziell an Chloridionen, im allgemeinen in Form von Halogenwasserstoff-, speziell Chlorwasserstoffbildung. Dies ist gegebenenfalls mit einer Abnahme der Selektivität bezüglich der Dimethylcarbonat-Bildung und einem Abfall der Katalysatoraktivität verbunden, was sich jedoch durch Zusatz schon geringer Mengen z.B. von Halogenwasserstoff, speziell von Chlorwasserstoff, zum Reaktandgasgemisch, wie er etwa in der genannten Patentanmeldung EP 425 197 beschrieben wird, vermeiden läßt. Durch die Einbringung der erwähnten geringen Mengen Halogenwasserstoff, speziell Chlorwasserstoff, ergeben sich erhöhte Anforderungen an die Werkstoffe, aus denen die berührten Anlagenteile zu fertigen sind. Ferner zu berücksichtigen ist die Bildung charakteristischer Nebenprodukte wie beispielsweise Methylchlorid, das gemäß Reaktionsgleichung (11) durch die parallel zur Konditionierung des Katalysators ablaufende Umsetzung von Chlorwasserstoff mit im Reaktandgasgemisch stets zu geringen Anteilen vorhandenen Methanol entsteht. In der Patentanmeldung EP 565 076 wird beispielhaft belegt, welch starken Desaktivierungserscheinungen (nur 50 bis 500 h bis zum nahezu vollständigen Verschwinden der Aktivität) auf Aktivkohle-Trägern basierende Katalysatoren unterliegen. Dort wird ein Verfahren zur absatzweisen Regenerierung dermaßen desaktivierter Kontakte beschrieben, das eine sequentielle Behandlung dieser Katalysatoren mit Wasserstoff und Halogenwasserstoff bei erhöhten Temperaturen beinhaltet. Ein derartiges Verfahren würde bei seiner technischen Realisierung ein beständiges An- und Abfahren der Produktionsanlage im Abstand von günstigstenfalls einigen hundert Betriebsstunden erfordern, um Regenerierungskampagnen zu ermöglichen. Alternativ könnte man sich auch eine doppelte Reaktorausführung vorstellen, die im Wechsel zwischen Produktions-und Regenerierungszyklen betrieben wird. Eine weitere Möglichkeit bestünde darin, absatzweise oder kontinuierlich Teilmengen des Katalysators aus der Reaktionszone zu entnehmen, extern zu regenerieren und dem Reaktor wieder zuzuführen. In jedem Fall würde die technische Lösung des Desaktivierungsproblems hohe zusätzliche Kosten verursachen und wäre unter wirtschaftlichen Gesichtspunkten höchst ungünstig. So kann es vorteilhaft sein, auf Katalysatoren zurückzugreifen, die keine oder nur eine vertretbar geringere Desaktivierung aufgrund des erwähnten Austrags von Halogenid-, speziell von Chloridionen, aufweisen, auch wenn derartige Kontakte, wie sie beispielsweise in den Patentanmeldungen EP 503 091, EP 503 618 und EP 523 728 beschrieben werden, eine eher ungünstigere Selektivität, bedingt durch Bildung von Oxalsäuredimethylester, zeigen.

$$CH_3OH + HCl \rightarrow CH_3Cl + H_2O \qquad (11)$$

Um das Akkumulieren von Nebenprodukten innerhalb eines technischen Kreisprozesses zu verhindern, müssen festzulegende Anteile des zirkulierenden Kreisgases und der kondensierten Reaktionsprodukte, soweit es sich dabei nicht um Dimethylcarbonat selbst handelt, kontinuierlich oder diskontinuierlich, vorzugsweise kontinuierlich ausgeschleust werden (purge).

Mit der Auffindung neuer Katalysatoren, die die Herstellung von Dimethylcarbonat durch die heterogen katalysierte Umsetzung von Methylntrit und Kohlenmonoxid in der Gasphase bei erheblich gesteigerten Selektivitäten ermöglichen, ergeben sich für die technische Realisierung neue Perspektiven. Kontakte dieses Typs, wie sie beispielsweise in den Patentanmeldungen EP 523 508, EP 538 676, EP 559 001, EP 558 996 und EP 581 240 beschrieben werden, ließen nur sehr wenig Oxalsäuredimethylester als Nebenprodukt. Beispielsweise durch kontinuierliche Zugabe geringster Mengen Chlorwasserstoff zum Reaktandgasgemisch lassen sich ihre Aktivität und ihre DMC-Selektivität für lange Zeit auf praktisch unverändert hohem Niveau halten.

Die Erfindung betrifft ein Verfahren zur kontinuierlichen Herstellung von Dimethylcarbonat aus Kohlenmonoxid und Methylnitrit und Recyclisieren des dabei entstehenden Stickoxids zur erneuten Bildung von Methylnitrit, das dadurch gekennzeichnet ist, daß man

(a) Kohlenmonoxid und Methylnitrit in der Gasphase in Gegenwart eines heterogenen, ein Platinmetall enthaltenden Katalysators, bevorzugt eines Palladium enthaltenden Trägerkatalysators, und eines Inertgases im Temperaturbereich von 50 bis 170°C, vorzugsweise von 70 bis 150°C, und im Druckbereich von 1 bis 5 bar, vorzugsweise 1,5-4 bar zur Reaktion bringt,wobei dem Gasgemisch als Aktivator Halogenwasserstoff, Halogen, Chlorameisensäuremethylester und/oder andere unter den Reaktionsbedingungen aktivierend wirkendes Halogen enthaltende Substanzen in einer Konzentration von 0-3000 ppm, bevorzugt 10-1000 ppm zugesetzt wird (werden),

(b) das bei (a) anfallende Gemisch in gasförmige und flüssige Reaktionsprodukte trennt, einen Teil von 0-7 Gew.-%, bevorzugt 0,1-5 Gew.-% des Gasstromes ausspeist, die darin enthaltenen Leichtsieder abtrennt und gegebenenfalls einer weiteren Aufarbeitung zuführt, das darin enthaltende Sticktoffmonoxid mit Sauerstoff und Methanol zu Methylnitrit umsetzt, abtrennt und in das Verfahren zurückführt und die verbleibenden akkumulierten inerten Gase aus dem Verfahren ausschleust,

(c) die gasförmigen Produkte zur erneuten Bildung des Methylnitrits mit Methanol, Sauerstoff sowie gegebenenfalls frisch zugesetztem Stickoxid oder Stickoxidäquivalenten umsetzt, wobei das Gasgemisch, welches das neu gebildete Methylnitrit enthält, abgeleitet und wiederum der Herstellung von Dimethylcarbonat zugeführt wird, und Wasser sowie gegebenenfalls entstehende weitere flüssige Nebenprodukte ebenfalls abgeleitet und, bevorzugt nach einer nachgeschalteten Rückgewinnung darin enthaltener Wertstoffe, ausgeschleust werden und

(d) die flüssigen Produkte von (b) einer destillativen Auftrennung unterwirft, in der das gesamte Produktgemisch zunächst einer ersten Destillation unterworfen wird, die bei einem Druck von 1 bis 25 bar, vorzugsweise von 1 bis 12 bar, durchgeführt wird, und dann entweder

(e1) das Kopfprodukt aus der ersten Destillation einer weiteren bei einem Druck von 200 bis 1500 mbar durchgeführten Destillation zuführt, in der als Bodenprodukt ein methanolreicher Ablauf erhalten wird, der zur Herstellung von Dimethylcarbonat, bevorzugt in den Teilschritt zur erneuten Bildung des Methylnitrits, zurückgeführt wird, und in der ein Kopfprodukt anfällt, das, gegebenenfalls gemeinsam mit anderen Rückströmen, wiederum in die erste Destillation zurückgeführt wird, oder

(e2) der Kopfprodukt aus der ersten Destillation einer Pervaporation oder einer Dampfpermeation, die auf der Retentatseite bei einer Temperatur von 20 bis 150°C und einem Druck von 0,5 bis 10 bar und auf der Permeatseite bei einer Temperatur von -30 bis +30°C und einem Druck von 0,5 bis 500 mbar betrieben wird, zuführt, in der als Permeat ein methanolreicher Abstrom erhalten wird, der zur Herstellung von Dimethylcarbonat, bevorzugt in den Teilschritt zur Regenerierung des Methylnitrits, zurückgeführt wird, und in der ein Retentat anfällt, das, gegebenenfalls gemeinsam mit anderen Rückströmen, wiederum in die erste Destillation zurückgeführt wird, und

(f) reines Dimethylcarbonat durch Destillation des als Bodenlauf der unter Überdruck durchgeführten ersten Destillation anfallenden Gemisches gewinnt.

Die beigefügten Zeichnungen Fig. 1 bis 6 haben folgende Bedeutung:

Fig. 1 zeigt einen anzustrebenden kontinuierlichen Kreisprozeß mit Methylnitritsynthese, DMC-Synthese, Isolierung und Reinigung von Dimethylcarbonat und Rückführung des entstehenden Stickstoffmonoxids.

Fig. 2 zeigt den kontinuierlichen Prozeß zur Herstellung von Dimethylcarbonat nach EP 523 728.

Fig. 3 zeigt das erfindungsgemäße Verfahren in der Variante gemäß (e1).

Fig. 4 zeigt das Schema zur Versuchsdurchführung des Beispiels 1 (Variante (e1)).

Fig. 5 zeigt das erfindungsgemäße Verfahren in der Variante gemäß (e2).

Fig. 6 zeigt das Schema zur Versuchsdurchführung des Beispiels 2 (Variante (e2)).

Fig. 7 zeigt das erfindungsmäße Verfahren in der Variante gemäß (e1) in ausführlicher Darstellung.

Fig. 8 zeigt ein Blockschema der apparativen Anordnung 8 (innerhalb der gestrichelten Linie) zur Abgasaufarbeitung/Wertstoffrückführung/Nebenproduktausschleusung, die in Fig. 7, 10, 12 und 13 nicht näher gezeigt wird.

Fig. 9 zeigt weitere Details der apparativen Anordnung 8 (vgl. Erläuterung zu Fig. 8).

Fig. 10 zeigt das Schema zur Versuchsdurchführung des Beispiels 3 (Variante (e1)).

Fig. 11 zeigt die apparative Anordnung 8, wie in den Beispielen 3 und 4 erläutert.

Fig. 12 zeigt das erfindungsgemäße Verfahren in der Variante gemäß (e2) in ausführlicher Darstellung.

Fig. 13 zeigt das Schema zur Versuchsdurchführung des Beispiels 4 (Variante (e2)).

Die Bezugszeichen für die Apparaturen haben in Fig. 1 bis 6 folgende Bedeutung: 1 = DMC-Synthese, 2 = Wäscher/Kondensator für das in 1 anfallende Gemisch, 2' = DMC-Extraktion (nur in Fig. 2), 3 = Methylnitritsynthese 4 = Druckdestillation, 4' = Methanol-Destillation I (nur in Fig. 2), 5 = DMC-Destillation 6 = Abwasser-Destillation, 6' =

Methanol-Destillation II (nur Fig. 2), 7 = Methanol-Abtrennung durch weitere Destillation (Variante (e1) in Fig. 3, 4, 7 und 10) bzw. durch Pervaporation oder Dampfpermeation (Variante (e2) in Fig. 5, 6, 12 und 13), 8 = Abgasbehandlung mit 8a = Methylnitrit-Desorber, 8b = Methylnitrit-Nachreaktor/Methanol-Abgaswäscher und 8c = Leichtsieder-Wäscher, 9, 10 = Speicherbehälter.

Die Bezugszeichen für Edukte und Produkte haben in Fig. 1 bis 6 folgende Bedeutung: I = CO, II = $CH_3OH$, III = $O_2$, IV = NaOH (wäßrig), V = $N_2$, VI = NO, VII = Abgas (Ausschleusung), VIII = DMC, IX = Abwasser (Ausschleusung), X = Oxalsäuredimethylester und andere Nebenprodukte (Ausschleusung); zusätzlich nur in Fig. 1: XI = gasförmige Produkte (hauptsächlich NO), XII = kondensierte Produkte (hauptsächlich DMC), XIII = Nebenkomponenten, die teils ausgeschleust werden (z.B. X) und teils recyclisiert werden, XIV = Aktivator, XV = $CO_2$, XVI = gegebenenfalls weitere Aufarbeitung. Auf die übrigen Bezugszeichen wird weiter unten eingegangen.

Platinmetalle für den Katalysator sind beispielsweise Ru, Rh, Pd, Ir oder Pt, bevorzugt Pd.

Katalysatorträger sind die dem Fachmann bekannten, beispielsweise Aluminiumoxide, Aktivkohlen, Metallphosphate, Zeolithe, Alumosilikate und Heteropolysäuren, bevorzugt Aluminiumoxid und A-Kohlen, besonders bevorzugt Aluminiumoxide.

Inertgase sind beispielsweise Kohlendioxid, Stickstoff oder Argon, bevorzugt Stickstoff und Kohlendioxid, besonders bevorzugt Kohlendioxid.

Das erfindungsgemäße Verfahren in der Variante gemäß (e1) ist in Fig. 3 schematisch abgebildet. Im wesentlichen beinhaltet es die in der Gasphase innerhalb eines geeigneten Reaktors in Gegenwart eines Inertgases ablaufende Umsetzung von Methylnitrit mit Kohlenmonoxid an einem Platinmetall, bevorzugt Palladium enthaltenden Katalysator. Die gasförmigen Reaktionsprodukte werden in einem Wäscher/Kondensator in gasförmige und flüssige Phasen aufgetrennt. Der gasförmige Anteil, der u.a. das bei der Umsetzung des Methylnitrits entstehende Stickstoffmonoxid enthält, wird zu dessen Wiedereinsatz mit Sauerstoff und Methanol auf geeignete Weise in einem weiteren Reaktor zur Reaktion gebracht. Das dabei anfallende Wasser wird in eine Abwasser-Aufarbeitung weitergegeben, während das wieder erhaltene Methylnitrit in die Dimethylcarbonat-Herstellung zurückgeführt wird. Die im Wäscher/Kondensator anfallenden flüssigen Reaktionsprodukte werden einer Druckdestillation zugeführt, in der Dimethylcarbonat und Schwersieder als Bodenprodukt vom Methanol und Dimethylcarbonat enthaltenden Kopfprodukt abgetrennt werden. Schließlich wird das gewünschte Reaktionsprodukt Dimethylcarbonat in einem letzten Destillationsschritt in hoher Reinheit isoliert, bei dem Dimethylcarbonat als Kopfprodukt und Schwersieder, z.B. Oxalsäuredimethylester, als Bodenprodukt erhalten werden, während Wertstoffe, wie Methanol, weitestgehend in den Prozeß zurückgeführt und Abfallstoffe, wie Abwasser, Abluft und Schwersieder, ausgeschleust werden. Das Kopfprodukt der Druckdesfillation wird unter normalem oder vermindertem Druck erneut destilliert (Variante gemäß (e1)), wobei als Bodenprodukt ein methanolreicher Ablauf erhalten wird, der recyclisiert wird, z.B. zur erneuten Bildung von Methylnitrit, und wobei das Kopfprodukt, gegebenenfalls gemeinsam mit anderen Rückströmen, der Druckdestillation zugeführt wird.

Die außerordentlich hohe Effektivität der erfindungsgemäßen Zusammenschaltung der genannten Apparaturen, die im nachfolgenden Text ausführlich erläutert wird, ist überraschend und wird in der Literatur nicht vorbeschrieben. Im Gegenteil wurden komplizierte bzw. gegenüber Nebenreaktionen bedeutend störanfälligere Operationen, wie die oben diskutierten und in EP 523 728 beschriebenen, vorgeschlagen. Besonders überraschend ist die hervorragende und insbesondere unter energetischen Gesichtspunkten vorteilhafte und damit ressourcenschonende Einpassung der Druckdestillation in das Gesamtverfahrenskonzept, wenn diese nicht, wie in der zitierten Literatur beschrieben, als isolierter Verfahrensschritt optimiert, sondern, wie es dem erfindungsgemäßen Verfahren entspricht, in Kombination mit Rückführungsschritten für Wertstoffe, wie insbesondere des zu recyclisierenden Methanols, eingesetzt wird.

Ein weiterer Vorteil des erfindungsgemäßen Verfahrens ist, daß eine besonders hohe Effizienz des Gesamtprozesses durch die bevorzugte Verwendung von Kohlendioxid als Inert- bzw. Trägergas erreicht wird. Während Verfahren zur Herstellung von Dimethylcarbonat auf Basis von Methylnitrit und Kohlenmonoxid, die Katalysatoren des beispielsweise in den Patentanmeldungen EP 425 197, EP 464 460, EP 503 091, EP 503 618 und EP 523 728 beschriebenen Typs einsetzen, wie alle dort zitierten Beispiele belegen, auf die Verwendung von Stickstoff als Inert- bzw. Trägergas angewiesen sind, eignen sich die genannten neuen Katalysatoren, die beispielsweise in den Patentanmeldungen EP 523 508, EP 538 676, EP 559 001, EP 558 996 und EP 581 240 beschrieben werden und die vorzugsweise, jedoch nicht ausschließlich, auch in dem erfindungsgemäßen Verfahren zur Anwendung kommen, vor allem für einen Einsatz in Gegenwart von Kohlendioxid als Inert- bzw. Trägergas. Aufgrund der völlig überraschenden Beobachtung, daß die Grenze für die Zündfähigkeit des Reaktand- bzw. Produktgasgemisches in den prozeßtypischen Gaskreisläufen bei Verwendung von Kohlendioxid als Inert- bzw. Trägergas bei ca. 30-50 mol-% liegt, bei Verwendung von Stickstoff jedoch deutlich höher, nämlich bei ca. 50-80 mol-% (je nach Art und Menge der übrigen Komponenten innerhalb des Gesamtgasgemischs), ergibt sich für das erfindungsgemäße Verfahren ein erheblicher ökonomischer Vorteil, beispielsweise dadurch, daß der nutzbare Volumenanteil (Gesamtvolumen abzüglich Inert- bzw. Trägergasanteil) im Falle des bevorzugten Einsatzes von Kohlendioxid größer ist.

Innerhalb der Apparatur 1 (Dimethylcarbonat-Synthese, vgl. Fig. 3) läuft entsprechend Reaktionsgleichung (1) die Bildung von Dimethylcarbonat ab. Dazu wird der der Apparatur 3 (Methylnitrit-Synthese) entstammende und das regenerierte Methylnitrit enthaltende Kreisgasstrom (d) nach Passieren eines Wärmeaustauschers, der die Einstellung

einer definierten Temperatur erlaubt, als Strom (d1) mit Kohlenmonoxid (a) sowie gegebenenfalls (nicht in Fig. 3 enthalten), absatzweise oder kontinuierlich, mit weiteren gasförmigen Hilfsstoffen, wie beispielsweise geringen Mengen Halogen oder Halogenwasserstoff, versetzt und als Feedgasstrom (d2) in die genannte Apparatur 1 geleitet. Die im Verlaufe der Bildung von Dimethylcarbonat freiwerdende Reaktionswärme ist dabei ganz oder teilweise abzuführen.

Bei der Apparatur 1 handelt es sich daher beispielsweise um einen Rohrbündelreaktor, der z.B. mit Warmwasser gekühlt wird.

Vorzugsweise handelt es sich bei der Apparatur 1 um eine in zwei Reaktionszonen geteilte Apparatur, innerhalb derer die erste Reaktionszone als Rohrbündelreaktor, der beispielsweise mit Warmwasser gekühlt wird, und die zweite als nachgeschalteter, adiabat betriebener Reaktor ausgestaltet ist. In einer anderen bevorzugten Variante wird die genannte Apparatur 1 als Flachbettreaktor, der adiabat mit Zwischenkühlung oder auch isotherm betrieben werden kann, ausgestaltet. In einer dritten bevorzugten Variante handelt es sich bei der genannten Apparatur 1 um einen adiabat mit Zwischenkühlung betriebenen Reaktor.

Grundsätzlich kann es von Vorteil sein, die Apparatur 1 doppelt auszuführen, so daß bei Einsatz von Katalysatoren, die einer regelmäßigen Regenerierung bedürfen, einer der Reaktoren zur Herstellung von Dimethylcarbonat genutzt werden kann, während der andere für die genannte Regenerierungsprozedur zur Verfügung steht. Die Verschaltung dieser beiden Reaktoren erfolgt auf an sich bekannte Weise.

Eine weitere grundsätzlich mögliche Ausführungsform der Apparatur 1 kann so beschaffen sein, daß Teilmengen des zur Anwendung kommenden Katalysators, absatzweise oder kontinuierlich, aus der Reaktionszone bzw. dem eigentlichen Reaktor an einer oder mehreren Stellen entnommen werden und einer externen Aufarbeitung. Regenerierung oder Reaktivierung bzw. einer Entsorgung zugeführt werden können, während, gleichfalls absatzweise oder kontinuierlich, an einer oder mehreren Stellen, aufgearbeiteter, regenerierter, reaktivierter oder frischer Katalysator wieder in den Reaktor bzw. die Reaktionszone eingebracht werden kann.

Innerhalb der Apparatur 2 (Dimethylcarbonat-Wäscher/Kondensator, vgl. Fig. 3) werden die der Dimethylcarbonat-Synthese entströmenden Produktgase (e) unter festgelegten Bedingungen (Druck, Temperatur, Gasgeschwindigkeit etc.) in kondensierbare und nicht kondensierbare Reaktionsprodukte (f) und (h) getrennt. Zusätzlich wird die Apparatur 2 gegebenenfalls mit einem Teilstrom (b2) des in den Gesamtprozeß eingeschleusten Frischmethanols beschickt, der in ihren oberen Bereich eingespeist wird. Der am Kopf der Apparatur 2 erhaltene Strom (f) wird nach Abzweigung des für die Ausschleusung bestimmten Anteils (f2) als Kreisstrom (f1) an einen Wärmeaustauscher weitergegeben, der die Einstellung der gewünschten Eingangstemperatur für die weitere Umsetzung innerhalb der Apparatur 3 (Methylnitrit-Synthese) erlaubt. Der so erwärmte Strom (f3) passiert den Verdichter und wird als Strom (f4) mit dem aus nachgeführtem Inertgas, vorzugsweise und wie in Fig. 3 abgebildet Stickstoff (s), und nachgeführtem frischem Stickoxid (in Fig. 3 Stickstoffmonoxid) (r) zusammengesetzten Strom (s1) vereinigt, bevor er als Feedgasstrom (f5) in die Herstellung des Methylnitrits einfließt.

Die ausgeschleuste Teilmenge des Kreisgases (f2) kann gegebenenfalls einer Behandlung durch nachgeschaltete Operationen, wie sie beispielsweise in DE-OS 3 834 065 beschrieben werden, unterzogen werden. Eine derartige Nachbehandlung kann insbesondere dem Ziel dienen, in der ausgeschleusten Teilmenge (f2) des Kreisgasstroms verbliebene Wertstoffe, wie beispielsweise unumgesetztes Methylnitrit, Stickstoffmonoxid oder gasförmiges Dimethylcarbonat, zurückzugewinnen und an geeigneter Stelle in den Prozeß der Dimethylcarbonat-Herstellung zurückzuführen. Auf diese Weise wird zugleich die Abgabe giftiger gasförmiger Stoffe, wie beispielsweise Methylnitrit oder Stickstoffmonoxid, an die Umwelt vermieden.

Bei der Apparatur 2 handelt es sich beispielsweise um einen Rohrbündelwärmeaustauscher, der in stehender oder liegender Bauweise ausgeführt sein kann, um einen Plattenwärmeaustauscher, einen Spiralstromwärmeaustauscher, einen Einsprühkondensator, einen mit einem Kopfkondensator versehenen Wäscher, einen Rippenrohrwärmeaustauscher oder um eine Kombination der genannten Kondensator- bzw. Wärmeaustauscher- und Wäschertypen, beispielsweise um einen Einspritzkondensator mit nachgeschaltetem Rippenrohrwärmeaustauscher.

In einer bevorzugten Ausführungsform wird die Apparatur 2 als Einsprühkondensator, d.h. als spezielle Variante eines Direktkontakt-Wärmeaustauschers, mit hintereinander angeordneten Sprühdüsen, die über einen Kreislauf mit kaltem Kondensat beschickt werden und auf diese Weise die Realisierung von zwei oder mehr theoretischen Trennstufen ermöglichen, ausgeführt.

In einer weiteren bevorzugten Ausführungsform handelt es sich bei der Apparatur 2 um einen mit Kopfkondensator versehenen Wäscher, innerhalb dessen eine Zahl von mehr als drei theoretischen Trennstufen realisiert wird.

Besonders bevorzugt wird die Apparatur 2, beispielsweise als Wäscher, in einer Weise betrieben, bei der im Sumpfbereich die dem Siedepunkt des Dimethylcarbonats unter dem gegebenen Reaktionsdruck entsprechende Temperatur eingestellt wird (beispielsweise ca. 128°C bei 3 bar), um an dieser Stelle im rohen Produktgemisch vorhandenes Methanol überwiegend als Teil des Kopfstroms (f) gasförmig und im wesentlichen als Azeotrop mit Dimethylcarbonat von nahezu reinem flüssigen Dimethylcarbonat als Hauptbestandteil des Bodenstroms (h) abtrennen zu können.

Innerhalb der Apparatur 3 (Methylnitrit-Synthese, vgl. Fig. 3) erfolgt, entsprechend beispielsweise einer der Reaktionsgleichungen (2), (3) oder (4), die Bildung bzw. erneute Bildung des Methylnitrits. Dazu werden die als Methylnitrit-Äquivalente bzw. -Vorstufen fungierenden Stickoxide mit Sauerstoff (c) und Methanol (Frischmethanol sowie Methanol-Rückströme aus Methanol-Abtrennung in Apparatur 7 und aus Abwasser-Destillation in Apparatur 6) (b3, b4, m1, m3) zur Reaktion gebracht. Das dabei entstehende Wasser sowie etwaige gebildete Nebenprodukte, wie etwa Salpetersäure, werden am Boden der Apparatur (g) abgeführt, das Methylnitrit enthaltende Produktgasgemisch (d) wird nach Passieren eines am Kopf der Apparatur befindlichen Kondensators (nicht in Fig. 3 enthalten) abgeleitet und für die Bildung von Dimethylcarbonat bereitgestellt. Das in die Apparatur 3 eingebrachte Methanol dient somit zum einen als Reaktionspartner bei der gemäß einer der Reaktionsgleichungen (2), (3) oder (4) erfolgenden Bildung von Methylnitrit, zum anderen, insbesondere in Form der am oberen Teil der Apparatur aufgebrachten Teilströme (b3, 1, m1) sowie dem vom Kopfkondensator zurücktropfenden Rücklauf als Waschflüssigkeit zur Entfernung des gebildeten Wassers. Bei den zur Umsetzung gelangenden Stickoxiden handelt es sich im wesentlichen um Stickstoffmonoxid, das im Zuge der Bildung von Dimethylcarbonat gemäß Reaktionsgleichung (1) freigesetzt und, im Gemisch mit weiteren gasförmigen Komponenten, wie beispielsweise dem Inertgas, und gegebenenfalls nicht vollständig umgesetzten gasförmigen Reaktanden, wie etwa Kohlenmonoxid oder Methylnitrit selbst, im Sinne eines Kreisprozesses (vgl. auch Fig. 1) gemäß Reaktionsgleichung (2) zurückgeführt wird (f->f1->f3->f4). Grundsätzlich mögliche Verluste an innerhalb des gesamten Kreisprozesses vorhandenem Methylnitrit selbst oder an Methylnitrit-Äquivalenten bzw. -Vorstufen, namentlich an Stickstoffmonoxid, Stickstoffdioxid, Distickstoffdioxid oder Distickstoffetroxid, können sich durch unerwünschte Nebenreaktionen oder auch durch Ausschleusungen (f2) ergeben. Bei den genannten unerwünschten Nebenreaktionen kann es sich beispielsweise um den im untergeordneten Ausmaß ablaufenden Prozeß der Bildung von Salpetersäure gemäß Reaktionsgleichung (4) oder um die eventuelle Bildung von Stickstoff oder Distickstoffmonoxid aus Stickstoffmonoxid handeln, wie sie sich etwa gemäß den Reaktionsgleichungen (12) und (13) formulieren läßt.

$$2\ NO + 2\ CO \rightarrow N_2 + 2\ CO_2 \qquad (12)$$

$$2\ NO + CO \rightarrow N_2O + CO_2 \qquad (13)$$

Verluste der genannten Art lassen sich beispielsweise dadurch ausgleichen, daß dem Gesamtprozeß das fehlende Methylnitrit selbst oder auch entsprechende Mengen an Methylnitrit-Äquivalenten oder -Vorstufen, namentlich Stickstoffmonoxid, Stickstoffdioxid, Distickstofftrioxid, Distickstofftetroxid oder Gemische einzelner dieser Komponenten absatzweise oder kontinuierlich, bevorzugt kontinuierlich, zugesetzt werden. In Fig. 3 geschieht dies beispielsweise durch die gemeinsame Zuspeisung des Inertgases Stickstoff und Stickstoffmonoxid (s1), jedoch ist das erfindungsgemäße Verfahren nicht auf diese Ausführungsform beschränkt.

Im allgemeinen ist es unter ökologischen Gesichtspunkten nicht akzeptabel, Salpetersäure enthaltende Abwässer, auch wenn es sich dabei nur um geringe Mengen handelt, ohne Nachbehandlung freizusetzen. Das erfindungsgemäße Verfahren beinhaltet aus diesem Grund einen bevorzugt kontinuierlich betriebenen Neutralisationsschritt, bei dem eine geeignete Base zum Abfangen solcher Salpetersäure verwendet wird. In Fig. 3 wird dazu beispielsweise Natronlauge verwendet, es kommen aber auch andere Basen, wie etwa Kalilauge, Kalkmilch oder wäßrige Lösungen von Natriumcarbonat und Natriumhydrogencarbonat in Frage. Nach Zumischung der betreffenden Base, beispielsweise Natronlauge (q), zu dem Bodenstrom (g) der Apparatur 3 wird somit ein neutralisierter, im wesentlichen Wasser und Methanol enthaltender Strom (g1) erhalten, der an Apparatur 6 (Abwasser-Destillation) weitergegeben wird.

Bei der Apparatur handelt es sich beispielsweise um einen kolonnenartigen Wäscher, der zur Verbesserung des Warme- und Stoffaustauschs mit Einbauten, wie sie üblicherweise bei thermischen Trennaufgaben verwendet werden, versehen ist. Als derartige Einbauten genannt seien beispielsweise Füllkörper, Böden, wie etwa Glockenböden, Siebböden oder Ventilböden, geordnete Packungen oder Spritzdüsen.

Gegebenenfalls ist der untere Bereich der Apparatur mit einem Sumpfverdampfer ausgestattet, mit dessen Hilfe eine definierte Sumpftemperatur in gewissen Grenzen eingestellt werden kann, die durch die Phasengleichgewichte der im Sumpf vorhandenen Stoffe bestimmt werden.

Grundsätzlich geeignet und einsetzbar für die Vermischung der am unteren Ende der Apparatur 3 einzuspeisenden flüssigen und gasförmigen Komponenten bzw. Ströme (b4, c, f5, m3) sind Statikmischer, Strahlmischer, rotierende Mischer, Ein-und Zweistoffdüsen, Wirbelschichtmischer, wie sie beispielsweise von der Firma Sulzer vertrieben werden, Mischkammern, wie sie beispielsweise von der Firma Pfaudler vertrieben werden, in line - Vollturbulenzrohre, HI-Mischer, wie sie beispielsweise von der Firma Toray vertrieben werden, Komax-Mischelemente, Wendelmischer, Kenix-Mischer, mit Füllkörpern, wie beispielsweise Raschig-Ringen, befüllte Rohre sowie Kombinationen derartiger Elemente. Die Zuspeisung von Sauerstoff (c) erfolgt bevorzugt in den unteren Teil der Apparatur 3 hinein. Ein Teilstrom (b3) des Frischmethanols sowie die Methanol-Rückströme (1) und (m1) werden in flüssiger Form in den oberen Teil des Reaktors eingeleitet. Ein anderer Teil des Methanols (b4) sowie der Methanol-Rückstrom (m3) werden getrennt oder gemeinsam mit dem aus zurückzuführendem Kreisgas sowie den frisch zugespeisten Stickoxid- und Inertgasströmen gebildeten Gesamtstrom (f5) in den unteren Teil der Apparatur 3 eingespeist. In Fig. 3 ist lediglich eine der erfindungsgemäßen möglichen Varianten abgebildet, bei der die Einspeisung des Sauerstoffs (c), des Methanol-Teilstroms (b4) sowie des vereinigten Stroms aus zurückzuführendem Kreisgas, neu zugespeistem Inertgas und frisch zugeführtem

Stickoxid (f5) in getrennter Form vorgenommen wird. Das erfindungsgemäße Verfahren ist jedoch keinesfalls auf diese Ausführungsform beschränkt. Insbesondere im Falle der Verwendung spezieller Einspeiseaggregate, wie insbesondere Zweistoffdüsen und/oder Statikmischern sowie Kombinationen derartiger Elemente, ist es von Vorteil, die Ströme (f5) und/oder (b4) und/oder (m3) sowie den Sauerstoffstrom (c) gemeinsam in den unteren Teil der Apparatur 3 einzuführen.

In einer bevorzugten Ausführungsform erfolgt die Vermischung der am unteren Ende der genannten Apparatur 3 einzuspeisenden flüssigen und gasförmigen Komponenten bzw. Ströme (b4, c, f5, m3) mittels eines effektiv arbeitenden Statikmischers, wie er beispielsweise von der Firma Sulzer (SMX- oder SMV-Typ) vertrieben wird. Der Einsatz derartiger Aggregate wird beispielsweise in Ing. Tech. 51 (5), S. 307 ff. (1979) oder Chem. Eng. Process 25, S. 59 ff. (1989) beschrieben.

In einer weiteren bevorzugten Ausführungsform verwendet man zur Vermischung der am unteren Ende der Apparatur 3 einzuspeisenden flüssigen und gasförmigen Komponenten bzw. Ströme (b4, c, f5, m3) Einstoff- oder Zweistoffdüsen oder Kombinationen aus Statikmischern und Ein- oder Zweistoffdüsen.

Bei Apparatur 4 (Druckdestillation, vgl. Fig. 3) handelt es sich um eine unter einem Sumpfdruck von ca. 1 bis 25 bar, bevorzugt von 1 bis 12 bar betriebene Destillationskolonne. Sie dient der Aufspaltung des Stromes (h) in ein aus Methanol und Dimethylcarbonat bestehendes azeotropnahes Gemisch, das am Kopf der Apparatur entnommen wird (k) und ein Bodenprodukt (i), das noch verbleibende Schwersieder, insbesondere Oxalsäuredimethylester, enthält und das nach Passieren eines Wärmeaustauschers, der die Einstellung einer bestimmten Temperatur erlaubt, als Strom (i1) an die Apparatur 5 (Dimethylcarbonat-Destillation) weitergeleitet wird. Die exakte Zusammensetzung des genannten Kopfproduktes ist dabei u.a. von dem absoluten Druck, unter dem die Destillation durchgeführt wurde, abhängig (vgl. DE-OS 2 607 003 sowie JP 02-212 456).

Für die genannte Apparatur kommen im allgemeinen Destillationskolonnen in Frage, die üblicherweise für die thermische Trennung von Stoffgemischen unter Druck Verwendung finden, die über einen Abtriebs- und einen Verstärkerteil verfügen und die, dem Trennproblem entsprechend, die erforderliche Anzahl theoretischer Trennstufen besitzen. Beispielsweise genannt seien Bodenkolonnen, Füllkörperkolonnen sowie Kolonnen, die als Einbauten geordnete Packungen enthalten. Bevorzugt genannt seien Füllkörperkolonnen sowie Kolonnen, die geordnete Packungen als Einbauten enthalten.

Bei Apparatur 5 (Dimethylcarbonat-Destillation, vgl. Fig. 3) handelt es sich um eine Destillationskolonne, die mit dem Bodenprodukt (i1) der Apparatur 4 (Druckdestillation) beschickt wird und die der Abtrennung des gewünschten Reaktionsproduktes Dimethylcarbonat (o) in spezifikationsgerechter Form von dem im wesentlichen aus Oxalsäuredimethylester sowie sonstigen möglichen Schwersiedern bestehenden Bodenprodukt (i) dient.

Für die Apparatur 5 kommen im allgemeinen Destillationskolonnen in Frage, wie sie für Apparatur 4 genannt werden; eine druckfeste Ausführung ist jedoch nicht erforderlich.

Bei Apparatur 6 (Abwasser-Destillation, vgl. Fig. 3) handelt es sich um eine Destillationskolonne, die mit dem neutralisierten Bodenablauf (g1) der Apparatur 3 (Methylnitrit-Synthese) beschickt wird. Aufgabe dieser Kolonne ist die Abtrennung und Rückführung des im Strom (g1) enthaltenen Methanols in Form des Kopfstroms (m), der in Form der Teilströme (m1) und (m3) in die Apparatur 3 (Methylnitrit-Synthese) und als Teilstrom (m2), mit dem Kopfstrom (n) der Apparatur 7 (Methanol-Abtrennung) zu dem Strom (n1) vereinigt, in die Apparatur 4 (Druckdestillation) zurückgeführt wird. Am Boden der Apparatur 6 fällt das eigentliche Abwasser des Gesamtprozesses (p) an.

Für die Apparatur 6 kommen im allgemeinen Destillationskolonnen in Frage, wie sie für die Apparatur 4 und 5 genannt werden; eine druckfeste Ausführung ist jedoch nicht erforderlich.

Bei Apparatur 7 (Methanol-Abtrennung, vgl. Fig. 3) handelt es sich um eine unter Normaldruck oder unter vermindertem Druck betriebene Destillationskolonne. Sie dient der Aufspaltung des als Kopfprodukt der Destillationskolonne 4 anfallenden Stromes (k) in ein aus Methanol und Dimethylcarbonat bestehendes Azeotrop, das am Kopf der Apparatur entnommen wird (n) und gemeinsam mit einem Teil des aus Destillationskolonne 6 (Abwasser-Kolonne) anfallenden Methanol-Rückstroms (m2) wieder in Destillationskolonne 4 eingespeist wird (n1), sowie einen im wesentlichen aus Methanol bestehenden Bodenablauf (1), der in den oberen Teil des Methylnitrit-Reaktors (Apparatur 3) zurückgeführt wird.

Für die Apparatur 7 kommen im allgemeinen Destillationskolonnen in Frage, wie sie für die Apparaturen 4, 5 und 6 genannt wurden; sie sind jedoch für einen Betrieb unter vermindertem Druck eingerichtet.

Im folgenden werden die in Fig. 3 beschriebenen Stoffströme sowie die zugehörigen Druck- und Temperaturbedingungen, wie sie bei Durchführung des erfindungsgemäßen Verfahrens realisiert werden, angegeben.

Der im kontinuierlichen Betrieb in die Apparatur 1 eingeleitete Gasstrom (d2) besitzt im allgemeinen eine Temperatur von 25 bis 120°C, vorzugsweise von 40 bis 110°C und einen Druck von 1 bis 5 bar, vorzugsweise von 2 bis 4 bar. Er ist im allgemeinen zusammengesetzt aus 4 bis 16 Mol-% Kohlenmonoxid, 0 bis 5 Mol-% Kohlendioxid, 5 bis 25 Mol-% Methylnitrit, 40 bis 80 Mol-% Stickstoff, 1 bis 10 Mol-% Methanol, 0 bis 5 Mol-% Dimethylcarbonat, 0 bis 5 Mol-% Stickstoffmonoxid, 0 bis 1 Mol-% Wasser und weniger als 5 Mol-% diverser, zumeist leicht flüchtiger Nebenkomponenten; vorzugsweise aus 8 bis 14 Mol-% Kohlenmonoxid, 0,5 bis 3 Mol-% Kohlendioxid, 10 bis 20 Mol-% Methylnitrit, 50 bis 75 Mol-% Stickstoff, 1,5 bis 8 Mol-% Methanol, 0 bis 2 Mol-% Dimethylcarbonat, 0,1 bis 5 Mol-% Stickstoffmonoxid,

0 bis 0,5 Mol-% Wasser und weniger als 4 Mol-% zumeist leicht flüchtiger Nebenkomponenten.

Der die Apparatur 1 im kontinuierlichen Betrieb verlassende Gasstrom (e) besitzt im allgemeinen eine Temperatur von 50 bis 170°C, vorzugsweise von 60 bis 160°C und einen Druck von 1 bis 5 bar, vorzugsweise von 1,5 bis 4 bar. Er ist im allgemeinen zusammengesetzt aus 0 bis 13 Mol-% Kohlenmonoxid, 0 bis 5 Mol-% Kohlendioxid, 0 bis 20 Mol-% Methylnitrit, 40 bis 80 Mol-% Stickstoff, 1 bis 10 Mol-% Methanol, 1 bis 10 Mol-% Dimethylcarbonat, 3 bis 15 Mol-% Stickstoffmonoxid, 0 bis 0,5 Mol-% Wasser und weniger als 5 Mol-% diverser, zumeist leicht flüchtiger Nebenkomponenten; vorzugsweise aus 1 bis 12 Mol-% Kohlenmonoxid, 0,5 bis 3 Mol-% Kohlendioxid, 5 bis 15 Mol-% Methylnitrit, 50 bis 75 Mol-% Stickstoff, 1,5 bis 8 Mol-% Methanol, 2 bis 8 Mol-% Dimethylcarbonat, 5 bis 14 Mol-% Stickstoffmonoxid, 0 bis 0,5 Mol-% Wasser und weniger als 4 Mol-% zumeist leicht flüchtiger Nebenkomponenten.

Der die Apparatur 2 im kontinuierlichen Betrieb verlassende Gasstrom (f) besitzt im allgemeinen eine Temperatur von 0 bis 40°C, vorzugsweise von 5 bis 35°C und einen Druck von 1 bis 5 bar, vorzugsweise von 2 bis 4 bar. Er ist im allgemeinen zusammengesetzt aus 0 bis 13 Mol-% Kohlenmonoxid, 0 bis 5 Mol-% Kohlendioxid, 0 bis 20 Mol-% Methylnitrit, 40 bis 80 Mol-% Stickstoff, 0 bis 10 Mol-% Methanol, 0 bis 3 Mol-% Dimethylcarbonat, 3 bis 15 Mol-% Stickstoffmonoxid, 0 bis 0,5 Mol-% Wasser und weniger als 5 Mol-% diverser, zumeist leicht flüchtiger Nebenkomponenten; vorzugsweise aus 1 bis 12 Mol-% Kohlenmonoxid, 0,5 bis 3 Mol-% Kohlendioxid, 5 bis 15 Mol-% Methylnitrit, 50 bis 75 Mol-% Stickstoff, 1 bis 8 Mol-% Methanol, 0 bis 2 Mol-% Dimethylcarbonat, 5 bis 14 Mol-% Stickstoffmonoxid, 0 bis 0,5 Mol-% Wasser und weniger als 4 Mol-% zumeist leicht flüchtiger Nebenkomponenten.

Die Menge des als Gasstrom (f2) ausgeschleusten Anteils des Kreisgases beträgt im kontinuierlichen Betrieb im allgemeinen 0 bis 7 Gew.-%, bevorzugt 0,1 bis 5 Gew.-%, bezogen auf den Kopfstrom (f) der Apparatur 2.

Der nach Ausschleusung des Gasstroms (f2) verbleibende Hauptstrom (f1) wird nach Passieren eines Wärmeaustauschers sowie eines Verdichters, gegebenenfalls (vgl. Fig. 3) unter vorheriger Hinzumischung frischen Stickoxids und/oder Inertgases, in die Apparatur 3 eingespeist.

Die Einspeiserate des im allgemeinen bei Umgebungstemperatur als Strom (r) herangeführten gasförmigen Stickstoffmonoxids in die Apparatur 3 (vgl. Fig. 3) beträgt im kontinuierlichen Betrieb 0 bis 3 Mol-%, bevorzugt 0 bis 1,5 Mol-%, bezogen auf den Gasstrom (f4). Anstelle des Stickstoffmonoxids können grundsätzlich auch äquivalente Mengen Stickstoffdioxid, Distickstofftrioxid, Distickstofftetroxid, Methylnitrit oder beliebige Gemische dieser Substanzen zugespeist werden.

Die Einspeiserate des im allgemeinen bei Umgebungstemperatur als Strom (s) herangeführten gasförmigen Stickstoffs in die Apparatur 3 (vgl. Fig. 3) beträgt im kontinuierlichen Betrieb 0 bis 3 Mol-%, bevorzugt 0 bis 1,5 Mol-%, bezogen auf den Gasstrom (f4).

Die Temperatur des Gasstroms (f5) beträgt bei kontinuierlichem Betrieb im allgemeinen 0 bis 70°C, bevorzugt 20 bis 60°C, bei einem Druck von 1 bis 5 bar, bevorzugt 1,5 bis 4 bar.

Im kontinuierlichen Betrieb wird die Menge des via Zuleitung (b4) eingespeisten Methanols vorteilhaft so gewählt, daß das Stoffmengenverhältnis zwischen diesem Methanol und dem im zugespeisten Gasstrom (f5) enthaltenen Stickstoffmonoxid 0,1 bis 5, bevorzugt 0,1 bis 2 beträgt. Die Temperatur des eingespeisten Methanols beträgt im allgemeinen 10 bis 80°C, bevorzugt 20 bis 60°C.

Die Menge des via Zuleitung (b3) im kontinuierlichen Betrieb eingespeisten Methanols wird vorteilhaft so gewählt, daß das Stoffmengenverhältnis zwischen diesem Methanol und dem im zugespeisten Gasstrom (f5) enthaltenen Stickstoffmonoxid 0,1 bis 5, bevorzugt 0,2 bis 4 beträgt. Die Temperatur des eingespeisten Methanols beträgt im allgemeinen 10 bis 40°C, bevorzugt 10 bis 35°C.

Im kontinuierlichen Betrieb wird die Menge des im allgemeinen bei Umgebungstemperatur via Zuleitung (c) herbeigeführten Sauerstoffs vorteilhaft so gewählt, daß das Stoffmengenverhältnis zwischen Sauerstoff und dem im zugespeisten Gasstrom (f5) enthaltenen Stickstoffmonoxid 0,125 bis 0,25, bevorzugt 0,15 bis 0,245 beträgt.

Der Innendruck der genannten Apparatur 3 beträgt im allgemeinen 1 bis 5 bar, bevorzugt 1,5 bis 4 bar, ihre Innentemperatur ist in weiten Grenzen variabel. Das Temperaturprofil über die gesamte Länge der Apparatur stellt sich in Abhängigkeit von Menge, Eintrittstemperatur und Aggregatzustand der Einzelzuspeisungen, Gesamtdruck, Umsatz der eingebrachten Reaktanden, Rücklauf des Kopfkondensators sowie Energiezufuhr am Sumpf der Apparatur ein.

Der die Apparatur 2 als Bodenprodukt verlassende und der Apparatur 4 zugeführte Strom (h) besitzt im allgemeinen eine Temperatur von 10 bis 150°C, vorzugsweise von 20 bis 140°C, und enthält 1 bis 60 mol-% Methanol, 20 bis 99 mol-% Dimethylcarbonat sowie gegebenenfalls geringe Mengen Schwersieder, wie beispielsweise Oxalsäuredimethylester neben weiteren Komponenten in einer Größenordnung von insgesamt weniger als 10 mol-%, bevorzugt weniger als 5 mol-%.

Der die Apparatur 4 als Bodenprodukt verlassende Strom (i) hat im allgemeinen eine Temperatur von 90 bis 240°C, vorzugsweise von 90 bis 190°C, und enthält im allgemeinen weniger als 0,1 mol-% Methanol, bevorzugt weniger als 0,05 mol-% Methanol, mehr als 90 mol-% Dimethylcarbonat, bevorzugt mehr als 95 mol-% Dimethylcarbonat, sowie gegebenenfalls geringe Mengen Schwersieder, wie beispielsweise Oxalsäuredimethylester und weitere Komponenten in einer Größenordnung von insgesamt weniger als 10 mol-%, bevorzugt weniger als 5 mol-%.

Der die Apparatur 4 als Kopfprodukt verlassende und der Apparatur 7 zugeführte Strom (k) hat im allgemeinen eine Temperatur von 80 bis 160°C, vorzugsweise von 90 bis 140°C, und enthält im allgemeinen 55 bis 97 mol-% Methanol,

2 bis 35 mol-% Dimethylcarbonat, 0 bis 15 mol-% Wasser sowie gegebenenfalls geringe Mengen Leichtsieder.

Der in die Apparatur 4 eingespeiste und als Kopfstrom aus Apparatur 7 herbeigeführte flüssige Strom (n) besitzt eine Temperatur von 40 bis 90°C, bevorzugt 50 bis 80°C, und enthält 50 bis 90 mol-% Methanol, 5 bis 30 mol-% Dimethylcarbonat sowie geringe Mengen leicht siedender Komponenten in der Größenordnung von im allgemeinen weniger als 10 mol-%.

Der die Apparatur 7 als Bodenstrom verlassende und in die Apparatur 3 eingespeiste Strom (1) enthält im allgemeinen mehr als 80 mol-% Methanol, bevorzugt mehr als 90 mol-% Methanol, sowie geringe Mengen Dimethylcarbonat und Wasser.

Die Apparatur 6 (Abwasser-Destillation) wird im allgemeinen bei einem Druck von 0,5 bis 2 bar, bevorzugt von 0,5 bis 1,5 bar, betrieben. Das kondensierte Kopfprodukt (m) fällt im kontinuierlichen Betrieb mit einer Temperatur von 15 bis 50°C, bevorzugt von 15 bis 35°C an und besitzt eine Zusammensetzung von 60 bis 95 Mol-% Methanol, 1 bis 35 Mol-% Dimethylcarbonat und 0 bis 7 Mol-% Wasser, vorzugsweise von 70 bis 88 Mol-% Methanol, 2 bis 30 Mol-% Dimethylcarbonat und 0 bis 5 Mol-% Wasser.

Die Menge des als Teilstrom (m2) zurückgeführten Methanol/Dimethylcarbonat-Gemisches kann, je nach Gehalt an Dimethylcarbonat, so eingestellt werden, daß sie 0 bis 90 %, bevorzugt 0 bis 30 % des gesamten Stroms (m) umfaßt.

Die Menge des als Teilstrom (m1) zurückgeführten Methanol/Dimethylcarbonat-Gemisches kann, je nach Gehalt an Dimethylcarbonat, so eingestellt werden, daß sie zwischen 0 und 100 % des gesamten Stroms (m) umfaßt. Gleiches gilt für die Menge des als Teilstrom (m3) zurückgeführten Methanols.

Das im allgemeinen bei Umgebungstemperatur als Strom (a) zugespeiste Kohlenmonoxid wird im allgemeinen in chemisch reiner Form eingesetzt, kann jedoch nach Herstellungsart Fremdgase, wie beispielsweise geringe Mengen Wasserstoff (< 0,1 Mol-%) oder Methan (< 0,1 Mol-%) enthalten. Seine Zuspeisung erfolgt im kontinuierlichen Betrieb in einer Weise, daß das Stoffmengenverhältnis zwischen zudosiertem Kohlenmonoxid und produziertem Dimethylcarbonat 1 bis 1,2 beträgt und eine konstante Konzentration des Kohlenmonoxids im Gasstrom (d2) vorliegt.

Das in Apparatur 5 destillierte Dimethylcarbonat hat, je nach Rücklauf/Entnahme-Verhältnis, eine Reinheit von 99,0 bis 99,9 %.

Eine weitere Anordnung für die Variante (e1) wird mit Hilfe von Fig. 7 wie folgt beschrieben:

Apparatur 1 gleicht der in Fig. 3. (y) ist die absatzweise oder kontinuierliche Einspeisung geringer Mengen gasförmiger Hilfsstoffe (z.B. Halogen oder Halogenwasserstoff). Als Inertgas wird $CO_2$ an Stelle von $N_2$ eingesetzt.
Apparatur 2 von Fig. 7 gleicht im wesentlichen der in Fig. 3. Über (b3) wird ein überwiegend Methanol enthaltender Teilstrom aus der apparativen Anordnung 8 (Abgasaufarbeitung/Wertstoffrückführung/Nebenproduktausschleusung), nämlich aus (b1) in den oberen Bereich der Apparatur 2 eingespeist.

Der am Kopf der Apparatur 2 erhaltene gasförmige Strom (f) kann nach Abzweigung des für die Ausschleusung bestimmten Anteils (f2) als Kreisstrom (f1) gegebenenfalls an einen Wärmeaustauscher weitergegeben werden (in Fig. 7 enthalten), der die Einstellung der gewünschten Eingangstemperatur für die weitere Umsetzung erlaubt. Der so erwärmte Strom (f3) passiert den Verdichter und wird als Strom (f4) in die apparative Anordnung 8 eingeleitet. Je nach Betriebsweise der Gesamtanlage kann gegebenenfalls auch auf einen derartigen vorgeschalteten Wärmeaustauscher verzichtet werden. Dieser apparativen Anordnung 8, deren Aufbau und Funktion weiter unten erläutert wird, wird der Strom (f5) entnommen, der als wesentliche Bestandteile das dem Prozeß zugeführte Frischmethanol sowie gelöstes Methylnitrit enthält. Der genannte Strom (f5) wird mit dem aus nachgeführtem Inertgas, vorzugsweise und wie in Fig. 7 abgebildet Kohlendioxid (s), und nachgeführtem frischem Stickoxid (in Fig. 7 Stickstoffmonoxid) (r) zusammengesetzten Strom (s1) vereinigt, bevor er als Feedgasstrom (f6) in die Herstellung des Methylnitrits einfließt. Ferner wird am Kopf der genannten Apparatur 2 ein flüssiger Strom (f7) abgenommen, der in die apparative Anordnung 8 geleitet wird (vgl. Beschreibung unten).

Die ausgeschleuste Teilmenge des Kreisgases (f2) kann, anstatt wie in der weiter unten beschriebenen Ausführungsform, die den Einsatz der apparativen Anordnung 8 beinhaltet, gegebenenfalls auch einer Behandlung durch andere geeignete nachgeschaltete Operationen, wie sie beispielsweise in DOS 3 834 065 beschrieben werden, unterzogen werden. Eine derartige Nachbehandlung dient für gewöhnlich insbesondere dem Ziel, in der ausgeschleusten Teilmenge (f2) des Kreisgasstroms verbliebene Wertstoffe, wie beispielsweise unumgesetztes Methylnitrit, Stickstoffmonoxid oder gasförmiges Dimethylcarbonat, zurückzugewinnen und in den Prozeß der Dimethylcarbonat-Herstellung zurückzuführen. Auf diese Weise wird zugleich die Abgabe giftiger gasförmiger Stoffe, wie beispielsweise Methylnitrit oder Stickstoffmonoxid, an die Umwelt vermieden. In diesem Sinne ist das erfindungsgemäße Verfahren nur beispielhaft und nicht notwendigerweise und unauflösbar mit der Abgasaufarbeitung/Wertstoffrückführung/Nebenproduktausschleusung, wie sie mit Hilfe der apparativen Anordnung 8 gelingt, verbunden, sondern läßt sich grundsätzlich auch mit anderen denkbaren Ausführungsformen der Abgas- und Prozeßgasbehandlung kombinieren.

Die Bauweise der Apparatur 2 gleicht der oben im Zusammenhang mit Fig. 3 beschriebenen, ebenso ihre Betriebsweise.

Innerhalb der Apparatur 3 (Methylnitrit-Synthese, vgl. Fig. 7) erfolgt, entsprechend beispielsweise einer der Reaktionsgleichungen (2), (3) oder (4), die Bildung bzw. Rückbildung des Methylnitrits. Dazu werden die als Methylnitrit-Äquivalente bzw. -Vorstufen fungierenden Stickoxide mit Sauerstoff (c2) und Methanol (mit gelöstem Methylnitrit beladenes Methanol aus der apparativen Anordnung 8 sowie Methanol-Rückströme aus Methanol-Abtrennung in Apparatur 7 und aus Abwasser-Destillation in Apparatur 6) (b4, b5, m1, m5, n1) zur Reaktion gebracht. Das dabei entstehende Wasser sowie etwaige gebildete Nebenprodukte, wie etwa Salpetersäure, werden am Boden der genannten Apparatur 3 als Strom (g) abgeführt; das Methylnitrit enthaltende Produktgasgemisch (d) wird nach Passieren eines am Kopf der Apparatur befindlichen Kondensators (nicht in Fig. 7 enthalten) abgeleitet und wieder für die Bildung von Dimethylcarbonat bereitgestellt. Das in die Apparatur 3 eingebrachte Methanol dient somit zum einen als Reaktionspartner bei der gemäß einer der Reaktionsgleichungen (2), (3) oder (4) erfolgenden Bildung von Methylnitrit, zum anderen, insbesondere in Form der am oberen Teil der Apparatur aufgebrachten Teilströme (b4, m1, n1) sowie dem vom Kopfkondensator zurücktropfenden Rücklauf, als Waschflüssigkeit zur Entfernung des gebildeten Wassers. Bei den zur Umsetzung gelangenden Stickoxiden handelt es sich im wesentlichen um Stickstoffmonoxid, das im Zuge der Bildung von Dimethylcarbonat gemäß Reaktionsgleichung (1) freigesetzt und, im Gemisch mit weiteren gasförmigen Komponenten, wie beispielsweise dem Inertgas und gegebenenfalls nicht vollständig umgesetzten gasförmigen Reaktanden, wie etwa Kohlenmonoxid oder Methylnitrit selbst, im Sinne eines Kreisprozesses (vgl. auch Fig. 1) gemäß Reaktionsgleichung (2) zurückgeführt wird, wobei der Weg (f->f1->f3->f4->f5) durchlaufen wird. Grundsätzlich möglich Verluste an innerhalb des gesamten Kreisprozesses vorhandenem Methylnitrit selbst oder an Methylnitrit-Äquivalenten bzw. -Vorstufen, namentlich an Stickstoffmonoxid, Stickstoffdioxid, Distickstofftrioxid oder Distickstofftetroxid, können sich durch unerwünschte Nebenreaktionen, wie sie grundsätzlich innerhalb der Apparatur 3 auftreten können, oder auch durch Ausschleusungen (p, t, v, w1,) ergeben. Bei den genannten unerwünschten Nebenreaktionen kann es sich beispielsweise um den im untergeordneten Ausmaß ablaufenden Prozeß der Bildung von Salpetersäure gemäß Reaktionsgleichung (4) oder um die eventuelle Bildung von Stickstoff oder Distickstoffmonoxid aus Stickstoffmonoxid handeln, wie sie sich etwa gemäß den Reaktionsgleichungen (12) und (13) (s. oben) formulieren läßt.

Verluste der genannten Art lassen sich beispielsweise dadurch ausgleichen, daß dem Gesamtprozeß das fehlende Methylnitrit selbst, das sich in einer vorgeschalteten Apparatur auf an sich bekannte Weise herstellen läßt, oder auch entsprechende Mengen an Methylnitrit-Äquivalenten oder -Vorstufen, namentlich Stickstoffmonoxid, Stickstoffdioxid, Distickstofftrioxid, Distickstofftetroxid oder Gemische einzelner dieser Komponenten, absatzweise oder kontinuierlich, bevorzugt kontinuierlich, zugesetzt werden. In Fig. 7 geschieht dies beispielsweise durch die gemeinsame Zuspeisung des Inertgases Kohlendioxid und Stickstoffmonoxid (s1), jedoch ist das erfindungsgemäße Verfahren nicht auf diese Ausführungsform beschränkt.

Im allgemeinen ist es unter ökologischen Gesichtspunkten nicht akzeptabel, Salpetersäure enthaltende Abwässer, auch wenn es sich dabei nur um geringe Mengen handelt, ohne Nachbehandlung freizusetzen. Das erfindungsgemäße Verfahren beinhaltet aus diesem Grund einen bevorzugt kontinuierlich betriebenen Neutralisationsschritt, bei dem eine geeignete Base zum Abfangen gegebenenfalls gebildeter Salpetersäure verwendet wird. In Fig. 7 wird dazu beispielsweise Natronlauge verwendet, es kommen aber auch andere Basen, wie etwa Kalilauge, Kalkmilch oder wäßrige Lösungen von Natriumcarbonat und Natriumhydrogencarbonat, in Frage. Nach Zumischung der betreffenden Base, beispielsweise Natronlauge (q) zu dem Bodenstrom (g) der Apparatur 3 wird somit ein neutralisierter, im wesentlichen Wasser und Methanol enthaltener Strom (g1) erhalten, der an Apparatur 6 (Abwasser-Destillation) weitergegeben wird.

Die Bauform der Apparatur 3 ist die in Verbindung mit Fig. 3 beschriebene.

Die Zuspeisung von Sauerstoff (c2) erfolgt bevorzugt in den unteren Teil der Apparatur 3 hinein. Ein Teilstrom (b4) des aus der Apparatur 8 herbeigeführten und mit gelöstem Methylnitrit beladenen Stroms (b1), der das dem Prozeß zugeführte Methanol enthält, sowie die Methanol-Rückströme (n1) und (m1) werden in flüssiger Form in den oberen Teil des Reaktors eingeleitet. Ein anderer Teil des Methanols (b4) sowie der Methanol enthaltenden Rückströme (m5) werden getrennt oder gemeinsam mit dem aus zurückzuführendem Kreisgas sowie den frisch zugespeisten Stickoxid- und Inertgasströmen gebildeten Gesamtstrom (f6) in den unteren Teil der Apparatur 3 eingespeist. In Fig. 7 ist lediglich eine der erfindungsgemäß möglichen Varianten abgebildet, bei der die Einspeisung des Sauerstoffs (c2), der Methanol-Teilströme (b5) und (m5) sowie des vereinigten Stroms aus zurückzuführenden Kreisgas, neu zugespeistem Inertgas und frisch zugeführtem Stickoxid (f6) aus Gründen der Übersichtlichkeit in getrennter Form vorgenommen wird. Das erfindungsgemäße Verfahren ist jedoch keinesfalls auf diese Ausführungsform beschränkt. Insbesondere im Falle der Verwendung spezieller Einspeiseaggregate, wie insbesondere Zweistoffdüsen und/oder Statikmischern sowie Kombinationen derartiger Elemente, ist es von Vorteil, die Ströme (f6) und/oder (b5) und/oder (m5) sowie den Sauerstoffstrom (c2) gemeinsam in den unteren Teil der Apparatur 3 einzuführen.

In einer bevorzugten Ausführungsform erfolgt die Vermischung der am unteren Ende der genannten Apparatur 3 einzuspeisenden flüssigen und gasförmigen Komponenten bzw. Ströme (b5, c2, f6, m5) vermittels eines effektiv arbeitenden Statikmischers, wie er beispielsweise von der Firma Sulzer (SMX- oder SMV-Typ) vertrieben wird. Der Einsatz derartiger Aggregate wird beispielsweise in Ing. Tech. 51 (5), S. 307 ff. (1979) oder in Chem. Eng. Process 25, S. 59 ff. (1989) beschrieben.

In einer weiteren bevorzugten Ausführungsform verwendet man zur Vermischung der am unteren Ende der

genannten Apparatur 3 einzuspeisenden flüssigen und gasförmigen Komponenten bzw. Stöme (b5, c2, f6, m5) Einstoff- oder Zweistoffdüsen oder Kombinationen aus Statikmischern und Ein- oder Zweistoffdüsen.

Arbeitsweise und Bauform von Apparaturen 4 und 5 entsprechen der Beschreibung im Zusammenhang mit Fig. 3.

Bei Apparatur 6 (Abwasser-Destillation, vgl. Fig. 7) handelt es sich um eine Destillationskolonne, die mit dem neutralisierten Bodenablauf (g1) der Apparatur 3 (Methylnitrit-Synthese) sowie mit dem Strom (u) aus der apparativen Anordnung 8 (Bodenstrom des Leichtsieder-Wäschers 8c der Abgasaufarbeitung/Wertstoffrückführung/Nebenproduktausschleusung, vgl. Fig. 9) beschickt wird. Aufgabe dieser Kolonne ist im wesentlichen die Abtrennung und Rückführung des in dem genannten Strom (g1) enthaltenen Methanols und gegebenenfalls noch vorhandenen Dimethylcarbonats als am oberen Teil entnommener Seitenstrom (m), der in Form der Teilströme (m1) und (m5) in die Apparatur 3 (Methylnitrit-Synthese), als Teilstrom (m3) in die Apparatur 7 (Methanol-Destillation) und gegebenenfalls auch als Teilstrom (m6), mit dem Strom (h) zu dem Strom (h1) vereinigt, in die Apparatur 4 (Druckdestillation) zurückgeführt wird. Am Boden der Apparatur 6 fällt das eigentliche Abwasser des Gesamtprozesses (p) an.

Für die genannte Apparatur 6 kommen im allgemeinen Destillationskolonnen in Frage, wie sie weiter oben für die Apparaturen 4 und 5 beschrieben wurden; eine druckfeste Ausführung ist jedoch nicht erforderlich.

Je nach gewählten Betriebsbedingungen, bei Störungen durch schwankende Qualitäten der Einsatzstoffe oder aus anderen Gründen kann es sinnvoll sein, aus dem oberen Teil der Apparatur 6 einen Teilstrom (w) (vgl. Fig. 7) abzuziehen und einer besonderen Behandlung, zum Beispiel einer Nebenproduktabtrennung, zu unterziehen (nicht Gegenstand der in Fig. 7 beschriebenen Verfahrensverschaltung), wobei die bei dieser Aufarbeitung zurückgewonnen Wertstoffe an geeigneter anderer Stelle gegebenenfalls wieder in den Prozeß eingespeist werden können.

Bei Apparatur 7 (Methanol-Abtrennung, vgl. Fig. 7) handelt es sich um eine unter Normaldruck oder unter vermindertem Druck betriebene Destillationskolonne. Sie dient der Aufspaltung des als Kopfprodukt der Destillationskolonne 4 anfallenden Stromes (k) in ein aus Methanol und Dimethylcarbonat bestehendes Azeotrop (1), das am Kopf der Apparatur entnommen wird und wieder in die Destillationskolonne 4 zurückgeführt wird, sowie einen im wesentlichen aus Methanol bestehenden Bodenablauf (n), der als Teilstrom (n1) in den oberen Teil des Methylnitrit-Reaktors (Apparatur 3) und als Teilstrom (n2) in die apparative Anordnung 8 (Abgasaufarbeitung/Wertstoffrückführung/Nebenproduktausschleusung) weitergeleitet wird. Zusätzlich gespeist wird sie durch den der Abwasser-Destillation entstammenden, Methanol enthaltenden Teilstrom (m3).

Je nach Betriebsweise, in Abhängigkeit von Störungen durch schwankende Qualitäten der Einsatzstoffe oder aus anderen Gründen kann es sinnvoll sein, das aus Methanol und Dimethylcarbonat bestehende Azeotrop als Strom (k) aus dem oberen Bereich der Apparatur 7 abzuziehen, zusätzlich aber am Kopf der genannten Apparatur einen gasförmigen Strom (z) zu entnehmen, der besonders reich an leichtsiedenden Nebenprodukten ist und der gegebenenfalls einer weiteren Aufarbeitung zugeführt werden kann (vgl. auch Beschreibung von Apparatur 6, dort Strom (w)). Diese Variante wurde auch in Fig. 7 aufgenommen.

Für die genannte Apparatur 7 kommen im allgemeinen Destillationskolonnen in Frage, wie sie für die Apparaturen 4, 5 bzw. 6 genannt wurden; sie sind jedoch für einen Betrieb unter vermindertem Druck eingerichtet.

In Fig. 8 ist in Form eines Blockdiagramms beispielshaft wiedergegeben, wie der Aufgabenkomplex Abgasaufarbeitung/Wertstoffrückführung/Nebenproduktausschleusung mit Hilfe der apparativen Anordnung 8 gelöst werden kann. Ebenfalls nur beispielhaft ist in Fig. 9 abgebildet, wie sich dieses Konzept konkret realisieren läßt. Hierbei handelt es sich um einzelne oder gegebenenfalls auch teilweise zusammengeschaltete Apparaturen.

Der aus dem die Apparatur 2 verlassenden Kreisgasstrom (f) abgezweigte Teilstrom (f2) wird zunächst in den unteren Bereich eines Leichtsieder-Wäschers (Apparatur 8c, vgl. Fig. 8 und 9) eingeleitet, in dessen oberen Bereich ein Teilstrom (x2) des dem Prozeß frisch zugeführten Methanols (b) und/oder (nicht in Fig. 7 bzw. Fig. 9 enthalten) ein Teil aus der Apparatur 7 herbeigeführten Stroms (n2) eingespeist wird. Der am Boden der genannten Apparatur 8c austretende Strom (u) wird der Abwasserkolonne (Apparatur 6 in Fig. 7) zugeleitet, während das am Kopf der Apparatur anfallende gasförmige Gemisch als Strom (x5) in den unteren Bereich der Apparatur 8b (Methylnitrit-Nachreaktor/Methanol-Abgaswäscher, Beschreibung siehe unten) eingespeist wird.

Bei der Apparatur 8c handelt es sich beispielsweise um einen gegebenenfalls mit einem Kopfkondensator ausgerüsteten kolonnenartigen Wäscher, der zur Verbesserung des Wärme- und Stoffaustauschs mit Einbauten, wie sie üblicherweise bei thermischen Trennaufgaben verwendet werden, versehen ist und innerhalb dessen eine Zahl von mehr als zwei theoretischen Trennstufen realisiert wird. Als derartige Einbauten genannt seien beispielsweise Füllkörper, Böden, wie etwa Glockenböden, Siebböden oder Ventilböden, geordnete Packungen oder Spritzdüsen.

Gegebenenfalls ist der untere Bereich der Apparatur mit einem Sumpfverdampfer ausgestattet, mit dessen Hilfe eine definierte Sumpftemperatur in durch die Phasengleichgewichte der im Sumpf vorhandenen Stoffe bestimmten gewissen Grenzen eingestellt werden kann.

Der Hauptteil des Kreisgasstroms (f4) wird in den unteren Bereich der Apparatur 8a (Methylnitrit-Desorber, vgl. Fig. 8 und 9) eingeleitet, in deren oberen Bereich der Methanol sowie gelöstes Methylnitrit enthaltende Teilstrom (x9), der aus der Apparatur 8b herbeigeführt wird, eingespeist wird. Am Kopf der genannten Appparatur 8a wird der Strom (f5) erhalten, der an die Apparatur 3 weitergegeben wird, während der anfallende Bodenstrom (x6) in die beiden Teilströme (x7) und (x8) aufgeteilt wird. Teilstrom (x7) wird mit dem aus Apparatur 2 herbeigeführten Strom (f7) zu dem Strom

(x10) vereinigt und direkt in die Apparatur 8b eingespeist (Beschreibung siehe unten), während Teilstrom (x8) mit dem aus Apparatur 7 herbeigeführten Teilstrom (n2) zu dem Strom (x3) vereinigt wird. Dieser wird, gegebenenfalls nach Passieren eines Wärmetauschers, der die Einstellung einer definierten Eintrittstemperatur erlaubt, als Strom (x4) in die Apparatur 8b eingeleitet (Beschreibung siehe unten).

Für die Apparatur 8a kommen, gemäß ihrer Funktion als Methylnitrit-Desorber, grundsätzlich zahlreiche Geräte verschiedenster Bauart in Frage. Beispielsweise kann es sich bei der Apparatur 8a um einen Rohrbündelwärmeaustauscher, der in stehender oder liegender Bauweise ausgeführt sein kann, um einen Fallfilmverdampfer, einen Einsprühkondensator, einen mit einem Kopfkondensator und/oder einem Sumpfverdampfer versehenen Wäscher, einen Rippenrohrwärmeaustauscher oder um eine Kombination der genannten Kondensator- bzw. Wärmeaustauscher-und Wäschertypen, etwa um einen Einspritzkondensator mit nachgeschaltetem Rippenrohrwärmeaustauscher, handeln. Ferner kommen für die genannte Apparatur 8a auch Destillationskolonnen in Frage, die üblicherweise für die thermische Trennung von Stoffgemischen unter Normaldruck oder erhöhtem Druck Verwendung finden, die gegebenenfalls über einen Abtriebs- und einen Verstärkerteil verfügen und die, dem Trennproblem entsprechend, die erforderliche Anzahl theoretischer Trennstufen besitzen. Beispielsweise genannt seien Bodenkolonnen, Füllkörperkolonnen sowie Kolonnen, die als Einbauten geordnete Packungen enthalten. Bevorzugt genannt seien Füllkörperkolonnen sowie Kolonnen, die geordnete Packungen als Einbauten enthalten.

Die Apparatur 8b fungiert als Methylnitrit-Nachreaktor und Methanol-Abgaswäscher (vgl. Fig. 8 und 9). Sie läßt sich, wie in Fig. 9 beschrieben und nachfolgend erläutert, als konstruktive Einheit realisieren, gegebenenfalls aber auch in Form zweier voneinander getrennter Apparate.

Der höher angeordnete Teil der genannten Apparatur 8b (Methanol-Abgaswäscher-Teil) fungiert im wesentlichen als Wäscher. In seinen oberen Bereich werden die überwiegend Methanol enthaltenden Ströme (x1) sowie (x4) eingespeist. Im mittleren Bereich der genannten Apparatur 8b befinden sich geeignete Einbauten, von denen sich die dort ansammelnde Flüssigkeit ganz oder teilweise als Strom (x9) abziehen und in die Apparatur 8a leiten läßt. Aus dem unteren Teil der genannten Apparatur 8b (Methylnitrit-Nachreaktor-Teil) aufsteigendes Gas wird mit dem in den mittleren Bereich der genannten Apparatur 8b eingebrachten Strom (x10) gewaschen, wodurch in ihm enthaltene Wertstoffe herausgelöst und in den Prozeß zurückgeführt werden können. Inerte Gase, wie beispielsweise Methan, Wasserstoff, sowie etwaige tiefsiedende Nebenprodukte, wie etwa Methylchlorid, verlassen die Gesamtapparatur als Kopfstrom (t) und können beispielsweise der Abgasverbrennung zugeführt werden.

Der untere Teil der genannten Apparatur 8b fungiert im wesentlichen als Methylnitrit-Reaktor, ähnlich der weiter oben beschriebenen Apparatur 3. Der Stickstoffmonoxid enthaltende, von der Apparatur 8c herbeigeleitete Gasstrom (x5) wird ebenso wie der Sauerstoffstrom (c1) in den unteren Bereich des Methylnitrit-Nachreaktor-Teils eingespeist, während der Methanol enthaltende Strom (x10) in den oberen Bereich, mithin etwa in den mittleren Bereich der Gesamtapparatur, eingebracht wird. Der am Boden der Gesamtapparatur anfallende Strom (b1) wird an die Apparaturen 2 und 3 (vgl. Fig. 7) weitergegeben.

Bei dem oberen Teil der genannten Apparatur 8b (Methanol-Abgaswäscher-Teil) handelt es sich beispielsweise um einen gegebenenfalls mit einem Kopfkondensator ausgerüsteten kolonnenartigen Wäscher, der zur Verbesserung des Wärme- und Stoffaustauschs mit Einbauten, wie sie üblicherweise bei thermischen Trennaufgaben verwendet werden, versehen ist und innerhalb dessen eine Zahl von mehr als zwei theoretischen Trennstufen realisiert wird. Als derartige Einbauten genannt seien beispielsweise Füllkörper, Böden, wie etwa Glockenböden, Siebböden oder Ventilböden, geordnete Packungen oder Spritzdüsen.

Bei dem unteren Teil der genannten Apparatur 8b (Methylnitrit-Nachreaktor) handelt es sich beispielsweise um einen kolonnenartigen Wäscher, der zur Verbesserung des Wärme- und Stoffaustauschs mit Einbauten, wie sie üblicherweise bei thermischen Trennaufgaben verwendet werden, versehen ist. Als derartige Einbauten genannt seien beispielsweise Füllkörper, Böden, wie etwa Glockenböden, Siebböden oder Ventilböden, geordnete Packungen oder Spritzdüsen.

Gegebenenfalls ist der untere Bereich der Apparatur mit einem Sumpfverdampfer ausgestattet, mit dessen Hilfe eine definierte Sumpftemperatur in durch die Phasengleichgewichte der im Sumpf vorhandenen Stoffe bestimmten gewissen Grenzen eingestellt werden kann.

Die Einspeisung der gasförmigen Ströme (c1) und (x4) in den Methylnitrit-Nachreaktor-Teil der Apparatur 8b kann in getrennter oder vermischter Form geschehen. Grundsätzlich geeignet und einsetzbar für die Vermischung der am unteren Ende der genannten Apparatur 8b einzuspeisenden gasförmigen Komponenten bzw. Ströme (c1, x5) sind Statikmischer, Strahlmischer, rotierende Mischer, Einstoffdüsen, Wirbelschichtmischer, wie sie beispielsweise von der Firma Sulzer vertrieben werden, Mischkammern, wie sie beispielsweise von der Firma Pfaudler vertrieben werden, In Line - Vollturbulenzrohre, HI-Mischer, wie sie beispielsweise von der Firma Toray vertrieben werden, Komax-Mischelemente, Wendelmischer, Kennix-Mischer, mit Füllkörpern, wie beispielsweise Raschig-Ringen, befüllte Rohre sowie Kombinationen derartiger Elemente. Die Zuspeisung von Sauerstoff (c1) erfolgt bevorzugt in den unteren Teil der Apparatur 8b hinein.

Im folgenden werden die in Fig. 7, 8 und 9 beschriebenen Stoffstöme sowie die zugehörigen Druck- und Temperaturbedingungen, wie sie bei Durchführung des erfindungsgemäßen Verfahrens realisiert werden, angegeben.

Der im kontinuierlichen Betrieb in die Apparatur 1 eingeleitete Gasstrom (d2) besitzt im allgemeinen eine Temperatur von 25 bis 120°C, vorzugsweise von 50 bis 110°C und einen Druck von 1 bis 5 bar, vorzugsweise 1,5 bis 4 bar. Er enthält im allgemeinen 4 bis 25 mol-% Kohlenmonoxid, 30 bis 80 mol-% Kohlendioxid, 5 bis 40 mol-% Methylnitrit, 0 bis 5 mol-% Stickstoff, 1 bis 10 mol-% Methanol, 0 bis 5 mol-% Dimethylcarbonat, 0,1 bis 5,0 mol-% Stickstoffmonoxid, 0 bis 1 mol-% Wasser und weniger als 15 mol-% diverse zumeist leicht flüchtige Nebenkomponenten, vorzugsweise 5 bis 20 mol-% Kohlenmonoxid, 35 bis 70 mol-% Kohlendioxid, 10 bis 35 mol-% Methylnitrit, 0 bis 4 mol-% Stickstoff, 2 bis 9 mol-% Methanol, 0 bis 4 mol-% Dimethylcarbonat, 0,5 bis 5,0 mol-% Stickstoffmonoxid, 0 bis 0,5 mol-% Wasser und 1 bis 12 mol-% diverse zumeist leicht flüchtige Nebenkomponenten.

Der dem Strom (d2) vor Eintritt in die Apparatur 1 gegebenenfalls beigemischte Strom (y) enthält als Aktivator Halogenwasserstoff und/oder Chlorameisensäuremethylester und/oder Halogen und/oder andere unter den Reaktionsbedingungen aktivierend wirkendes Halogen enthaltende Substanzen, bevorzugt Chlorwasserstoff und/oder Chlor in reiner oder verdünnter Form und wird so dosiert, daß der in die Apparatur 1 eintretende Gasstrom 0 bis 3000 ppm, bevorzugt 10 bis 1000 ppm dieser Komponenten enthält.

Der die Apparatur 1 im kontinuierlichen Betrieb verlassende Gasstrom (e) besitzt im allgemeinen eine Temperatur von 50 bis 170°C, vorzugsweise von 60 bis 160°C und einen Druck von 1 bis 5 bar, vorzugsweise 1,5 bis 4 bar. Er enthält im allgemeinen 0 bis 12 mol-% Kohlenmonoxid, 30 bis 80 mol-% Kohlendioxid, 0 bis 20 mol-% Methylnitrit, 0 bis 5 mol-% Stickstoff, 2 bis 12 mol-% Methanol, 3 bis 25 mol-% Dimethylcarbonat, 5 bis 40 mol-% Stickstoffmonoxid, 0 bis 0,5 mol-% Wasser und weniger als 16 mol-% diverse, zumeist leicht flüchtige Nebenkomponenten, vorzugsweise 1 bis 10 mol-% Kohlenmonoxid, 35 bis 70 mol-% Kohlendioxid, 1 bis 19 mol-% Methylnitrit, 0 bis 5 mol-% Stickstoff, 3 bis 10 mol-% Methanol, 5 bis 20 mol-% Dimethylcarbonat, 5 bis 35 mol-% Stickstoffmonoxid, 0 bis 0,5 mol-% Wasser und weniger als 13 mol-% diverse, zumeist leicht flüchtige Nebenkomponenten.

Der die Apparatur 2 im kontinuierlichen Betrieb verlassende Gasstrom (f) besitzt im allgemeinen eine Temperatur von 0 bis 50°C, vorzugsweise von 5 bis 40°C und einen Druck von 1 bis 5 bar, vorzugsweise 1,5 bis 4 bar. Er enthält im allgemeinen 0 bis 15 mol-% Kohlenmonoxid, 30 bis 80 mol-% Kohlendioxid, 0 bis 20 mol-% Methylnitrit, 0 bis 5 mol-% Stickstoff, 0 bis 12 mol-% Methanol, 0 bis 5 mol-% Dimethylcarbonat, 5 bis 45 mol-% Stickstoffmonoxid, 0 bis 1 mol-% Wasser und weniger als 20 mol-% diverse, zumeist leicht flüchtige Nebenkomponenten, vorzugsweise 1 bis 12 mol-% Kohlenmonoxid, 35 bis 70 mol-% Kohlendioxid, 3 bis 15 mol-% Methylnitrit, 0 bis 5 mol-% Stickstoff, 3 bis 10 mol-% Methanol, 0 bis 3 mol-% Dimethylcarbonat, 5 bis 40 mol-% Stickstoffmonoxid, 0 bis 0,5 mol-% Wasser und weniger als 14 mol-% diverse, zumeist leicht flüchtige Nebenkomponenten.

Die Menge des als Gasstrom (f2) ausgeschleusten Anteils des Kreisgases beträgt im kontinuierlichen Betrieb im allgemeinen 0 bis 7 Gew.-%, bevorzugt 0,1 bis 5 Gew.-%, bezogen auf den gasförmigen Kopfstrom (f) der Apparatur 2.

Der im oberen Bereich der Apparatur 2 abgenommene flüssige Strom (f7) enthält 20 bis 90 Gew.-% Methanol, 10 bis 80 Gew.-% Dimethylcarbonat sowie weniger als 30 Gew.-% weitere, zumeist minder flüchtige, gelöste Komponenten, bevorzugt 25 bis 60 Gew.-% Methanol, 40 bis 75 Gew.-% Dimethylcarbonat sowie weniger als 20 Gew.-% weitere, zumeist minder flüchtige, gelöste Komponenten.

Der nach Ausschleusung des Gasstroms (f2) verbleibende gasförmige Hauptstrom (f1) wird nach Passieren eines Wärmeaustauschers als Strom (f3) sowie nachfolgend eines Verdichters als Strom (f4) der apparativen Anordnung 8 zugeführt. Der genannte Strom (f4) besitzt dann eine Temperatur von 10 bis 70°C, vorzugsweise von 20 bis 60°C, und einen Druck von 1 bis 5 bar, vorzugsweise 1,5 bis 4 bar.

Der aus der apparativen Anordnung 8 zurückgeführte Strom (f5) besitzt im allgemeinen eine Temperatur von 0 bis 70°C, vorzugsweise von 20 bis 60°C und einen Druck von 1 bis 5 bar, vorzugsweise 1,5 bis 4 bar. Er enthält im allgemeinen 0 bis 15 mol-% Kohlenmonoxid, 30 bis 80 mol-% Kohlendioxid, 0 bis 20 mol-% Methylnitrit, 0 bis 5 mol-% Stickstoff, 0 bis 16 mol-% Methanol, 0 bis 5 mol-% Dimethylcarbonat, 5 bis 45 mol-% Stickstoffmonoxid, 0 bis 1 mol-% Wasser und weniger als 20 mol-% diverse, zumeist leicht flüchtige Nebenkomponenten, vorzugsweise 1 bis 12 mol-% Kohlenmonoxid, 35 bis 70 mol-% Kohlendioxid, 3 bis 15 mol-% Methylnitrit, 0 bis 5 mol-% Stickstoff, 3 bis 10 mol-% Methanol, 0 bis 3 mol-% Dimethylcarbonat, 5 bis 40 mol-% Stickstoffmonoxid, 0 bis 0,5 mol-% Wasser und weniger als 14 mol-% diverse, zumeist leicht flüchtige Nebenkomponenten.

Der die Apparatur 2 als Bodenprodukt verlassende Strom (h) hat im allgemeinen eine Temperatur von 10 bis 150°C, vorzugsweise 20 bis 140°C und enthält im allgemeinen 0 bis 40 mol-% Methanol, 20 bis 99 mol-% Dimethylcarbonat sowie gegebenenfalls geringe Mengen Wasser, Schwersieder, wie beispielsweise Oxalsäuredimethylester, und weitere Komponenten in einer Größenordnung von weniger als 5 mol-%.

Die Einspeiserate des im allgemeinen bei Umgebungstemperatur als Strom (r) herbeigeführten gasförmigen Stickstoffmonoxids in dieApparatur 3 (vgl. Fig. 7) beträgt im kontinuierlichen Betrieb 0 bis 3 mol-%, bevorzugt 0 bis 1,5 mol-%, bezogen auf den Gasstrom (f5). Anstelle des Stickstoffmonoxids können grundsätzlich auch äquivalente Mengen Stickstoffdioxid, Distickstofftrioxid, Distickstofftetroxid, Methylnitrit oder beliebige Gemische dieser Substanzen zugespeist werden.

Die Einspeiserate des im allgemeinen bei Umgebungstemperatur als Strom (s) herbeigeführten gasförmigen Kohlendioxids in die Apparatur 3 (vgl. Fig. 7) beträgt im kontinuierlichen Betrieb 0 bis 3 mol-%, bevorzugt 0 bis 1,5 mol-%, bezogen auf den Gasstrom (f5).

Der durch Zusammenfügung der Ströme (s1) und (f5) resultierende Gasstrom (f6) besitzt bei kontinuierlichem Betrieb im allgemeinen eine Temperatur von 0 bis 70°C, vorzugsweise 20 bis 60°C, und einen Druck von 1 bis 5 bar, vorzugsweise 1,5 bis 4 bar.

Der aus der apparativen Anordnung 8 herbeigeführte Strom (b2) wird im allgemeinen so bemessen, daß das Stoffmengenverhältnis zwischen dem darin enthaltenen Methanol und dem im Gasstrom (f6) enthaltenen Stickstoffmonoxid 1 bis 10, vorzugsweise 1 bis 5 beträgt. Der genannte Strom (b2) wird gegebenenfalls in die Teilströme (b4) und (b5) aufgetrennt, wobei der Teilstrom (b4) im allgemeinen 50 bis 100 %, vorzugsweise 70 bis 100 % des Stroms (b2) umfaßt.

In einer speziellen Ausführungsform des erfindungsgemäßen Verfahrens wird die Menge des via Zuleitung (b5) im kontinuierlichen Betrieb eingespeisten Methanols vorteilhaft so gewählt, daß das Stoffmengenverhältnis zwischen diesem Methanol und dem im zugespeisten Gasstrom (f6) enthaltenen Stickstoffmonoxid 0,1 bis 2,5, bevorzugt 0,1 bis 1,5 beträgt.

Im kontinuierlichen Betrieb wird die Menge des im allgemeinen bei Umgebungstemperatur via Zuleitung (c2) herbeigeführten Sauerstoffs vorteilhaft so gewählt, daß das Stoffmengenverhältnis zwischen Sauerstoff und dem im zugespeisten Gasstrom (f6) enthaltenen Stickstoffmonoxid 0,15 bis 0,26, bevorzugt 0,20 bis 0,25 beträgt.

Der Innendruck der genannten Apparatur 3 beträgt im allgemeinen 1 bis 5 bar, bevorzugt 1,5 bis 4 bar, ihre Innentemperatur ist in weiten Grenzen variabel. Das Temperaturprofil über die gesamte Länge der Apparatur stellt sich in Abhängigkeit von Menge, Eintrittstemperatur und Aggregatzustand der Einzelzuspeisungen, Gesamtdruck, Umsatz der eingebrachten Reaktanden, Rücklauf des Kopfkondensators sowie Energiezufuhr am Sumpf der Apparatur ein.

Der die Apparatur 3 im kontinuierlichen Betrieb als Bodenprodukt verlassende flüssige Strom (g) hat im allgemeinen eine Temperatur von 10 bis 150°C, bevorzugt 20 bis 140°C, und enthält im allgemeinen 0 bis 70 mol-% Methanol, 10 bis 80 mol-% Wasser, 0 bis 20 mol-% Dimethylcarbonat sowie gegebenenfalls geringe Mengen Salpetersäure und weitere gelöste Komponenten.

Der im allgemeinen bei Umgebungstemperatur herbeigeleitete, die zur Neutralisation bestimmte Base enthaltende Strom (q) wird im allgemeinen so bemessen, daß er die im genannten Strom (g) enthaltene Säure zu neutralisieren vermag.

Der die Apparatur 3 im kontinuierlichen Betrieb als Kopfprodukt verlassende Strom (d) hat im allgemeinen eine Temperatur von 0 bis 60°C, bevorzugt 10 bis 50°C, einen Druck von 1 bis 5 bar, bevorzugt 1,5 bis 4 bar und enthält im allgemeinen 0 bis 20 mol-% Kohlenmonoxid, 30 bis 80 mol-% Kohlendioxid, 5 bis 40 mol-% Methylnitrit, 0 bis 5 mol-% Stickstoff, 1 bis 12 mol-% Methanol, 0 bis 5 mol-% Dimethylcarbonat, 0,1 bis 5 mol-% Stickstoffmonoxid, 0 bis 1 mol-% Wasser und weniger als 15 mol-% diverse, zumeist leicht flüchtige Nebenkomponenten, vorzugsweise 5 bis 15 mol-% Kohlenmonoxid, 35 bis 70 mol-% Kohlendioxid, 10 bis 35 mol-% Methylnitrit, 0 bis 4 mol-% Stickstoff, 2 bis 9 mol-% Methanol, 0 bis 4 mol-% Dimethylcarbonat, 0,5 bis 5 mol-% Stickstoffmonoxid, 0 bis 0,5 mol-% Wasser und 1 bis 12 mol-% diverse, zumeist leicht flüchtige Nebenkomponenten.

Der genannte Strom (d) wird gegebenenfalls mit Hilfe eines Wärmeaustauschers auf die für seine Einspeisung in die Apparatur 1 gewünschte Temperatur gebracht. Das dem dermaßen erhaltenen Strom (d1) im allgemeinen bei Umgebungstemperatur als Strom (a) zugespeiste Kohlenmonoxid wird im allgemeinen in chemisch reiner Form eingesetzt, kann jedoch je nach Herstellungsart Fremdgase, wie beispielsweise geringe Mengen Wasserstoff (< 0,1 mol-%) oder Methan (<0,1 mol-%) enthalten. Seine Zuspeisung erfolgt im kontinuierlichen Betrieb in einer Weise, daß das Stoffmengenverhältnis zwischen zudosiertem Kohlenmonoxid und produziertem Dimethylcarbonat 1 bis 1,2 beträgt und eine zeitlich konstante Konzentration des Kohlenmonoxids im Gasstrom (d2) vorliegt.

Die Apparatur 6 (Abwasser-Destillation) wird im allgemeinen bei einem Druck von 0,5 bis 2 bar, bevorzugt 0,5 bis 1,5 bar betrieben. Der im oberen Bereich dieser Apparatur abgenommene flüssige Strom (m) fällt im kontinuierlichen Betrieb mit einer Temperatur von 25 bis 80°C, bevorzugt 40 bis 75°C an und enthält im allgemeinen 60 bis 95 mol-% Methanol, 1 bis 35 mol-% Dimethylcarbonat und 0 bis 7 mol-% Wasser, vorzugsweise 70 bis 95 mol-% Methanol, 2 bis 30 mol-% Dimethylcarbonat und 0 bis 5 mol-% Wasser.

Die Menge des als Teilstrom (m3) des genannten Stroms (m) zurückgeführten flüssigen Gemisches kann, je nach Gehalt an Dimethylcarbonat, in weiten Grenzen so gewählt werden, daß sie 0 bis 100 %, bevorzugt 30 bis 100 % des gesamten Stroms (m) umfaßt.

Die Menge des als Teilströme (m1) und (m5) in den oberen und/oder den unteren Bereich der Apparatur 3 geleiteten flüssigen Gemisches kann, je nach Gehalt an Dimethylcarbonat, in weiten Grenzen so gewählt werden, daß sie 0 bis 90 %, vorzugsweise 0 bis 30 % des gesamten Stroms (m) umfaßt. Gleiches gilt für die Menge des als Teilstrom (m6) zurückgeführten flüssigen Gemisches.

Der die Apparatur 6 verlassende gasförmige Strom (v) enthält im allgemeinen Kohlendioxid sowie geringe Mengen Methanol, Dimethylcarbonat, Methylnitrit und diverse Leichtsieder. Er kann gegebenenfalls einer weiteren Aufarbeitung mit dem Ziel der Rückgewinnung darin noch enthaltener Wertstoffe unterworfen werden.

Der im oberen Bereich der genannten Apparatur 6 abgenommene flüssige Strom (w) hat im kontinuierlichen Betrieb eine Temperatur von 10 bis 80°C, bevorzugt 20 bis 70°C und enthält neben Methanol und geringen Mengen Dimethylcarbonat und Wasser vor allem Leichtsieder, wie beispielsweise Formaldehyddimethylacetal und Ameisensäuremethylester. Er wird gegebenenfalls, ähnlich wie der aus Apparatur 7 herbeigeführte Strom (z), einer weiteren, gegebenenfalls gemeinsamen Aufarbeitung (vgl. Ströme (z), (w), (w1) in Fig. 7) mit dem Ziel der Rückgewinnung darin noch enthaltener Wertstoffe unterworfen.

Der in den oberen Bereich der genannten Apparatur 6 eingeleitete und aus der apparativen Anordnung 8 herbeigeführte Strom (u) besitzt im allgemeinen eine Temperatur von -15 bis 30°C, bevorzugt -10 bis 15°C an und enthält 60 bis 99 mol-% Methanol, 0 bis 10 mol-% Dimethylcarbonat, 0 bis 10 mol-% Methylnitrit und 0 bis 5 mol-% Wasser sowie gegebenenfalls geringe Mengen weiterer leichtsiedender Komponenten in der Größenordnung von 0 bis 10 mol-%, vorzugsweise von 70 bis 98 mol-% Methanol, 0 bis 5 mol-% Dimethylcarbonat, 1 bis 7 mol-% Methylnitrit und 0 bis 3 mol-% Wasser sowie gegebenenfalls geringe Mengen weiterer leichtsiedender Komponenten in der Größenordnung von 0 bis 6 mol-%.

Der die genannte Apparatur 6 als Bodenprodukt verlassende Strom (p) enthält im wesentlichen das Prozeßabwasser sowie geringe Mengen darin gelöster und der Neutralisation des Bodenstroms (g) der Apparatur 3 entstammende Salze.

Der die Apparatur 2 als Bodenprodukt verlassende Strom (h) wird mit dem aus Apparatur 6 herbeigeführten Strom (m6) zum Strom (h1) vereinigt und in die Apparatur 4 eingeleitet. Die genannte Apparatur 4 wird, in Abhängigkeit von dem gewählten Innendruck, im allgemeinen in einem Temperaturbereich von 90 bis 240°C betrieben.

Der in die genannte Apparatur 4 eingespeiste und aus Apparatur 7 herbeigeführte flüssige Strom (1) besitzt eine Temperatur von 40 bis 90°C, bevorzugt 50 bis 80°C und enthält 70 bis 95 mol-% Methanol, 5 bis 30 % Dimethylcarbonat sowie geringe Mengen leicht siedender Komponenten in der Größenordnung von im allgemeinen weniger als 5 mol-%.

Der die Apparatur 4 als Bodenprodukt verlassende Strom (i) hat im allgemeinen eine Temperatur von 90 bis 240°C, vorzugsweise von 90 bis 190°C und enthält im allgemeinen weniger als 0,1 mol-% Methanol, bevorzugt weniger als 0,05 mol-% Methanol, mehr als 90 mol-% Dimethylcarbonat, bevorzugt mehr als 95 mol-% Dimethylcarbonat, sowie gegebenenfalls geringe Mengen Schwersieder, wie beispielsweise Oxalsäuredimethylester und weitere Komponenten in einer Größenordnung von insgesamt weniger als 10 mol-%, bevorzugt weniger als 5 mol-%.

Der die Apparatur 4 als Kopfprodukt verlassende Strom (k) hat im allgemeinen eine Temperatur von 80 bis 160°C, vorzugsweise von 90 bis 140°C und enthält im allgemeinen 55 bis 97 mol-% Methanol, 2 bis 35 mol-% Dimethylcarbonat, 0 bis 15 mol-% Wasser sowie gegebenenfalls geringe Mengen Leichtsieder.

Die genannte Apparatur 7 (Methanol-Destillation) wird in einem Druckbereich von 0,2 bis 1,5 bar, vorzugsweise 0,4 bis 1 bar, betrieben. Die Betriebstemperatur liegt in Abhängigkeit von dem gewählten Innendruck im allgemeinen im Bereich von 20 bis 80°C, bevorzugt 40 bis 70°C.

Der die Apparatur 7 verlassende gasförmige Strom (z) enthält im allgemeinen Methanol sowie geringere Mengen Kohlendioxid, Dimethylcarbonat und diverse Leichtsieder, wie beispielsweise Ameisensäuremethylester und Formaldehyddimethylacetal.

Der die Apparatur 7 als Bodenprodukt verlassende Strom (n) liegt hinsichtlich seiner Temperatur im Bereich 20 bis 80°C, vorzugsweise 40 bis 70°C und enthält im allgemeinen mehr als 80 mol-% Methanol, bevorzugt mehr als 90 mol-% Methanol, sowie geringe Mengen Dimethylcarbonat und Wasser. Der genannte Strom (n) wird gegebenenfalls in zwei Teilströme (n1) und (n2) aufgeteilt, wobei (n1) etwa 0 bis 30 %, bevorzugt 0 bis 20 % des Gesamtstroms (n) beträgt.

Der die Apparatur 4 verlassende Strom (i) wird mit Hilfe eines Wärmeaustauschers auf die zur Beschickung der Apparatur 5 (Dimethylcarbonat-Destillation) gewünschte Temperatur gebracht. Der so erhaltene Strom (i1) hat eine Temperatur von 20 bis 180°C, bevorzugt von 40 bis 170°C. Die genannte Apparatur 5 wird im allgemeinen im Druckbereich von 0,75 bis 1,25 bar, bevorzugt bei ca. 1 bar, betrieben.

Das in Apparatur 5 destillierte Dimethylcarbonat (o) hat, je nach Rücklauf/Entnahme-Verhältnis und nach Trennstufenzahl, eine Reinheit von 99,0 bis 99,9 %.

Das in Apparatur 5 anfallende Bodenprodukt enthält im wesentlichen Schwersieder, die verworfen oder einer weiteren Aufarbeitung zugeführt werden.

Das dem Gesamtprozeß im allgemeinen bei Umgebungstemperatur zugeführte Frischmethanol (b) wird in die flüssigen Teilströme (x1) und (x2) zerlegt, wobei der Anteil des Teilstroms (x1) 50 bis 100 % des Gesamtstroms (b) umfaßt. Der Teilstom (x2) wird in den oberen Bereich der Apparatur 8c (Leichtsieder-Wäscher), die im Temperaturbereich -15 bis 30°C, vorzugsweise -10 bis 15°C, und im Druckbereich 1 bis 5 bar, vorzugsweise 1,5 bis 4 bar, betrieben wird, eingebracht.

Der die genannte Apparatur 8c verlassende Strom (x5) besitzt im allgemeinen eine Temperatur von -15 bis 30°C, vorzugsweise -10 bis 15°C und enthält 0 bis 15 mol-% Kohlenmonoxid, 30 bis 80 mol-% Kohlendioxid, 0 bis 20 mol-% Methylnitrit, 0 bis 5 mol-% Stickstoff, 0 bis 12 mol-% Methanol, 0 bis 3 mol-% Dimethylcarbonat, 5 bis 45 mol-% Stickstoffmonoxid, 0 bis 0,5 mol-% Wasser und weniger als 15 mol-% diverse, zumeist leicht flüchtige Nebenkomponenten, vorzugsweise 1 bis 12 mol-% Kohlenmonoxid, 35 bis 70 mol-% Kohlendioxid, 0 bis 7 mol-% Methylnitrit, 0 bis 5 mol-% Stickstoff, 0 bis 7 mol-% Methanol, 0 bis 1 mol-% Dimethylcarbonat, 5 bis 40 mol-% Stickstoffmonoxid, 0 bis 0,5 mol-% Wasser und weniger als 10 mol-% diverse, zumeist leicht flüchtige Nebenkomponenten.

Der oben genannte Teilstrom (x1) des dem Gesamtprozeß zugeführten Frischmethanols wird in den oberen Bereich der Apparatur 8b (Methylnitrit-Nachreaktor/Methanol-Abgaswäscher) eingespeist, die im Temperaturbereich -

10 bis 60°C, vorzugsweise 0 bis 40°C, und im Druckbereich 1 bis 5 bar, vorzugsweise 1,5 bis 4 bar, betrieben wird.

Im kontinuierlichen Betrieb wird die Teilmenge (c1) des dem Gesamtprozeß im allgemeinen bei Umgebungstemperatur via Zuleitung (c) herbeigeführten Sauerstoffs vorteilhaft so gewählt, daß das Stoffmengenverhältnis zwischen Sauerstoff und dem im zugespeisten Gasstrom (x5) enthaltenen Stickstoffmonoxid 0,20 bis 0,30, vorzugsweise 0,23 bis 0,28 beträgt.

Der die Apparatur 8b als Bodenprodukt verlassende Strom (b1) liegt hinsichtlich seiner Temperatur im Bereich 0 bis 60°C, vorzugsweise 0 bis 40°C, und enthält im allgemeinen mehr als 70 mol-% Methanol, bevorzugt mehr als 80 mol-% Methanol, sowie geringe Mengen Dimethylcarbonat, Wasser, Methylnitrit und diverse Leichtsieder. Der genannte Strom (b1) wird gegebenenfalls in zwei Teilströme (b2) und (b3) aufgeteilt, wobei (b2) etwa 70 bis 100 %, bevorzugt 80 bis 100 % des Gesamtstroms (b1) umfaßt.

Bei dem die genannte Apparatur 8b als gasförmiges Kopfprodukt verlassenden Strom (t) handelt es sich um Abgas, das gegebenenfalls einer zusätzlichen Nachbehandlung zugeführt wird. Es enthält im wesentlichen Kohlendioxid und geringe Mengen Kohlenmonoxid, Methanol sowie gegebenenfalls Inertgase und gasförmige Leichtsieder.

Der der Apparatur 8b seitlich entnommene flüssige Strom (x9) enthält im allgemeinen mehr als 70 mol-% Methanol, bevorzugt mehr als 80 % Methanol, sowie geringe Mengen Dimethylcarbonat, Wasser, Methylnitrit und diverse Leichtsieder. Er wird der Apparatur 8a zugeführt.

Die genannte Apparatur 8a (Methylnitrit-Desorber) wird in einem Temperaturbereich von 0 bis 70°C, bevorzugt von 20 bis 60°C, und in einem Druckbereich von 1 bis 5 bar, bevorzugt von 1,5 bis 4 bar betrieben.

Der die Apparatur 8a als Bodenprodukt verlassende Strom (x6) liegt hinsichtlich seiner Temperatur im Bereich 0 bis 70°C, vorzugsweise 20 bis 60°C, und enthält im allgemeinen mehr als 70 mol-% Methanol, bevorzugt mehr als 80-mol-% Methanol, sowie geringe Mengen Dimethylcarbonat, Wasser, Methylnitrit und diverse Leichtsieder. Der Strom (x6) wird gegebenenfalls in zwei Teilströme (x7) und (x8) aufgeteilt, wobei (x7) etwa 70 bis 100 %, bevorzugt 80 bis 100 % des Gesamtstroms (x6) umfaßt.

Der Teilstrom (x8) wird mit dem aus Apparatur 7 herbeigeführten Strom (n2) zum Strom (x3) vereinigt und mit Hilfe eines Wärmeaustauschers auf die zur Einspeisung in den oberen Bereich der Apparatur 8b gewünschte Temperatur gebracht. Der sich dermaßen ergebende Strom (x4) liegt hinsichtlich seiner Temperatur im Arbeitsbereich der Apparatur 8b.

Der Teilstrom (x7) wird mit dem aus Apparatur 2 herbeigeführten Strom (f7) zum Strom (x10) vereinigt und gleichfalls in die Apparatur 8b eingespeist.

Das Kopfprodukt der Druckdestillation kann alternativ auch gemäß Variante (e2) durch Pervaporation oder Dampfpermeation bezüglich der Komponente Methanol, die den wesentlichen Teil des Permeats darstellt, abgereichert werden. Während das Retentat in die genannte Druckdestillation zurückgeführt wird, führt man besagtes Permeat in den Prozeß zurück. Somit ergibt sich eine weitere unter energetischen Gesichtspunkten vorteilhafte und damit ressourcenschonende Einpassung der Druckdestillation sowie der Pervaporation bzw. Dampfpermeation in das Gesamtverfahrenskonzept, sofern man diese Operationen nicht, wie in der zitierten Literatur beschrieben, als isolierte Verfahrensschritte optimiert, sondern, wie es dem erfindungsgemäßen Verfahren entspricht, in Kombination mit Rückführungsschritten für Wertstoffe wie insbesondere das zu recyclisierende Methanol einsetzt.

In Fig. 5 ist lediglich eine der erfindungsgemäßen möglichen Varianten gemäß (e2) abgebildet, bei der die Einspeisung des Sauerstoffs (c), der Methanol-Teilströme (b4) und (n2) sowie des vereinigten Stroms aus zurückführendem Kreisgas, neu zugespeistem Inertgas und frisch zugeführtem Stickoxid (f5) in getrennter Form vorgenommen wird. Das erfindungsgemäße Verfahren ist jedoch keinesfalls auf diese Ausführungsform beschränkt. Insbesondere im Falle der Verwendung spezieller Einspeiseaggregate, wie insbesondere Zweistoffdüsen und/oder Statikmischern sowie Kombinationen derartiger Elemente, ist es von Vorteil, die Ströme (f5) und/oder (b4) und/oder (n2) sowie den Sauerstoffstrom (c) gemeinsam in den unteren Teil der Apparatur 3 einzuführen.

Apparatur 3 in Fig. 5 kann, wie für Fig. 3 beschrieben, betrieben werden. In einer weiteren bevorzugten Ausführungsform verwendet man zur Vermischung der am unteren Ende der genannten Apparatur 3 einzuspeisenden flüssigen und gasförmigen Komponenten bzw. Ströme (b4, c, f5, n2) Einstoff- oder Zweistoffdüsen oder Kombinationen aus Statikmischern und Ein- oder Zweistoffdüsen.

Der Druck, unter dem der Destillationsprozeß innerhalb der Apparatur 4 bei Anwendung der Variante nach (e2) ausgeführt wird, wird so gewählt, daß die Temperatur des Kondensates bzw. der Brüden, die am Kopf der Apparatur als Strom (k) erhalten werden, der bevorzugten Temperatur für die nachgeschaltete, in der Apparatur 7 (Pervaporation oder Dampfpermeation) ablaufende Trennung entspricht. Der Kopfdruck liegt demnach allgemein bei 1 bis 5 bar. Bei Anwendung der Pervaporationstechnik wird der Brüdenstrom (k) kondensiert und der Membran bei optimaler Tempertur zugeführt. Auch in diesem Fall wird der Kolonnendruck entsprechend einreguliert

Bei Apparatur 6 (Abwasser-Destillation, vgl. Fig. 5) handelt es sich wie in Fig. 3 um eine Destillationskolonne, die mit dem neutralisierten Bodenablauf (g1) der Apparatur 3 (Methylnitrit-Synthese) beschickt wird. Aufgabe dieser Kolonne ist die Abtrennung und Rückführung des in dem genannten Strom (g1) enthaltenen Methanols in Form des Kopfstroms (m), der als Teilstrom (m1) in die Apparatur 3 (Methylnitrit-Synthese) und als Teilstrom (m2), mit dem Bodenstrom (h) der Apparatur 2 (Dimethylcarbonat-Kondensator) zu dem Strom (h1) vereinigt, in die Apparatur 4

(Druckdestillation) zurückgeführt wird. Am Boden der Apparatur 6 fällt das eigentliche Abwasser des Gesamtprozesses (p) an. Bezüglich der Verwendung des Methanol-Teilstroms (m2) wird hier eine von Fig. 3 abweichende Variante dargestellt.

Bei Apparatur 7 (Methanol-Abtrennung durch Pervaporation, vgl. Fig. 5) handelt es sich beispielsweise um eine Pervaporationsanlage, wie sie in der Dissertation von M. Francke, 22.06.1990, Rheinisch-Westfälische Technische Hochschule Aachen, Bundesrepublik Deutschland, beschrieben wird. Bei Verwendung spezieller plasmapolymerisierter Membranen, wie sie etwa von der Firma GFT vertrieben werden, gelingt die Abtrennung von Methanol aus Gemischen mit Dimethylcarbonat bei sehr hohen Selektivitäten und Permeatflüssen. Neben dieser hocheffizienten Abtrennung von Methanol aus dem azeotropnahen Gemischen mit Dimethylcarbonat bietet die Pervaporation an derartigen plasmapolymerisierten Membranen den zusätzlichen Vorteil, selbst geringe Mengen gegebenenfalls noch im Strom (k) enthaltenen Wassers auf äußerst effektive Weise zugleich mit dem Methanol entfernen zu können. Auch diesbezüglich sind die Membranen des genannten Typs herkömmlichen Membranen, wie sie beispielsweise in EP 331 846, in US 4 877 529 oder in EP 423 949 beschrieben werden, weit überlegen. Das im Zuge der Pervaporation anfallende Permeat wird als Strom (n), gegebenenfalls nach Aufspaltung in die Teilströme (n1) und (n2), in die Apparatur 3 (Methylnitrit-Synthese) zurückgeführt. Das Retentat, das aus angereichertem Dimethylcarbonat besteht, wird als Strom (1) in die Apparatur 4 zurückgeleitet.

Für die Apparatur 7 kommen im allgemeinen Pervaporationsanlagen in Frage, die in Wickelmodul-, Tubularmodul- oder in Plattenmodul-Bauweise ausgeführt sind.

In einer alternativen Variante verwendet man als Apparatur 7 eine Dampfpermeationsanlage, die gegenüber der Pervaporation Energieeinsparungen sowie den Vorzug einer einfacheren Bauweise bietet. Diese Vorteile resultieren im wesentlichen aus dem Fortfall der bei Pervaporationsanlagen zur Aufbringung der Verdampfungswärmen der permeierenden Komponenten erforderlichen Wärmeaustauscher zwischen den Moduleinheiten (vgl. Dissertation von M. Francke, 22.06.1990, Rheinisch-Westfälische Technische Hochschule Aachen). Auch gelingt bei Verwendung der genannten speziellen plasmapolymerisierten Membranen, wie sie etwa von der Firma GFT vertrieben werden, die Abtrennung von Methanol aus Gemischen mit Dimethylcarbonat bei sehr hohen Selektivitäten und Permeatflüssen. Neben dieser hocheffizienten Abtrennung von Methanol aus dem azeotropnahen Gemischen mit Dimethylcarbonat bietet auch die Dampfpermeation an derartigen plasmapolymerisierten Membranen den überraschenden zusätzlichen Vorteil, selbst geringe Mengen gegebenenfalls noch im Strom (k) enthaltenen Wassers auf äußerst effektive Weise zugleich mit dem Methanol zu entfernen. Das im Zuge der Dampfpermeation anfallende Permeat wird als Strom (n) in die Apparatur 3 (Methylnitrit-Synthese) zurückgeführt. Das Retentat, das aus angereichertem Dimethylcarbonat besteht, wird als Strom (1) in die Apparatur 4 zurückgeleitet.

Die Apparatur 7 (Pervaporation bzw. Dampfpermeation) wird im allgemeinen auf der Permeatseite bei einem Druck von 0,5 bis 500 mbar, vorzugsweise von 1 bis 100 mbar und einer Temperatur von -30 bis +30°C, vorzugsweise -15 bis +10°C betrieben. Das kondensierte Permeat (n) besitzt im kontinuierlichen Betrieb eine Zusammensetzung von etwa 80 bis 97 mol-% Methanol, 0,5 bis 15 % Dimethylcarbonat und 0 bis 7 mol-% Wasser, vorzugsweise 70 bis 88 mol-% Methanol, 2 bis 30 mol-% Dimethylcarbonat und 0 bis 5 mol-% Wasser.

Auf der Retentatseite werden ein Druck von 0,5 bis 10 bar und eine Temperatur von 20 bis 150°C eingestellt. Auf der Retentatseite herrscht stets ein höherer Druck als auf der Permeatseite.

Die Menge des als Teilstrom (n1) zurückgeführten kondensierten Permeats kann, je nach Gehalt an Dimethylcarbonat, so eingestellt werden, daß sie zwischen 0 und 100 % des gesamten Strom (n) umfaßt. Die Menge des als Teilstrom (n2) zurückgeführten kondensierten Permeats kann, je nach Gehalt an Dimethylcarbonat, so eingestellt werden, daß sie zwischen 0 und 90 % des gesamten Stroms (n) beträgt.

Für die im Zusammenhang mit Fig. 5 nicht erwähnten Apparaturen und Stoffströme gilt das oben zu Fig. 3 Gesagte.

Eine weitere Anordnung für die Variante (e2) wird mit Hilfe von Fig. 12 wie folgt beschrieben:

Die Apparaturen 1 und 2 gleichen im Aufbau und Betrieb denen in Fig. 3 bzw. Fig. 5. (y) ist die absatzweise oder kontinuierliche Einspeisung geringer Mengen gasförmiger Hilfsstoffe (z.B. Halogen oder Halogenwasserstoff). Über (b3) wird ein überwiegend Methanol enthaltender Teilstrom aus der apparativen Anordnung 8 (Abgasaufbereitung/Wertstoffrückführung/Nebenproduktausschleusung), nämlich aus (b1) in den oberen Bereich der Apparatur 2 eingespeist.

Der am Kopf der Apparatur 2 erhaltene gasförmige Strom (f) kann nach Abzweigung des für die Ausschleusung bestimmten Anteils (f2) als Kreisstrom (f1) gegebenenfalls an einen Wärmeaustauscher weitergegeben werden (in Fig. 12 enthalten), der die Einstellung der gewünschten Eingangstemperatur für die weitere Umsetzung erlaubt. Der so erwärmte Strom (f3) passiert den Verdichter und wird als Strom (f4) in die apparative Anordnung 8 eingeleitet. Je nach Betriebsweise der Gesamtanlage kann gegebenenfalls auch auf einen derartigen vorgeschalteten Wärmeaustauscher verzichtet werden. Dieser apparativen Anordnung 8, deren Aufbau und Funktion erläutert wurde, wird der Strom (f5) entnommen, der als wesentliche Bestandteile das dem Prozeß zugeführte Frischmethanol sowie gelöstes Methylnitrit enthält. Der genannte Strom (f5) wird mit dem aus nachgeführtem Inertgas, vorzugsweise und wie in Fig. 12 abgebildet, Kohlendioxid (s), und nachgeführtem frischem Stickoxid (in Fig. 12 Stickstoffmonoxid) (r) zusammengesetzten Strom

(s1) vereinigt, bevor er als Feedgasstrom (f6) in die Herstellung des Methylnitrits einfließt. Ferner wird am Kopf der genannten Apparatur 2 ein flüssiger Strom (f7) abgenommen, der in die apparative Anordnung 8 geleitet wird (vgl. Beschreibung oben).

Die ausgeschleuste Teilmenge des Kreisgases (f2) kann, anstatt wie in der weiter unten beschriebenen Ausführungsform, die den Einsatz der apparativen Anordnung 8 beinhaltet, gegebenenfalls auch einer Behandlung durch andere geeignete nachgeschaltete Operationen, wie sie beispielsweise in DOS 3 834 065 beschrieben werden, unterzogen werden (vgl. oben).

Innerhalb der Apparatur 3 (Methylnitrit-Synthese, vgl. Fig. 12) laufen die Vorgänge in gleicher Weise ab, wie oben im Zusammenhang mit Fig. 7 beschrieben.

Die Bauform der Apparatur 3 ist die in Verbindung mit Fig. 3 und Fig. 5 beschriebene.

Arbeitsweise und Bauform von Apparaturen 4 und 5, entsprechen der Beschreibung im Zusammenhang mit Fig. 3 und Fig. 5.

Bei Apparatur 7 (Methanol-Abtrennung durch Pervaporation oder Dampfpermeation, vgl. Fig. 12) handelt es sich um eine Pervaporations- oder eine Dampfpermeationsanlage, die der Aufspaltung des als Kopfprodukt der Destillationskolonne 4 anfallenden Stromes (k) in ein aus Methanol und Dimethylcarbonat bestehendes Retentat, das auf der sogenannten Retentatseite der Apparatur entnommen wird (1) und über die Apparatur 10 ganz oder teilweise als Storm (11) wieder in Destillationskolonne 4 zurückgeführt wird, sowie ein im wesentlichen aus Methanol bestehendes Permeat (n), das auf der sogenannten Permeatseits entnommen und als Teilstrom (n1) in den oberen Teil des Methylnitrit-Reaktors (Apparatur 3) sowie als Teilstrom (n2) in die apparative Anordnung 8 (Abgasaufarbeitung/Wertstoffrückführung/Nebenproduktausschleusung) weitergeleitet wird, dient. Zusätzlich gespeist wird die genannte Apparatur 7 auf der Retentatseite durch den der Abwasser-Destillation entstammenden Methanol enthaltenden Teilstrom (m3), und zwar bevorzugt in der Form, daß die Ströme (k) und (m3) zuvor in einem Speichergefäß Apparatur 9 vereinigt werden, aus dem der Strom (k1) zur Beschickung der Apparatur 7 abgenommen wird.

Bei Apparatur 7 (Fall: Methanol-Abtrennung durch Pervaporation) handelt es sich beispielsweise um eine der weiter oben beschriebenen Art.

Bei der bereits oben genannten Apparatur 9 handelt es sich um einen druckfesten gerührten Speicherbehälter, der zum Sammeln der Ströme (m3) und (k) bestimmt ist. Bei der oben genannten Apparatur 10 handelt es sich ebenfalls um einen druckfesten, gerührten Speicherbehälter Je nach Betriebsweise, in Abhängigkeit von Störungen durch schwankende Qualitäten der Einsatzstoffe oder aus anderen Gründen kann es sinnvoll sein, aus der genannten Apparatur 10 durch Entspannung einen zusätzlichen gasförmigen Strom (z) zu entnehmen, der besonders reich an leichtsiedenden Nebenprodukten ist und der gegebenenfalls einer weiteren Aufarbeitung, zum Beispiel einer Nebenproduktabtrennung, zugeführt werden kann (nicht Gegenstand der in Fig. 12 beschriebenen Verfahrensverschaltung), wobei die bei dieser Aufarbeitung zurückgewonnen Wertstoffe an geeigneter anderer Stelle gegebenenfalls wieder in den Prozeß eingespeist werden (vgl. auch Beschreibung von Apparatur 6, dort Strom (w)).

Die obigen Beschreibungen zu Fig. 8 und Fig. 9 und den darin dargestellten Apparaturen gelten auch im Zusammenhang mit Fig. 12.

Im folgenden werden die in Fig. 12, 8 und 9 beschriebenen Stoffstöme sowie die zugehörigen Druck- und Temperaturbedingungen, wie sie bei Durchführung des erfindungsgemäßen Verfahrens realisiert werden, angegeben.

Der im kontinuierlichen Betrieb in die Apparatur 1 eingeleitete Gasstrom (d2) besitzt im allgemeinen eine Temperatur von 25 bis 120°C, vorzugsweise von 50 bis 110°C und einen Druck von 1 bis 5 bar, vorzugsweise 1,5 bis 4 bar. Er enthält im allgemeinen 4 bis 25 mol-% Kohlenmonoxid, 30 bis 80 mol-% Kohlendioxid, 5 bis 40 mol-% Methylnitrit, 0 bis 5 mol-% Stickstoff, 1 bis 10 mol-% Methanol, 0 bis 5 mol-% Dimethylcarbonat, 0,1 bis 5,0 mol-% Stickstoffmonoxid, 0 bis 1 mol-% Wasser und weniger als 15 mol-% diverse, zumeist leicht flüchtige Nebenkomponenten, vorzugsweise 5 bis 20 mol-% Kohlenmonoxid, 35 bis 70 mol-% Kohlendioxid, 10 bis 35 mol-% Methylnitrit, 0 bis 4 mol-% Stickstoff, 2 bis 9 mol-% Methanol, 0 bis 4 mol-% Dimethylcarbonat, 0,5 bis 5,0 mol-% Stickstoffmonoxid, 0 bis 0,5 mol-% Wasser und 1 bis 12 mol-% diverse, zumeist leicht flüchtige Nebenkomponenten.

Der dem Strom (d2) vor Eintritt in die Apparatur 1 gegebenenfalls beigemischte Strom (y) enthält als Aktivator Halogenwasserstoff und/oder Chlorameisensäuremethylester und/oder Halogen und/oder andere unter den Reaktionsbedingungen aktivierend wirkendes Halogen enthaltende Substanzen, bevorzugt Chlorwasserstoff und/oder Chlor, in reiner oder verdünnter Form und wird so dosiert, daß der in die Apparatur 1 eintretende Gasstrom 0 bis 3000 ppm, bevorzugt 10 bis 1000 ppm dieser Komponenten enthält.

Der die Apparatur 1 im kontinuierlichen Betrieb verlassende Gasstrom (e) besitzt im allgemeinen eine Temperatur von 50 bis 170°C, vorzugsweise von 60 bis 160°C und einen Druck von 1 bis 5 bar, vorzugsweise 1,5 bis 4 bar. Er enthält im allgemeinen 0 bis 12 mol-% Kohlenmonoxid, 30 bis 80 mol-% Kohlendioxid, 0 bis 20 mol-% Methylnitrit, 0 bis 5 mol-% Stickstoff, 2 bis 12 mol-% Methanol, 3 bis 25 mol-% Dimethylcarbonat, 5 bis 40 mol-% Stickstoffmonoxid, 0 bis 0,5 mol-% Wasser und weniger als 16 mol-% diverse, zumeist leicht flüchtige Nebenkomponenten, vorzugsweise 1 bis 10 mol-% Kohlenmonoxid, 35 bis 70 mol-% Kohlendioxid, 1 bis 19 mol-% Methylnitrit, 0 bis 5 mol-% Stickstoff, 3 bis 10 mol-% Methanol, 5 bis 20 mol-% Dimethylcarbonat, 5 bis 35 mol-% Stickstoffmonoxid, 0 bis 0,5 mol-% Wasser und weniger als 13 mol-% diverse, zumeist leicht flüchtige Nebenkomponenten.

Der die Apparatur 2 im kontinuierlichen Betrieb verlassende Gasstrom (f) besitzt im allgemeinen eine Temperatur von 0 bis 50°C, vorzugsweise von 5 bis 40°C und einen Druck von 1 bis 5 bar, vorzugsweise 1,5 bis 4 bar. Er enthält im allgemeinen 0 bis 15 mol-% Kohlenmonoxid, 30 bis 80 mol-% Kohlendioxid, 0 bis 20 mol-% Methylnitrit, 0 bis 5 mol-% Stickstoff, 0 bis 12 mol-% Methanol, 0 bis 5 mol-% Dimethylcarbonat, 5 bis 45 mol-% Stickstoffmonoxid, 0 bis 1 mol-% Wasser und weniger als 20 mol-% diverse, zumeist leicht flüchtige Nebenkomponenten, vorzugsweise 1 bis 12 mol-% Kohlenmonoxid, 35 bis 70 mol-% Kohlendioxid, 3 bis 15 mol-% Methylnitrit, 0 bis 5 mol-% Stickstoff, 3 bis 10 mol-% Methanol, 0 bis 3 mol-% Dimethylcarbonat, 5 bis 40 mol-% Stickstoffmonoxid, 0 bis 0,5 mol-% Wasser und weniger als 14 mol-% diverse, zumeist leicht flüchtige Nebenkomponenten.

Die Menge des als Gasstrom (f2) ausgeschleusten Anteils des Kreisgases beträgt im kontinuierlichen Betrieb im allgemeinen 0 bis 7 Gew.-%, bevorzugt 0,1 bis 5 Gew.-%, bezogen auf den gasförmigen Kopfstrom (f) der Apparatur 2.

Der im oberen Bereich der Apparatur 2 abgenommene flüssige Strom (f7) enthält 20 bis 90 Gew.-% Methanol, 10 bis 80 Gew.-% Dimethylcarbonat sowie weniger als 30 Gew.-% weitere, zumeist minder flüchtige gelöste Komponenten, bevorzugt 25 bis 60 Gew.-% Methanol, 40 bis 75 Gew.-% Dimethylcarbonat sowie weniger als 20 Gew.-% weitere, zumeist minder flüchtige gelöste Komponenten.

Der nach Ausschleusung des Gasstroms (f2) verbleibende gasförmige Hauptstrom (f1) wird nach Passieren eines Wärmeaustauschers als Strom (f3) sowie nachfolgend eines Verdichters als Strom (f4) der apparativen Anordnung 8 zugeführt. Der genannte Strom (f4) besitzt dann eine Temperatur von 10 bis 70°C, vorzugsweise von 20 bis 60°C, und einen Druck von 1 bis 5 bar, vorzugsweise 1,5 bis 4 bar.

Der aus der apparativen Anordnung 8 zurückgeführte Strom (f5) besitzt im allgemeinen eine Temperatur von 0 bis 70°C, vorzugsweise von 20 bis 60°C und einen Druck von 1 bis 5 bar, vorzugsweise 1,5 bis 4 bar. Er enthält im allgemeinen 0 bis 15 mol-% Kohlenmonoxid, 30 bis 80 mol-% Kohlendioxid, 0 bis 20 mol-% Methylnitrit, 0 bis 5 mol-% Stickstoff, 0 bis 16 mol-% Methanol, 0 bis 5 mol-% Dimethylcarbonat, 5 bis 45 mol-% Stickstoffmonoxid, 0 bis 1 mol-% Wasser und weniger als 20 mol-% diverse, zumeist leicht flüchtige Nebenkomponenten, vorzugsweise 1 bis 12 mol-% Kohlenmonoxid, 35 bis 70 mol-% Kohlendioxid, 3 bis 15 mol-% Methylnitrit, 0 bis 5 mol-% Stickstoff, 3 bis 10 mol-% Methanol, 0 bis 3 mol-% Dimethylcarbonat, 5 bis 40 mol-% Stickstoffmonoxid, 0 bis 0,5 mol-% Wasser und weniger als 14 mol-% diverse, zumeist leicht flüchtige Nebenkomponenten.

Der die Apparatur 2 als Bodenprodukt verlassende Strom (h) hat im allgemeinen eine Temperatur von 10 bis 150°C, vorzugsweise 20 bis 140°C und enthält im allgemeinen 0 bis 40 mol-% Methanol, 20 bis 99 mol-% Dimethylcarbonat sowie gegebenenfalls geringe Mengen Wasser, Schwersieder, wie beispielsweise Oxalsäuredimethylester und weitere Komponenten in einer Größenordnung von weniger als 5 mol-%.

Die Einspeiserate des im allgemeinen bei Umgebungstemperatur als Strom (r) herbeigeführten gasförmigen Stickstoffmonoxids in die Apparatur 3 (vgl. Fig. 12) beträgt im kontinuierlichen Betrieb 0 bis 3 mol-%, bevorzugt 0 bis 1,5 mol-%, bezogen auf den Gasstrom (f5). Anstelle des Stickstoffmonoxids können grundsätzlich auch äquivalente Mengen Stickstoffdioxid, Distickstofftrioxid, Distickstofftetroxid, Methylnitrit oder beliebige Gemische dieser Substanzen zugespeist werden.

Die Einspeiserate des im allgemeinen bei Umgebungstemperatur als Strom (s) herbeigeführten gasförmigen Kohlendioxids in die Apparatur 3 (vgl. Fig. 12) beträgt im kontinuierlichen Betrieb 0 bis 3 mol-%, bevorzugt 0 bis 1,5 mol-%, bezogen auf den Gasstrom (f5).

Der durch Zusammenfügung der Ströme (s1) und (f5) resultierende Gasstrom (f6) besitzt bei kontinuierlichem Betrieb im allgemeinen eine Temperatur von 0 bis 70°C, vorzugsweise 20 bis 60°C, und einen Druck von 1 bis 5 bar, vorzugsweise 1,5 bis 4 bar.

Der aus der apparativen Anordnung 8 herbeigeführte Strom (b2) wird im allgemeinen so bemessen, daß das Stoffmengenverhältnis zwischen dem darin enthaltenen Methanol und dem im Gasstrom (f6) enthaltenen Stickstoffmonoxid 1 bis 10, vorzugsweise 1 bis 5 beträgt. Der genannte Strom (b2) wird gegebenenfalls in die Teilströme (b4) und (b5) aufgetrennt, wobei der Teilstrom (b4) im allgemeinen 50 bis 100 %, vorzugsweise 70 bis 100 % des Stroms (b2) umfaßt.

In einer speziellen Ausführungsform des erfindungsgemäßen Verfahrens wird die Menge des via Zuleitung (b5) im kontinuierlichen Betrieb eingespeisten Methanols vorteilhaft so gewählt, daß das Stoffmengenverhältnis zwischen diesem Methanol und dem im zugespeisten Gasstrom (f6) enthaltenen Stickstoffmonoxid 0,1 bis 2,5, bevorzugt 0,1 bis 1,5 beträgt.

Im kontinuierlichen Betrieb wird die Menge des im allgemeinen bei Umgebungstemperatur via Zuleitung (c2) herbeigeführten Sauerstoffs vorteilhaft so gewählt, daß das Stoffmengenverhältnis zwischen Sauerstoff und dem im zugespeisten Gasstrom (f6) enthaltenen Stickstoffmonoxid 0,15 bis 0,26, bevorzugt 0,20 bis 0,25 beträgt.

Der Innendruck der genannten Apparatur 3 beträgt im allgemeinen 1 bis 5 bar, bevorzugt 1,5 bis 4 bar, ihre Innentemperatur ist in weiten Grenzen variabel. Das Temperaturprofil über die gesamte Länge der Apparatur stellt sich in Abhängigkeit von Menge, Eintrittstemperatur und Aggregatzustand der Einzelzuspeisungen, Gesamtdruck, Umsatz der eingebrachten Reaktanden, Rücklauf des Kopfkondensators sowie Energiezufuhr am Sumpf der Apparatur ein.

Der die Apparatur 3 im kontinuierlichen Betrieb als Bodenprodukt verlassende flüssige Strom (g) hat im allgemeinen eine Temperatur von 10 bis 150°C, bevorzugt 20 bis 140°C, und enthält im allgemeinen 0 bis 70 mol-% Methanol, 10 bis 99 mol-% Wasser, 0 bis 20 mol-% Dimethylcarbonat sowie gegebenenfalls geringe Mengen Salpetersäure und

weitere gelöste Komponenten.

Der im allgemeinen bei Umgebungstemperatur herbeigeleitete, die zur Neutralisation bestimmte Base enthaltende Strom (q) wird im allgemeinen so bemessen, daß er die im genannten Strom (g) enthaltene Säure zu neutralisieren vermag.

Der die Apparatur 3 im kontinuierlichen Betrieb als Kopfprodukt verlassende Strom (d) hat im allgemeinen eine Temperatur von 0 bis 60°C, bevorzugt 10 bis 50°C, einen Druck von 1 bis 5 bar, bevorzugt 1,5 bis 4 bar und enthält im allgemeinen 0 bis 20 mol-% Kohlenmonoxid, 30 bis 80 mol-% Kohlendioxid, 5 bis 40 mol-% Methylnitrit, 0 bis 5 mol-% Stickstoff, 1 bis 12 mol-% Methanol, 0 bis 5 mol-% Dimethylcarbonat, 0,1 bis 5 mol-% Stickstoffmonoxid, 0 bis 1 mol-% Wasser und weniger als 15 mol-% diverse, zumeist leicht flüchtige Nebenkomponenten, vorzugsweise 5 bis 15 mol-% Kohlenmonoxid, 35 bis 70 mol-% Kohlendioxid, 10 bis 35 mol-% Methylnitrit, 0 bis 4 mol-% Stickstoff, 2 bis 9 mol-% Methanol, 0 bis 4 mol-% Dimethylcarbonat, 0,5 bis 5 mol-% Stickstoffmonoxid, 0 bis 0,5 mol-% Wasser und 1 bis 12 mol-% diverse, zumeist leicht flüchtige Nebenkomponenten.

Der genannte Strom (d) wird gegebenenfalls mit Hilfe eines Wärmeaustauschers auf die für seine Einspeisung in die Apparatur 1 gewünschte Temperatur gebracht.

Das dem dermaßen erhaltenen Strom (d1) im allgemeinen bei Umgebungstemperatur als Strom (a) zugespeiste Kohlenmonoxid wird im allgemeinen in chemisch reiner Form eingesetzt, kann jedoch je nach Herstellungsart Fremdgase, wie beispielsweise geringe Mengen Wasserstoff (< 0,1 mol-%) oder Methan (< 0,1 mol-%) enthalten. Seine Zuspeisung erfolgt im kontinuierlichen Betrieb in einer Weise, daß das Stoffmengenverhältnis zwischen zudosiertem Kohlenmonoxid und produziertem Dimethylcarbonat 1 bis 1,2 beträgt und eine zeitlich konstante Konzentration des Kohlenmonoxids im Gasstrom (d2) vorliegt.

Die Apparatur 6 (Abwasser-Destillation) wird im allgemeinen bei einem Druck von 0,5 bis 2 bar, bevorzugt 0,5 bis 1,5 bar, betrieben. Der im oberen Bereich dieser Apparatur abgenommene flüssige Strom (m) fällt im kontinuierlichen Betrieb mit einer Temperatur von 25 bis 80°C, bevorzugt 40 bis 75°C an und enthält im allgemeinen 60 bis 95 mol-% Methanol, 1 bis 35 mol-% Dimethylcarbonat und 0 bis 7 mol-% Wasser, vorzugsweise 70 bis 95 mol-% Methanol, 2 bis 30 mol-% Dimethylcarbonat und 0 bis 5 mol-% Wasser.

Die Menge des als Teilstrom (m3) des genannten Stroms (m) zurückgeführten flüssigen Gemisches kann, je nach Gehalt an Dimethylcarbonat, in weiten Grenzen so gewählt werden, daß sie 0 bis 100 %, bevorzugt 30 bis 100 % des gesamten Stroms (m) umfaßt.

Die Menge der als Teilströme (m1) und (m5) in den oberen und/oder den unteren Bereich der Apparatur 3 geleiteten flüssigen Gemisches kann, je nach Gehalt an Dimethylcarbonat, in weiten Grenzen so gewählt werden, daß sie 0 bis 90 %, vorzugsweise 0 bis 30 % des gesamten Stroms (m) umfaßt. Gleiches gilt für die Menge des als Teilstrom (m6) zurückgeführten flüssigen Gemisches.

Der die Apparatur 6 verlassende gasförmige Strom (v) enthält im allgemeinen Kohlendioxid sowie geringe Mengen Methanol, Dimethylcarbonat, Methylnitrit und diverse Leichtsieder. Er kann gegebenenfalls einer weiteren Aufarbeitung mit dem Ziel der Rückgewinnung darin noch enthaltener Wertstoffe unterworfen werden.

Der im oberen Bereich der genannten Apparatur 6 abgenommene flüssige Strom (w) hat im kontinuierlichen Betrieb eine Temperatur von 10 bis 80°C, bevorzugt 20 bis 70°C und enthält neben Methanol und geringen Mengen Dimethylcarbonat und Wasser vor allem Leichtsieder, wie beispielsweise Formaldehyddimethylacetal und Ameisensäuremethylester. Er wird gegebenenfalls, ähnlich wie der aus dem Speichergefäß Apparatur 10 herbeigeführte Strom (z), einer weiteren Aufarbeitung mit dem Ziel der Rückgewinnung darin noch enthaltener Wertstoffe unterworfen, die dann an geeigneter anderer Stelle wieder in den Prozeß zurückgeführt werden können.

Der in den oberen Bereich der genannten Apparatur 6 eingeleitete und aus der apparativen Anordnung 8 herbeigeführte Strom (u) besitzt im allgemeinen eine Temperatur von -15 bis 30°C, bevorzugt -10 bis 15°C an und enthält 60 bis 99 mol-% Methanol, 0 bis 10 mol-% Dimethylcarbonat, 0 bis 10 mol-% Methylnitrit und 0 bis 5 mol-% Wasser sowie gegebenenfalls geringe Mengen weiterer leichtsiedender Komponenten in der Größenordnung von 0 bis 10 mol-%, vorzugsweise von 70 bis 98 mol-% Methanol, 0 bis 5 mol-% Dimethylcarbonat, 1 bis 7 mol-% Methylnitrit und 0 bis 3 mol-% Wasser sowie gegebenenfalls geringe Mengen weiterer leichtsiedender Komponenten in der Größenordnung von 0 bis 6 mol-%.

Der die Apparatur 6 als Bodenprodukt verlassende Strom (p) enthält im wesentlichen das Prozeßabwasser sowie geringe Mengen darin gelöster und der Neutralisation des Bodenstroms (g) der Apparatur 3 entstammende Salze.

Der die Apparatur 2 als Bodenprodukt verlassende Strom (h) wird mit dem aus Apparatur 6 herbeigeführten Strom (m6) zum Strom (h1) vereinigt und in die Apparatur 4 eingeleitet. Die genannte Apparatur 4 wird, in Abhängigkeit von dem gewählten Innendruck, im allgemeinen in einem Temperaturbereich von 90 bis 240°C betrieben.

Der in die Apparatur 4 eingespeiste und aus Apparatur 10 herbeigeführte flüssige Strom (11) besitzt eine Temperatur von 40 bis 100°C, bevorzugt 50 bis 80°C und enthält 15 bis 45 mol-% Methanol, 40 bis 75 mol- % Dimethylcarbonat sowie geringe Mengen leicht siedender Komponenten in der Größenordnung von im allgemeinen weniger als 10 mol-%.

Der die Apparatur 4 als Bodenprodukt verlassende Strom (i) hat im allgemeinen eine Temperatur von 90 bis 240°C, vorzugsweise von 90 bis 140°C und enthält im allgemeinen weniger als 0,1 mol-% Methanol, bevorzugt weniger als

0,05 mol-% Methanol, mehr als 90 mol-% Dimethylcarbonat, bevorzugt mehr als 95 mol-% Dimethylcarbonat, sowie gegebenenfalls geringe Mengen Schwersieder, wie beispielsweise Oxalsäuredimethylester und weitere Komponenten in einer Größenordnung von insgesamt weniger als 10 mol-%, bevorzugt weniger als 5 mol-%.

Die Destillation in der Apparatur 4 (Druck-Destillation) wird im Falle der Anwendung der Dampfpermeationstechnik vorzugsweise bei einem solchen Druck durchgeführt, daß die als Strom (k) anfallenden Brüden die für ihre weitere Auftrennung optimale Temperatur besitzen. Die Drücke liegen dann im allgemeinen bei 1 bis 4 bar. Im Falle der Anwendung der Pervaporationstechnik wird der Brüdenstrom (k) kondensiert und der Membran bei optimaler Temperatur zugeführt. Auch in diesem Fall wird der Kolonnendruck entsprechend einreguliert.

Der die Apparatur 4 verlassende Strom (k) wird in den Speicherbehälter Apparatur 9 geleitet. Er enthält im allgemeinen 55 bis 97 mol-% Methanol, 2 bis 35 mol-% Dimethylcarbonat, 0 bis 15 mol-% Wasser sowie gegebenenfalls geringe Mengen Leichtsieder.

In der genannten Apparatur 9 werden bevorzugt die Ströme (m3) und (k) gesammelt. Der Inhalt dieses Apparates wird gegebenenfalls gerührt und, gegebenenfalls vermittels äußerer Regelung, in einem Temperaturbereich von 20 bis 150°C, bevorzugt 50 bis 90°C, und einem Druckbereich von 1 bis 5 bar, bevorzugt 1,5 bis 4 bar, gehalten.

Der die Apparatur 10 verlassende gasförmige Strom (z) enthält im allgemeinen Methanol sowie geringere Mengen Kohlendioxid, Dimethylcarbonat und diverse Leichtsieder, wie beispielsweise Ameisensäuremethylester und Formaldehyddimethylacetal. Er wird gegebenenfalls, ähnlich wie der aus der Apparatur 6 entnommene Strom (w), einer weiteren Aufarbeitung mit dem Ziel der Rückgewinnung darin noch enthaltener Wertstoffe unterworfen, die dann an geeigneter anderer Stelle wieder in den Prozeß zurückgeführt werden können.

Die genannte Apparatur 7 (Pervaporation bzw. Dampfpermeation) wird in einem Druckbereich von 0,5 bis 10 bar, vorzugsweise 1 bis 5 bar auf der Retentatseite und 0,5 bis 500 mbar, vorzugsweise 1 bis 100 mbar auf der Permeatseite, betrieben. Die Betriebstemperatur liegt auf der Retentatseite bei 20 bis 150°C, bevorzugt 50 bis 90°C und bei -30 bis +30°C, bevorzugt -15 bis +10°C auf der Permeatseite.

Der die Apparatur 7 als Permeat verlassende Strom (n) liegt hinsichtlich seiner Temperatur im Bereich -30 bis +30°C, vorzugsweise -15 bis +10°C und enthält im allgemeinen mehr als 70 mol-% Methanol, bevorzugt mehr als 90 mol-% Methanol, sowie geringe Mengen Dimethylcarbonat und Wasser. Der genannte Strom (n) wird gegebenenfalls in zwei Teilströme (n1) und (n2) aufgeteilt, wobei (n1) etwa 0 bis 30 %, bevorzugt 0 bis 20 % des Gesamtstroms (n) beträgt.

Der die Apparatur 4 verlassende Strom (i) wird mit Hilfe eines Wärmeaustauschers auf die zur Beschickung der Apparatur 5 (Dimethylcarbonat-Destillation) gewünschte Temperatur gebracht. Der so erhaltene Strom (i1) hat eine Temperatur von 20 bis 180°C, bevorzugt von 40 bis 170°C. Die genannte Apparatur 5 wird im allgemeinen im Druckbereich von 0,75 bis 1,25 bar, bevorzugt bei ca. 1 bar, betrieben.

Das in Apparatur 5 destillierte Dimethylcarbonat (o) hat, je nach Rücklauf/Entnahme-Verhältnis, eine Reinheit von 99,0 bis 99,9 %.

Das in Apparatur 5 anfallende Bodenprodukt enthält im wesentlichen Schwersieder, die verworfen oder einer weiteren Aufarbeitung zugeführt werden.

Das dem Gesamtprozeß im allgemeinen bei Umgebungstemperatur zugeführte Frischmethanol (b) wird in die flüssigen Teilströme (x1) und (x2) zerlegt, wobei der Anteil des Teilstroms (x1) 50 bis 100 % des Gesamtstroms (b) umfaßt. Der Teilstom (x2) wird in den oberen Bereich der Apparatur 8c (Leichtsieder-Wäscher), die im Temperaturbereich -15 bis 30°C, vorzugsweise -10 bis 15°C, und im Druckbereich 1 bis 5 bar, vorzugsweise 1,5 bis 4 bar, betrieben wird, eingebracht.

Der die Apparatur 8c verlassende Strom (x5) besitzt im allgemeinen eine Temperatur von -15 bis 30°C, vorzugsweise -10 bis 15°C und enthält 0 bis 15 mol-% Kohlenmonoxid, 30 bis 80 mol-% Kohlendioxid, 0 bis 20 mol-% Methylnitrit, 0 bis 5 mol-% Stickstoff, 0 bis 12 mol-% Methanol, 0 bis 3 mol-% Dimethylcarbonat, 5 bis 45 mol-% Stickstoffmonoxid, 0 bis 0,5 mol-% Wasser und weniger als 15 mol-% diverse, zumeist leicht flüchtige Nebenkomponenten, vorzugsweise 1 bis 12 mol-% Kohlenmonoxid, 35 bis 70 mol-% Kohlendioxid, 0 bis 7 mol-% Methylnitrit, 0 bis 5 mol-% Stickstoff, 0 bis 7 mol-% Methanol, 0 bis 1 mol-% Dimethylcarbonat, 5 bis 40 mol-% Stickstoffmonoxid, 0 bis 0,5 mol-% Wasser und weniger als 10 mol-% diverse, zumeist leicht flüchtige Nebenkomponenten.

Der oben genannte Teilstrom (x1) des dem Gesamtprozeß zugeführten Frischmethanols wird in den oberen Bereich der Apparatur 8b (Methylnitrit-Nachreaktor/Methanol-Abgaswäscher) eingespeist, die im Temperaturbereich -10 bis 60°C, vorzugsweise 0 bis 40°C, und im Druckbereich 1 bis 5 bar, vorzugsweise 1,5 bis 4 bar, betrieben wird.

Im kontinuierlichen Betrieb wird die Teilmenge (c1) des dem Gesamtprozeß im allgemeinen bei Umgebungstemperatur via Zuleitung (c) herbeigeführten Sauerstoffs vorteilhaft so gewählt, daß das Stoffmengenverhältnis zwischen Sauerstoff und dem im zugespeisten Gasstrom (x5) enthaltenen Stickstoffmonoxid 0,20 bis 0,30, vorzugsweise 0,23 bis 0,28 beträgt.

Der die Apparatur 8b als Bodenprodukt verlassende Strom (b1) liegt hinsichtlich seiner Temperatur im Bereich 0 bis 60°C, vorzugsweise 0 bis 40°C, und enthält im allgemeinen mehr als 70 mol-% Methanol, bevorzugt mehr als 80 mol-% Methanol sowie geringe Mengen Dimethylcarbonat, Wasser, Methylnitrit und diverse Leichtsieder. Der genannte Strom (b1) wird gegebenenfalls in zwei Teilströme (b2) und (b3) aufgeteilt, wobei (b2) etwa 70 bis 100 %, bevorzugt 80

bis 100 % des Gesamtstroms (b1) umfaßt.

Bei dem die Apparatur 8b als gasförmiges Kopfprodukt verlassenden Strom (t) handelt es sich um Abgas, das gegebenenfalls einer zusätzlichen Nachbehandlung zugeführt wird. Es enthält im wesentlichen Kohlendioxid und geringe Mengen Kohlenmonoxid, Methanol sowie gegebenenfalls Inertgase und gasförmige Leichtsieder.

Der der Apparatur 8b seitlich entnommene flüssige Strom (x9) enthält im allgemeinen mehr als 70 mol-% Methanol, bevorzugt mehr als 80 % Methanol, sowie geringe Mengen Dimethylcarbonat, Wasser, Methylnitrit und diverse Leichtsieder. Er wird der Apparatur 8a zugeführt.

Die Apparatur 8a (Methylnitrit-Desorber) wird in einem Temperaturbereich von 0 bis 70°C, bevorzugt von 20 bis 60°C, und in einem Druckbereich von 1 bis 5 bar, bevorzugt von 1,5 bis 4 bar, betrieben.

Der die Apparatur 8a als Bodenprodukt verlassende Strom (x6) liegt hinsichtlich seiner Temperatur im Bereich 0 bis 70°C, vorzugsweise 20 bis 60°C, und enthält im allgemeinen mehr als 70 mol-% Methanol, bevorzugt mehr als 80-mol-% Methanol, sowie geringe Mengen Dimethylcarbonat, Wasser, Methylnitrit und diverse Leichtsieder. Der Strom (x6) wird gegebenenfalls in zwei Teilströme (x7) und (x8) aufgeteilt, wobei (x7) etwa 70 bis 100 %, bevorzugt 80 bis 100 % des Gesamtstroms (x6) umfaßt.

Der Teilstrom (x8) wird mit dem aus Apparatur 7 herbeigeführten Strom (n2) zum Strom (x3) vereinigt und mit Hilfe eines Wärmeaustauschers auf die zur Einspeisung in den oberen Bereich der genannten Apparatur 8b gewünschte Temperatur gebracht. Der sich so ergebende Strom (x4) liegt hinsichtlich seiner Temperatur im Arbeitsbereich der Apparatur 8b.

Der Teilstrom (x7) wird mit dem aus Apparatur 2 herbeigeführten Strom (f7) zum Strom (x10) vereinigt und gleichfalls in die Apparatur 8b eingespeist.

**Beispiel 1**

**Katalysatorherstellung**

0,835 g $PdCl_2$ wurden unter Zusatz von 2 g Natriumchlorid in 4 ml Wasser gelöst. Bei Raumtemperatur wurden unter Rühren 25 ml einer 25 %igen Ammoniaklösung hinzugefügt. 100 ml Aktivkohle Norit ROX 0,8 wurden mit dieser Lösung getränkt und anschließend im Stickstoffstrom getrocknet.

**Herstellung von Dimethylcarbonat**

In einen aus dem Werkstoff 1.4571 gefertigten, aus 4 Reaktionsrohren bestehenden Rohrbündelreaktor von ca. 900 mm Länge (Abmessung der Rohre: 25,0 x 800 mm, Wandstärke 2,0 mm) wurden 960 ml des obigen Katalysators eingebracht, wobei die Katalysator-Füllhöhe ca. 700 mm betrug.

Man ließ bei 3130 mbar durchschnittlich 4927 g/h eines mittels eines vorgeschalteten Rohrbündelwärmeaustauschers auf ca. 80°C temperierten Gemisches strömen, das 13,2 Mol-% Kohlenmonoxid, 2,2 Mol-% Kohlendioxid, 15,1 Mol-% Methylnitrit, 60,2 Mol-% Stickstoff, 5,1 Mol-% Methanol, 0,7 Mol-% Dimethylcarbonat, 3,3 Mol-% Stickstoffmonoxid und 0,2 Mol-% Wasser enthielt (vgl. Strom (d2) in Fig. 4). Im kontinuierlichen Betrieb setzte sich dieses Gasgemisch aus dem am Kopf des Methylnitrit-Reaktors entströmenden Gas (vgl. Strom (d) in Fig. 4) und ca. 128 g/h frisch zudosiertem Kohlenmonoxid (vgl. Strom (a) in Fig. 4) zusammen. Die an dem Reaktor anliegende Kühlung (Wasser) wurde automatisch so einreguliert, daß der den Reaktor verlassende Gasstrom (vgl. Strom (e) in Fig. 4) eine Temperatur von ca. 120°C besaß. Dieser Gasstrom wurde seitlich in einen mit Glas-Raschigringen (4 x 4 mm) befüllten Wäscher/Kondensator (Abmessung: 600 x 48,3 mm, Wandstärke 2,6 mm) eingeleitet, in dem eine Teilkondensation erfolgte. Am Kopf dieser Apparatur war ein mantelseitig kondensierender Rohrbündelwärmeaustauscher angebracht, der aus 7 Rohren der Abmessung 2000 x 10 mm bestand (Wandstärke 1 mm) und durch Kühlung mit Kaltwasser (Temperatur <10°C) so einreguliert wurde, daß das hinter diesem Kondensator austretende Gas (vgl. Strom (f) in Fig. 4) eine Temperatur von ca. 15°C besaß. Bei einem sich dabei einstellenden Druck von 3020 mbar enthielt dieses Gas im Mittel 10,7 Mol-% Kohlenmonoxid, 2,4 Mol-% Kohlendioxid, 9,6 Mol-% Methylnitrit, 63,9 Mol-% Stickstoff, 2,6 Mol-% Methanol, 0,8 Mol-% Dimethylcarbonat und 10 Mol-% Stickstoffmonoxid. Der Bodenstrom (vgl. Strom (h) in Fig. 4), im Mittel 527 g/h, lief bei einer Temperatur von ca. 47°C ab, enthielt 46,3 Mol-% Methanol, 49,1 Mol-% Dimethylcarbonat, 2,2 Mol-% Oxalsäuredimethylester sowie 2,2 Mol-% Wasser und wurde in das Puffergefäß A (vgl. Fig. 4) geleitet.

Ca. 0,2 Gew.-% des am Kopf des Wäscher/Kondensators anfallenden Gasstroms wurden kontinuierlich ausgeschleust, um der Anreicherung gasförmiger Nebenprodukte innerhalb des Kreisprozesses vorzubeugen (vgl. Strom (f2) in Fig. 4). Das verbleibende Gas wurde über einen Wärmeaustauscher sowie einen Kolbenverdichter auf einen Druck von ca. 3250 mbar und eine Temperatur von ca. 28°C gebracht, mit durchschnittlich zusätzlichen 2,4 g/h Stickstoffmonoxid und 4,9 g/h Stickstoff vereinigt und mit ca. 56 g/h Frischmethanol sowie ca. 71 g/h Sauerstoff in den unteren Teil des Methylnitrit-Reaktors (vgl. Apparatur 3 in Fig. 4) so eingespeist, daß eine möglichst schlagartige und vollständige Vermischung aller Reaktanden gegeben war, ohne daß dabei sicherheitstechnisch kritische Konzentrationsbereiche zündfähiger Gemische durchschritten wurden.

Dies geschah so, daß innerhalb eines ersten Statikmischerelementes die Vermischung des Kreisgases mit den frisch zudosierten Gasen Stickstoffmonoxid (vgl. Strom (r) in Fig. 4) und Stickstoff (vgl. Strom (s) in Fig. 4) erfolgte, gleich dahinter über eine Einstoffdüse das Methanol eingesprüht wurde und unmittelbar nach der Methanol-Zugabe und noch vor dem nachfolgenden Statikmischerelement, in dem alle dann vorhandenen Reaktionskomponenten intensiv vermischt werden, Sauerstoff zugespeist wurde.

In den oberen Teil des Reaktors wurden dabei im Mittel 226 g/h Frischmethanol (vgl. Strom (b3) in Fig. 4), 80 g/h des zurückgeführten Methanols (vgl. Strom (m1) in Fig. 4), das aus dem Puffergefäß C (vgl. Fig. 4) entnommen wurde, sowie 155 g/h des im wesentlichen Methanol enthaltenden Gemisches (vgl. Strom (1) in Fig. 4), das aus dem Puffergefäß E (vgl. Fig. 4) entnommen wurde, gepumpt. Bei dem Methylnitrit-Reaktor (Abmessungen: 2200 x 48,3 mm, Wandstärke 2,6 mm) handelte es sich um ein Füllkörper (Glasringe der Abmessung 4 x 4 mm) enthaltendes Reaktionsgefäß, das ein inneres freies Volumen von ca. 2,25 l besaß.

Am Kopf dieser Apparatur war ein mantelseitig kondensierender Rohrbündelwärmeaustauscher angebracht, der aus 7 Rohren der Abmessung 10 x 1000 mm bestand (Wandstärke 1 mm) und dessen Kühlleistung durch Kühlung mit Kaltwasser (Temperatur <15°C) so einreguliert wurde, daß das hinter diesem Kondensator austretende Gas (vgl. Strom (d) in Fig. 4) eine Temperatur von ca. 28°C besaß. Es stand im Mittel unter einem Druck von ca. 3140 mbar und wurde kontinuierlich in den Dimethylcarbonat-Reaktor zurückgeführt (siehe oben).

Der flüssige Bodenstrom (vgl. Strom (g) in Fig. 4), im Mittel 219 g/h, lief bei einer Temperatur von ca. 28°C ab, enthielt 38,9 Mol-% Methanol, 5,1 Mol-% Dimethylcarbonat sowie 55,6 Mol-% Wasser und wurde nach Neutralisation der geringen Mengen darin enthaltener Salpetersäure in das Puffergefäß B (vgl. Fig. 4) geleitet.

Das in Puffergefäß A (vgl. Fig. 4) befindliche ca. 45°C warme Gemisch wurde seitlich in eine kontinuierlich arbeitende Druckkolonne (Apparatur 4 in Fig. 4) eingespeist, die in einem Druckbereich von 9085 (Boden) bis 9050 mbar (Kopf) betrieben wurde. Bei dieser Druckkolonne handelte es sich um eine mit Füllkörpern (Glasringe der Abmessung 4 x 4 mm) befüllte Destillationskolonne (Abmessungen 33,7 x 2500 mm, Wandstärke 2,6 mm), in der ca. 15 theoretische Trennstufen realisiert waren und die bei einem Rücklaufverhältnis von 1,1:1 arbeitete. Diese Druckkolonne wurde außerdem noch aus dem Puffergefäß F (vgl. Fig. 4) mit dem Rückstrom (n1) (vgl. Fig. 4) beschickt (siehe unten). Den unteren Abschluß dieser Druckkolonne bildete ein Kolonnenschuß mit Mantelbeheizung. Am oberen Ende der Druckkolonne befand sich ein liegender Doppelrohrkondensator, der mit Kühlwasser betrieben wurde.

Das am Boden ablaufende flüssige Gemisch (vgl. Strom (i) in Fig. 4), im Mittel 398 g/h, hatte eine Temperatur von im Mittel ca. 180°C und wurde in das Puffergefäß D (vgl. Fig. 4) übergeführt. Das am Kopf gewonnene Kondensat (vgl. Strom (k) in Fig. 4), im Mittel 744 g/h, besaß eine Temperatur von ca. 125°C und enthielt im Mittel ca. 86 Mol-% Methanol, ca. 11 Mol-% Dimethylcarbonat, etwa 1 bis 1,5 Mol-% Wasser sowie geringe Mengen Leichtsieder. Es wurde seitlich in eine bei Normaldruck betriebene, kontinuierlich arbeitende Destillationskolonne (vgl. Apparatur 7 in Fig. 4) eingespeist, in der eine Trennung in einen Bodenablauf, der überwiegend Methanol sowie Wasser enthielt und der in das Puffergefäß E übergeführt wurde, und in ein Kopfprodukt (vgl. Strom (n) in Fig. 4), im Mittel 589 g/h, das ca. 83 Mol-% Methanol, ca. 14 Mol-% Dimethylcarbonat sowie geringe Mengen Leichtsieder enthielt, und das als Kondensat mit einer Temperatur von ca. 60°C in das Puffergefäß F (vgl. Fig. 4) geleitet wurde, erfolgte. In dieses Puffergefäß E, dessen Inhalt magnetisch gerührt wurde, wurden außerdem im Mittel 28 g/h des Gemisches aus dem Puffergefäß C (vgl. Fig. 4), das der Abwasser-Destillation (vgl. Apparat 6 in Fig. 4) entstammte, geleitet. Im Mittel 615 g/h des Gemisches aus dem Puffergefäß F (vgl. Fig. 4) wurden seitlich in die Druckdestillation (vgl. Apparatur 4 in Fig. 4) eingepumpt, stellten also somit den zweiten Zustrom dar, aus dem die in dieser Apparatur ablaufende destillative Auftrennung gespeist wurde. Bei dieser Kolonne handelte es sich um eine mit Füllkörpern (Glasringe der Abmessung 4 x 4 mm) befüllte Destillationskolonne (Abmessungen 42,4 x 3200 mm, Wandstärke 2,0 mm), in der ca. 25 theoretische Trennstufen realisiert waren und die bei einem Rücklaufverhältnis von 1,7:1 arbeitete. Den unteren Abschluß der genannten Kolonne bildete ein Kolonnenschuß mit Mantelbeheizung. Am oberen Ende der besagten Kolonne befand sich ein liegender Doppelrohrkondensator, der mit Kühlwasser betrieben wurde.

Das im Puffergefäß D (vgl. Fig. 4) befindliche Stoffgemisch wurde, nach Abkühlen auf ca. 95°C mittels eines zwischengeschalteten einfachen Rohrbündelwärmeaustauschers, dessen Kühlleistung automatisch so geregelt wurde, daß die austretende Flüssigkeit die gewünschte Temperatur hatte, seitlich in eine bei Normaldruck betriebene kontinuierlich arbeitende Kolonne eingespeist (vgl. Strom (i1) in Fig. 4). Bei dieser Kolonne handelte es sich um eine mit Füllkörpern (Glasringe der Abmessung 4 x 4 mm) befüllte Destillationskolonne (Abmessungen 33,7 x 2200 mm, Wandstärke 2,6 mm), in der ca. 15 theoretische Trennstufen realisiert waren und die bei einem Rücklaufverhältnis von 1:1 arbeitete. Den unteren Abschluß der genannten Kolonne bildete ein Kolonnenschuß mit Mantelbeheizung. Am oberen Ende dieser Kolonne befand sich ein liegender Doppelrohrkondensator, der mit Kühlwasser betrieben wurde.

Als Kopfprodukt der Destillation (vgl. Strom (o) in Fig. 4) wurden im Mittel 375 g/h Dimethylcarbonat mit einer Reinheit von >99,7 % (GC) gewonnen. Im Bodenablauf, im Mittel 23 g/h, befanden sich Schwersieder, im wesentlichen, zu mehr als 90 %, Oxalsäuredimethylester.

Der im Puffergefäß B (vgl. Fig. 4) befindliche neutralisierte Bodenablauf des Methylnitrit-Reaktors (vgl. Apparatur 3 in Fig. 4) wurde seitlich in eine bei Normaldruck betriebene Kolonne eingespeist (vgl. Strom (g1) in Fig. 4). Bei dieser Kolonne handelte es sich um eine mit Füllkörpern (Glasringe der Abmessung 4 x 4 mm) befüllte Destillationskolonne

(Abmessungen 33,7 x 3200 mm, Wandstärke 2,6 mm), in der ca. 22 theoretische Trennstufen realisiert waren und die bei einem Rücklaufverhältnis von 1:1 arbeitete. Den unteren Abschluß der genannten Kolonne bildete ein Kolonnenschuß mit Mantelbeheizung. Am oberen Ende dieser Kolonne befand sich ein liegender Doppelrohrkondensator, der mit Kühlwasser betrieben wurde.

Als Kopfprodukt der Destillation (vgl. Strom (m) in Fig. 4) wurden im Mittel 139 g/h eines Gemisches erhalten, das durchschnittlich 86 Mol-% Methanol, 11 % Dimethylcarbonat und Wasser enthält und das in das Puffergefäß C (vgl. Fig. 4) geleitet wurde. Im Bodenablauf, im Mittel 82 g/h, (vgl. Strom (p) in Fig. 4) befanden sich Wasser sowie insbesondere wasserlösliche Salze.

**Beispiel 2**

Als Katalysator wurde der in Beispiel 1 beschriebene eingesetzt.

**Herstellung von Dimethylcarbonat**

In einen aus dem Werkstoff 1.4571 gefertigten aus 4 Reaktionsrohren bestehenden Rohrbündelreaktor von ca. 900 mm Länge (Abmessung der Rohre: 25,0 x 800 mm, Wandstärke 2,0 mm) wurden 960 ml des Katalysators eingebracht, wobei die Katalysator-Füllhöhe ca. 700 mm betrug.

Man ließ bei 3130 mbar durchschnittlich 4923 g/h eines mittels eines vorgeschalteten Rohrbündelwärmeaustauschers auf ca. 90°C temperierten Gemisches strömen, das 13,2 Mol-% Kohlenmonoxid, 2,2 Mol-% Kohlendioxid, 15,1 Mol-% Methylnitrit, 60,0 Mol-% Stickstoff, 5,1 Mol-% Methanol, 0,7 Mol-% Dimethylcarbonat, 3,3 Mol-% Stickstoffmonoxid und 0,2 Mol-% Wasser enthielt (vgl. Strom (d2) in Fig. 6). Im kontinuierlichen Betrieb setzte sich dieses Gasgemisch aus dem am Kopf des Methylnitrit-Reaktors entströmenden Gas (vgl. Strom (d) in Fig. 6) und ca. 128 g/h frisch zudosiertem Kohlenmonoxid (vgl. Strom (a) in Fig. 6) zusammen. Die an dem Reaktor anliegende Kühlung (Wasser) wurde automatisch so einreguliert, daß der den Reaktor verlassende Gasstrom (vgl. Strom (e) in Fig. 6) eine Temperatur von ca. 120°C besaß. Dieser Gasstrom wurde seitlich in einen mit Glas-Raschigringen (4 x 4 mm) befüllten Wäscher/Kondensator (Abmessung: 600 x 48,3 mm, Wandstärke 2,6 mm) eingeleitet, in dem eine Teilkondensation erfolgte. Am Kopf dieser Apparatur war ein mantelseitig kondensierender Rohrbündelwärmeaustauscher angebracht, der aus 7 Rohren der Abmessung 2000 x 10 mm bestand (Wandstärke 1 mm) und durch Kühlung mit Kaltwasser (Temperatur <10°C) so einreguliert wurde, daß das hinter diesem Kondensator austretende Gas (vgl. Strom (f) in Fig. 6) eine Temperatur von ca. 15°C besaß. Bei einem sich dabei einstellenden Druck von 3020 mbar enthielt dieses Gas im Mittel 10,7 Mol-% Kohlenmonoxid, 2,4 Mol-% Kohlendioxid, 9,6 Mol-% Methylnitrit, 63,8 Mol-% Stickstoff, 2,6 Mol-% Methanol, 0,8 Mol-% Dimethylcarbonat und 10,0 Mol-% Stickstoffmonoxid. Der Bodenstrom (vgl. Strom (h) in Fig. 6), im Mittel 526 g/h, lief bei einer Temperatur von ca. 47°C ab, enthielt 46,3 Mol-% Methanol, 49,2 Mol-% Dimethylcarbonat, 2,2 Mol-% Oxalsäuredimethylester sowie 2,3 Mol-% Wasser und wurde in das Puffergefäß A (vgl. Fig. 6) geleitet.

Ca. 0,2 Gew.-% des am Kopf des Wäscher/Kondensators anfallenden Gasstroms wurden kontinuierlich ausgeschleust, um der Anreicherung gasförmiger Nebenprodukte innerhalb des Kreisprozesses vorzubeugen (vgl. Strom (f2) in Fig. 6). Das verbleibende Gas wurde über einen Wärmeaustauscher sowie einen Kolbenverdichter auf einen Druck von ca. 3250 mbar und eine Temperatur von ca. 28°C gebracht, mit durchschnittlich zusätzlichen 2,4 g/h Stickstoffmonoxid (vgl. Strom (3) in Fig. 6) und 4,9 g/h Stickstoff (vgl. Strom (s) in Fig. 6) vereinigt und mit ca. 56 g/h Frischmethanol (vgl. Strom (b4) in Fig. 6) sowie ca. 71 g/h Sauerstoff (vgl. Strom (c) in Fig. 6) in den unteren Teil des Methylnitrit-Reaktors (vgl. Apparatur 3 in Fig. 6) so eingespeist, daß eine möglichst schlagartige und vollständige Vermischung aller Reaktanden gegeben war, ohne daß dabei sicherheitstechnisch kritische Konzentrationsbereiche zündfähiger Gemische durchschritten wurden.

Dies geschah so, daß innerhalb eines ersten Statikmischerelementes die Vermischung des Kreisgases mit den frisch zudosierten Gasen Stickstoffmonoxid (vgl. Strom (r) in Fig. 6) und Stickstoff (vgl. Strom (s) in Fig. 6) erfolgte, gleich dahinter über eine Einstoffdüse das Methanol eingesprüht wurde und unmittelbar nach der Methanol-Zugabe und noch vor dem nachfolgenden Statikmischerelement, in dem alle dann vorhandenen Reaktionskomponenten intensiv vermischt wurden, Sauerstoff zugespeist wurde.

In den oberen Teil des Reaktors wurden dabei im Mittel 225 g/h Frischmethanol (vgl. Strom (b3) in Fig. 6) 111 g/h des hauptsächlich Methanol enthaltenden Gemisches (vgl. Strom (m1) in Fig. 6), das aus dem Puffergefäß C (vgl. Fig. 6) entnommen wurde und das im Schnitt ca. 11 g/h Dimethylcarbonat sowie ca. 3 g/h Wasser enthielt, sowie 155 g/h des im wesentlichen Methanol enthaltenden Gemisches (vgl. Strom (n1) in Fig. 6), das aus dem Puffergefäß E (vgl. Fig. 6) entnommen wurde, gepumpt. Bei dem Methylnitrit-Reaktor (Abmessungen: 2200 x 48,3 mm, Wandstärke 2,6 mm) handelte es sich um ein Füllkörper (Glasringe der Abmessung 4 x 4 mm) enthaltendes Reaktionsgefäß, das ein inneres freies Volumen von ca. 2,25 l besaß.

Am Kopf dieser Apparatur war ein mantelseitig kondensierender Rohrbündelwärmeaustauscher angebracht, der aus 7 Rohren der Abmessung 10 x 1000 mm bestand (Wandstärke 1 mm) und dessen Kühlleistung durch Kühlung mit Kaltwasser (Temperatur <15°C) so einreguliert wurde, daß das hinter diesem Kondensator austretende Gas (vgl. Strom

(d) in Fig. 6) eine Temperatur von ca. 28°C besaß. Es stand im Mittel unter einem Druck von ca. 3140 mbar und wurde kontinuierlich in den Dimethylcarbonat-Reaktor zurückgeführt (siehe oben).

Der flüssige Bodenstrom (vgl. Strom (g) in Fig. 6), im Mittel 220 g/h, lief bei einer Temperatur von ca. 28°C ab, enthielt 39 Mol-% Methanol, ca. 5 Mol-% Dimethylcarbonat sowie 56 Mol-% Wasser und wurde nach Neutralisation der geringen Mengen darin enthaltener Salpetersäure in das Puffergefäß B (vgl. Fig. 6) geleitet.

Das in dem Puffergefäß A befindliche ca. 45°C warme Gemisch wurde seitlich in eine kontinuierlich arbeitende Druckkolonne (Apparatur 4 in Fig. 6) eingespeist, die bei einem Druck von ca. 3000 mbar betrieben wurde. Den unteren Abschluß dieser Kolonne bildete ein Kolonnenschuß mit Mantelbeheizung. Am oberen Ende der Kolonne befand sich ein liegender Doppelrohrkondensator, der mit Kühlwasser betrieben wurde.

Bei der Druckkolonne handelte es sich um eine mit Füllkörpern (Glasringe der Abmessung 4 x 4 mm) befüllte Destillationskolonne (Abmessungen 33,7 x 2500 mm, Wandstärke 2,6 mm), in der ca. 15 theoretische Trennstufen realisiert waren und die bei einem Rücklaufverhältnis von 1,5:1 arbeitete. Die Druckkolonne wurde außerdem noch kontinuierlich mit dem Inhalt des Puffergefäßes F beschickt, das aus dem Retentat der Pervaporation (vgl. Strom (1), Fig. 6) sowie einem Teilstrom, durchschnittlich ca. 28 g/h, des aus Puffergefäß C entnommenen Kopfproduktes der Abwasserkolonne (Strom(m2), vgl. Fig. 6) gespeist wurde (siehe unten). Den unteren Abschluß der Druckkolonne bildete ein Kolonnenschuß mit Mantelheizung. Am oberen Ende der Druckkolonne befand sich ein liegender Doppelrohrkondensator, der mit Kühlwasser betrieben wurde.

Das am Boden der Druckkolonne ablaufende flüssige Gemisch (vgl. Strom (i) in Fig. 6) hatte eine Temperatur von im Mittel ca. 130°C und wurde in das Puffergefäß D (vgl. Fig. 6) übergeführt. Das am Kopf gewonnene Kondensat (vgl. Strom (k) in Fig. 6) besaß nach Kondensation eine Temperatur von 80 bis 90°C und enthielt im Mittel ca. 77 Mol-% Methanol, ca. 17 Mol-% Dimethylcarbonat, etwa 3 bis 3,5 Mol-% Wasser sowie geringe Mengen Leichtsieder.

Es wurde in eine Pervaporationsanlage eingespeist, die auf der Retentatseite mit einem Druck von ca. 1,4 bar und einer Temperatur von ca. 80°C und auf der Permeatseite mit einem Druck von 30 bis 50 mbar und einer Temperatur von ca. 0 bis 3°C betrieben wurde. Bei dieser Apparatur handelte es sich um drei in Serie geschaltete Plattenmodule mit Flächen von jeweils 200 $cm^2$, die Membranen des Typs AERK 300 enthalten.

Das Permeat enthielt durchschnittlich ca. 94 Mol-% Methanol, 1,5 bis 2 Mol-% Dimethylcarbonat und 4 bis 5 Mol-% Wasser und wurde in das Puffergefäß E geleitet, während das Retentat, im Durchschnitt 127 g/h, dem Puffergefäß F zugeführt wurde.

Das im Puffergefäß D (vgl. Fig. 6) befindliche Stoffgemisch wurde, nach Abkühlen auf ca. 60°C mittels eines zwischengeschalteten einfachen Rohrbündelwärmeaustauschers, dessen Kühlleistung automatisch so geregelt wurde, daß die austretende Flüssigkeit die gewünschte Temperatur hatte, seitlich in eine bei Normaldruck betriebene, kontinuierlich arbeitende Kolonne eingespeist (vgl. Strom (i1) in Fig. 6).

Bei dieser Kolonne handelte es sich um eine mit Füllkörpern (Glasringe der Abmessung 4 x 4 mm) befüllte Destillationskolonne (Abmessungen 33,7 x 2200 mm, Wandstärke 2,6 mm), in der ca. 15 theoretische Trennstufen realisiert waren und die bei einem Rücklaufverhältnis von 1:1 arbeitete. Den unteren Abschluß der genannten Kolonne bildete ein Kolonnenschuß mit Mantelbeheizung. Am oberen Ende dieser Kolonne befand sich ein liegender Doppelrohrkondensator, der mit Kühlwasser betrieben wurde.

Als Kopfprodukt der Destillation (vgl. Strom (o) in Fig. 6) wurden im Mittel 375 g/h Dimethylcarbonat mit einer Reinheit von >99,7 % (GC) gewonnen. Im Bodenablauf befanden sich Schwersieder, im wesentlichen, zu mehr als 90 %, Oxalsäuredimethylester.

Der im Puffergefäß B (vgl. Fig. 6) befindliche neutralisierte Bodenablauf des Methylnitrit-Reaktors (vgl. Apparatur 3 in Fig. 6) wurde seitlich in eine bei Normaldruck betriebene Kolonne eingespeist (vgl. Strom (g1) in Fig. 6). Bei dieser Kolonne handelte es sich um eine mit Füllkörpern (Glasringe der Abmessung 4 x 4 mm) befüllte Destillationskolonne (Abmessungen 33,7 x 3200 mm, Wandstärke 2,6 mm), in der ca. 22 theoretische Trennstufen realisiert waren und die bei einem Rücklaufverhältnis von 1:1 arbeitete. Den unteren Abschluß der genannten Kolonne bildete ein Kolonnenschuß mit Mantelbeheizung. Am oberen Ende der Kolonne befand sich ein liegender Doppelrohrkondensator, der mit Kühlwasser betrieben wurde.

Als Kopfprodukt der Abwasser-Destillation (vgl. Strom (m) in Fig. 6) wurden im Mittel 139 g/h eines Gemisches erhalten, das durchschnittlich 86 Mol-% Methanol, 11 % Dimethylcarbonat sowie Wasser enthielt und das in das Puffergefäß C (vgl. Fig. 6) geleitet wurde. Im Bodenablauf (vgl. Strom (p) in Fig. 6) befanden sich Wasser sowie insbesondere wasserlösliche Salze.

## Beispiel 3

### Katalysatorherstellung

13,33 g $PdCl_2$ wurden unter Zusatz von 6,37 g Lithiumchlorid in 64 ml Wasser gelöst. 1000 ml $\gamma$-$Al_2O_3$ (SPH 535 der Firma RHONE POULENC) wurden mit dieser Lösung getränkt und anschließend im Stickstoffstrom getrocknet. Der erhaltene Katalysator wurde mit dem gleichen Volumen inerter Glaskörper gleicher Abmessungen (Durchmesser ca. 2

mm) verdünnt.

**Herstellung von Dimethylcarbonat**

In einen aus dem Werkstoff 1.4571 gefertigten Rohrreaktor von ca. 3 m Länge (Abmessung des Reaktionsrohrs: 21 x 2900 mm, Wandstärke 2 mm) wurden 970 ml des wie oben beschriebenen verdünnten Katalysators eingebracht, wobei die Katalysator-Füllhöhe ca. 2800 mm betrug. Der Reaktor war mit einem von einer Kühlflüssigkeit durchströmten Mantel versehen. Die durchschnittliche Temperatur des Kühlmittels betrug 70°C.

Man ließ bei 3130 mbar durchschnittlich 1874,2 g/h eines vermittels eines vorgeschalteten Rohrbündelwärmeaustauschers auf ca. 62°C temperierten Gemisches strömen, das im Mittel 13,2 mol-% Kohlenmonoxid, 43,2 mol-% Kohlendioxid, 25,8 mol-% Methylnitrit, 6,6 mol-% Methanol, 0,9 mol-% Dimethylcarbonat, 0,4 mol-% Stickstoffmonoxid, 0,1 mol-% Wasser, 1000 ppm Chlorwasserstoff und 9,7 mol-% Leichtsieder (Ameisensäuremethylester, Formaldehyddimethylacetal, Methylchlorid, Stickstoff, Methan und Wasserstoff) enthielt (vgl. Strom (d2) in Fig. 10). Im kontinuierlichen Betrieb setzte sich dieses Gasgemisch aus dem am Kopf des Methylnitrit-Reaktors entströmenden Gas (vgl. Strom (d) in Fig. 10), ca. 125,3 g/h frisch zudosiertem Kohlenmonoxid (vgl. Strom (a) in Fig. 10) und ca. 1,2 g/h frisch zudosiertem Chlorwasserstoff (vgl. Strom (y) in Fig. 10) zusammen. Der den Reaktor verlassende Gasstrom (vgl. Strom (e) in Fig. 10) besaß eine Temperatur von ca. 70°C. Dieser Gasstrom wurde seitlich in einen mit Glas-Raschigringen (4 x 4 mm) befüllten Wäscher/Kondensator (Abmessung: 2000 x 33,7 mm, Wandstärke 1,6 mm) eingeleitet, in dem eine Teilkondensation erfolgte. Am Kopf dieser Apparatur war ein mantelseitig kondensierender Rohrbündelwärmeaustauscher angebracht, der aus 7 Rohren der Abmessung 1000 x 10 mm bestand (Wandstärke 1 mm) und durch Kühlung mit Kaltwasser (Temperatur < 15°C) so einreguliert wurde, daß das hinter diesem Kondensator austretende Gas (vgl. Strom (f) in Fig. 10) eine Temperatur von ca. 30°C besaß. Bei einem sich dabei einstellenden Druck von 3010 mbar enthielt dieses Gas im Mittel 3,0 mol-% Kohlenmonoxid, 48,9 mol-% Kohlendioxid, 5,8 mol-% Methylnitrit, 5,4 mol-% Methanol, 1,9 mol-% Dimethylcarbonat, 23,7 mol-% Stickstoffmonoxid, 0,1 mol-% $H_2O$ und 11,2 mol-% der oben genannten Leichtsieder. Der Bodenstrom (vgl. Strom (h) in Fig. 10) im Mittel 366,8 g/h, lief bei einer Temperatur von ca. 63°C ab, enthielt 13,2 mol-% Methanol, 85,7 mol-% Dimethylcarbonat, 0,7 mol-% Oxalsäuredimethylester sowie 0,2 mol-% Wasser und wurde in das Puffergfäß A (vgl. Fig. 10) geleitet.

Dem Rücklauf des Kondensators wurde ein flüssiger Strom von im Mittel 16,7 g/h (vgl. Strom (f7) in Fig. 10) entnommen und dem Puffergefäß G (vgl. Fig. 11), dessen Inhalt magnetisch gerührt wurde, zugeführt. Es besaß eine Temperatur von ca. 30°C und enthielt im Mittel 60,6 mol-% Methanol, 37,4 mol-% Dimethylcarbonat, 1,3 mol-% Wasser und 0,7 mol-% der genannten Leichtsieder.

Ca. 3,5 Gew.-% des am Kopf des Wäscher/Kondensators anfallenden Gasstroms (vgl. Strom (f) in Fig. 10) wurden kontinuierlich ausgeschleust und dem unteren Bereich des Leichtsieder-Wäschers (vgl. Apparatur 8c in Fig. 11) zugeführt, um der Anreicherung gasförmiger Nebenprodukte innerhalb des Kreisprozeßes vorzubeugen (vgl. Strom (f2) in Fig. 10). Das verbleibende Gas wurde über einen Wärmeaustauscher sowie einen Kolbenverdichter auf einen Druck von ca. 3250 mbar und eine Temperatur von ca. 42°C gebracht (vgl. Ströme (f3), (f4) in Fig. 10) und in den unteren Bereich des Methylnitrit-Desorbers (vgl. Apparatur 8a in Fig. 11) eingeleitet. Der am Kopf dieses Methylnitrit-Desorbers gewonnene Gasstrom (vgl. Strom (f5) in Fig. 10) besaß eine Temperatur von ca. 26°C und enthielt im Mittel 3,0 mol-% Kohlenmonoxid, 49,9 mol-% Kohlendioxid, 6,4 mol-% Methylnitrit, 5,1 mol-% Methanol, 0,4 mol-% Dimethylcarbonat, 24,0 mol-% Stickstoffmonoxid und 11,2 mol-% der oben genannten Leichtsieder.

Dieser Strom wurde mit durchschnittlich zusätzlichen 6,2 g/h Stickstoffmonoxid (vgl. Strom (r) in Fig. 10) und 12,3 g/h Kohlendioxid (vgl. Ströme (s), (s1) in Fig. 10) vereinigt (vgl. Strom (f6) in Fig. 10) und mit einem Strom von ca. 96,5 g/h (vgl. Strom (b3) in Fig. 10) aus dem Puffergefäß H (vgl. Fig. 10) sowie ca. 69,3 g/h Sauerstoff (vgl. Strom (c2) in Fig. 10) in den unteren Teil des Methylnitrit-Reaktors (vgl. Apparatur 3 in Fig. 10) so eingespeist, daß eine möglichst schlagartige und vollständige Vermischung aller Reaktanden gegeben war, ohne daß dabei sicherheitstechnisch kritische Konzentrationsbereiche zündfähiger Komponenten durchschritten wurden.

Dies geschah im Fall des hier beschriebenen Beispiels auf die Weise, daß innerhalb eines ersten Statikmischerelementes die Vermischung des Kreisgases mit den frisch zudosierten Gasen Stickstoffmonoxid (vgl. Strom (r) in Fig. 10) und Kohlendioxid (vgl. Strom (r), (s1) in Fig. 10) erfolgte, gleich dahinter über eine Einstoffdüse der im wesentlichen Methanol enthaltende Strom aus dem Puffergefäß H (vgl. Fig. 10) eingesprüht wurde und unmittelbar nach der Methanol-Zugabe und noch vor dem nachfolgenden Statikmischerelement, in dem alle dann vorhandenen Reaktionskomponenten intensiv vermischt wurden, Sauerstoff (vgl. Srom (c2) in Fig. 10) zugespeist wurde.

In den oberen Teil des Reaktors wurde dabei ein Strom von im Mittel 386,2 g/h (vgl. Strom (b2) in Fig. 10) aus dem Puffergefäß H (vgl. Fig. 6) gepumpt.

Bei dem Methylnitrit-Reaktor (Abmessungen: 7000 x 51 mm, Wandstärke 1,6 mm) handelte es sich um ein Füllkörper (Glasringe der Abmessung 4 x 4 mm) enthaltendes Reaktionsgefäß, das ein inneres freies Volumen von ca. 12,61 besaß.

Am Kopf dieser Apparatur war ein mantelseitig kondensierender Rohrbündelwärmeaustauscher angebracht, der aus 7 Rohren der Abmessung 10 x 1000 mm bestand (Wandstärke 1 mm) und dessen Kühlleistung durch Kühlung mit

Kaltwasser (Temperatur < 15°C) so einreguliert wurde, daß das hinter diesem Kondensator austretende Gas (vgl. Strom (d) in Fig. 10) eine Temperatur von ca. 34°C besaß. Es stand im Mittel unter einem Druck von ca. 3140 mbar und wurde kontinuierlich in den Dimethylcarbonat-Reaktor zurückgeführt (siehe oben).

Der flüssige Bodenstrom (vgl. Strom (g) in Fig. 10), im Mittel 224,8 g/h, lief bei einer Temperatur von ca. 51°C ab, enthielt 39,1 mol-% Methanol, 5,4 mol-% Dimethylcarbonat, 54,4 mol-% Wasser sowie 1,1 mol-% Salpetersäure und wurde nach Neutralisation der darin enthaltenen Salpetersäure mit ca. 6,6 g/h 50%iger Natronlauge (vgl. Strom (q) in Fig. 10) in das Puffergefäß B (vgl. Fig. 10) geleitet (vgl. Strom (g1) in Fig. 10).

Ca. 366,8 g/h von dem im Puffergefäß A und 397,0 g/h von dem im Puffergefäß F (vgl. Fig. 10) befindlichen Gemisch wurden seitlich in eine kontinuierlich arbeitende Druckkolonne (Apparatur 4 in Fig. 10) eingespeist, die in einem Druckbereich von 10072 (Boden) bis 10030 mbar (Kopf) betrieben wurde. Bei dieser Druckkolonne handelte es sich um eine mit Füllkörpern (Glasringe der Abmessung 4 x 4 mm) befüllte Destillationskolonne (Abmessungen 51 x 3000 mm, Wandstärke 1,6 mm), in der ca. 16 theoretische Trennstufen realisiert waren und die bei einem Rücklaufverhältnis von 1,44:1 arbeitete. Den unteren Abschluß der Druckkolonne bildete ein Kolonnenschuß mit Mantelbeheizung. Am oberen Ende der Druckkolonne befand sich ein liegender Doppelrohrkondensator, der mit Kühlwasser betrieben wurde.

Das am Boden ablaufende flüssige Gemisch (vgl. Strom (i) in Fig. 10), im Mittel 380,0 g/h, hatte eine Temperatur von ca. 185°C. Es enthielt 99,1 mol-% Dimethylcarbonat sowie 0,8 mol-% Oxalsäuredimethylester und wurde in das Puffergefäß D (vgl. Fig. 10) geleitet.

Das am Kopf gewonnene Kondensat (vgl. Strom (k) in Fig. 10), im Mittel 383,8 g/h, besaß eine Temperatur von ca. 120°C und enthielt im Mittel ca. 88,1 mol-% Methanol, 8,7 mol-% Dimethylcarbonat, 0,1 mol-% Wasser sowie 3,1 mol-% Leichtsieder. Es wurde dem Puffergefäß C (vgl. Fig. 10) zugeführt, dessen Inhalt magnetisch gerührt wurde und in das außerdem der Strom (m) (vgl. Fig. 10), im Mittel 179,5 g/h, der der Abwasser-Destillation (vgl. Apparatur 6 in Fig. 10) entstammte, geleitet wurde.

Aus diesem Puffergefäß wurde ein Strom von ca. 563,3 g/h (vgl. Strom (k1) in Fig. 10) seitlich in eine bei Normaldruck betriebene kontinuierlich arbeitende Destillationskolonne (vgl. Apparatur 7 in Fig. 10) eingespeist, in der eine Trennung in einen Bodenablauf (vgl. Strom (n) in Fig. 10), im Mittel 161,3 g/h, dessen Temperatur ca. 67°C betrug, der ca. 99,0 mol-% Methanol, 0,7 mol-% Dimethylcarbonat und 0,3 mol-% Wasser enthielt und der in das Puffergefäß E (vgl. Fig. 10) übergeführt wurde, sowie in ein Kopfprodukt (vgl. Strom (1) in Fig. 10), im Mittel 397,0 g/h, das ca. 84,4 mol-% Methanol, 12,6 mol-% Dimethylcarbonat sowie 3,0 mol-% Leichtsieder enthielt, und das als Kondensat mit einer Temperatur von ca. 62°C in das Puffergefäß F (vgl. Fig. 10) geleitet wurde, erfolgte.

Bei dieser Kolonne handelte es sich um eine mit Füllkörpern (Glasringe der Abmessung 4 x 4 mm) befüllte Destillationskolonne (Abmessungen: 51 x 5500 mm, Wandstärke: 1,6 mm), in der ca. 36 theoretische Trennstufen realisiert waren und die bei einem Rücklaufverhältnis von 1,5:1 arbeitete. Den unteren Abschluß der Kolonne bildete ein Kolonnenschuß mit Mantelbeheizung. Am oberen Ende der Kolonne befand sich ein liegender Doppelrohrkondensator, der mit Kühlwasser betrieben wurde und dem ein gasförmiger Strom von im Mittel 4,9 g/h entnommen wurde, der ca. 0,1 mol-% Kohlendioxid, 75,8 mol-% Methanol, 11,7 mol-% Dimethylcarbonat und 12,3 mol-% Leichtsieder enthielt (vgl. Strom (z) in Fig. 10) und der gegebenenfalls mit dem Ziel der Wiedergewinnung darin enthaltener Wertstoffe weiter aufgearbeitet wurde (in vorliegendem Beispiel nicht beschrieben).

Ca. 380,0 g/h des im Puffergefäß D (vgl. Fig. 10) befindlichen Stoffgemisches wurden nach Abkühlen auf ca. 51°C vermittelt eines zwischengeschalteten einfachen Rohrbündelwärmeaustausches, dessen Kühlleistung automatisch so geregelt wurde, daß die austretende Flüssigkeit die gewünschte Temperatur hatte, seitlich in eine bei Normaldruck betriebene kontinuierlich arbeitende Kolonne eingespeist (vgl. Strom (i1) in Fig. 10). Bei dieser Kolonne handelte es sich um eine mit Füllkörpern (Glasringe der Abmessung 4 x 4 mm) befüllte Destillationskolonne (Abmessungen 33,7 x 1500 mm, Wandstärke 1,6 mm), in der ca. 14 theoretische Trennstufe realisiert waren und die bei einem Rücklaufverhältnis von 0,2:1 arbeitete. Den unteren Abschluß der Kolonne bildete ein Kolonnenschuß mit Mantelbeheizung. Am oberen Ende der Kolonne befand sich ein liegender Doppelrohrkondensator, der mit Kühlwasser betrieben wurde.

Als Kopfprodukt der Destillation (vgl. Strom (o) in Fig. 10) wurden im Mittel 372,2 g/h Dimethylcarbonat mit einer Reinheit von 99,9 % (GC) gewonnen. Im Bodenablauf, im Mittel 7,6 g/h, befanden sich 56,2 mol-% Dimethylcarbonat und 43,8 mol-% Oxalsäuredimethylester.

Von dem im Puffergefäß B (vgl Fig. 10) befindlichen neutralisierten Bodenablauf des Methylnitrit-Reaktors (vgl. Apparatur 3 in Fig. 10) wurden ca. 231,3 g/h (vgl. Strom (g1) in Fig. 10) und von dem im Puffergefäß J (vgl. Fig. 10) befindlichen Bodenablauf des Leichtsieder-Wäschers (vgl. Apparatur 8c in Fig. 11) wurden ca. 61,8 g/h (vgl. Strom (u) in Fig. 10) jeweils seitlich in den mittleren bzw. in den oberen Bereich einer bei Normaldruck betriebenen Kolonne eingespeist. Bei dieser Kolonne handelte es sich um eine mit Füllkörpern (Glasringe der Abmessung 4 x 4 mm) befüllte Destillationskolonne (Abmessungen 42 x 3000 mm, Wandstärke 1,6 mm), in der ca. 20 theoretische Trennstufen realisiert waren und die bei einem Rücklaufverhältnis von 49,8:1 arbeitete. Den unteren Abschluß der Kolonne bildete ein Kolonnenschuß mit Mantelbeheizung. Am oberen Ende der Kolonne befand sich ein liegender Doppelrohrkondensator, der mit Kühlwasser betrieben wurde und dem ein Abgasstrom von im Mittel 10,1 g/h entnommen wurde, welcher ca. 18,2 mol-% Kohlendioxid, 38,1 mol-% Methylnitrit, 17,9 mol-% Methanol, 3,1 mol-% Dimethylcarbonat und 22,3 mol-%

Leichtsieder enthielt.

Als Kopfprodukt der Destillation (vgl. Strom (w) in Fig. 10) wurden im Mittel 13,5 g/h eines Gemisches erhalten, das durchschnittlich 0,5 mol-% Methylnitrit, 82,8 mol-% Methanol, 13,4 mol-% Dimethylcarbonat und 3,2 mol-% Leichtsieder enthielt und das in das Puffergefäß I (vgl. Fig. 10) geleitet wurde (vgl. Strom (w) in Fig. 10), dessen Inhalt gegebenenfalls einer kontinuierlichen oder diskontinuierlichen Aufarbeitung mit dem Ziel der Rückgewinnung darin enthalter Wertstoffe unterworfen wurde (nicht in dem vorligenden Beispiel beschrieben). Im Bodenablauf (vgl. Strom (p) in Fig. 10), im Mittel 90,0 g/h, befanden sich 98,1 mol-% Wasser sowie 1,9 mol-% Natriumnitrat.

Dem oberen Teil der Kolonne wurde ein flüssiger Seitenstrom von im Mittel 179,5 g/h entnommen, der eine Temperatur von ca. 66°C besaß, der ca. 91,3 mol-% Methanol, 8,5 mol-% Dimethylcarbonat und 0,2 mol-% Wasser enthielt und der dem Puffergefäß C (vgl. Fig. 6) zugeführt wurde.

Bei dem Leichtsieder-Wäscher (vgl. Apparatur 8c in Fig. 11), dem im unteren Teil der oben beschriebene gasförmige Strom (f2) (vgl. Fig. 7) zugeführt wurde, handelte es sich um eine mit Füllkörpern (Glasringe der Abmessung 4 x 4 mm) befüllte Kolonne (Abmessungen: 33,7 x 2000 mm, Wandstärke: 1,6 mm), die bei einem Druck von 3000 mbar und einer Temperatur von 0°C betrieben wurde und der im oberen Teil durchschnittlich 50,0 g/h frisches Methanol, das eine Temperatur von ca. 15°C besaß, zugeführt wurden (vgl. Ströme (b), (x2) in Fig. 11).

Die dieser Kolonne entnommenen Ströme, die eine Temperatur von 0°C besaßen, bestanden aus dem Bodenablauf, im Mittel 61,8 g/h (vgl. Strom (u) in Fig. 11), der in das Puffergefäß J (vgl. Fig. 10) geleitet wurde, und der ca. 1,6 mol-% Kohlendioxid, 4,2 mol-% Methylnitrit, 90,7 mol-% Methanol, 1,4 mol-% Dimethylcarbonat und 2,0 mol-% Leichtsieder enthielt, sowie dem gasförmigen Kopfstrom, im Mittel 40,4 g/h (vgl. Strom (x5) in Fig. 11), der ca. 3,6 mol-% Kohlenmonoxid, 56,4 mol-% Kohlendioxid, 1,3 mol-% Methanol, 28,6 mol-% Stickstoffmonoxid und 10,1 mol-% Leichtsieder enthielt und der, zusammen mit ca. 2,5 g/h Sauerstoff (vgl. Strom (c1) in Fig. 11), in den unteren Teil des Methylnitrit-Nachreaktors (vgl. Apparatur 8b in Fig. 11) eingeleitet wurde.

Bei diesem Methylnitrit-Nachreaktor handelte es sich um eine mit Füllkörpern (Glasringe der Abmessungen 4 x 4 mm) befüllte Kolonne (Abmessungen: 33,7 x 3000 mm, Wandstärke: 1,6 mm), die bei einem Druck von 3126 mbar und einer Temperatur von ca. 22°C betrieben wurde und der im oberen Teil durchschnittlich 241,9 g/h frisches Methanol, das eine Temperatur von ca. 15°C besaß (vgl. Ströme (b), (x1) in Fig. 11) sowie 161,3 g/h des flüssigen Gemisches aus dem Puffergefäß E (vgl. Fig. 10), das vermittels eines vorgeschalteten Rohrbündelwärmeaustauschers auf ca. 15°C abgekühlt wurde (vgl. Ströme (x3), (x4) in Fig. 11), zugeführt wurden. In den mittleren Bereich der Kolonne wurden im Mittel 1502,3 g/h (vgl. Strom (x10) in Fig. 11) aus den, Puffergefäß G (vgl. Fig. 11), dessen Inhalt magnetisch gerührt wurde, eingespeist.

Der dem Mittelteil der Kolonne entnommene flüssige Strom (vgl. Strom (x9) in Fig. 11), im Mittel 1448,2 g/h, der eine Temperatur von 21,9°C besaß und der ca. 1,0 mol-% Kohlendioxid, 0,9 mol-% Methylnitrit, 91,2 mol-% Methanol, 4,9 mol-% Dimethylcarbonat, 1,4 mol-% Wasser und 0,6 mol-% Leichtsieder enthielt, wurde in das Puffergefäß L (vgl. Fig. 11) geleitet. Der flüssige Bodenablauf (vgl. Strom (b1) in Fig. 11), im Mittel 482,7 g/h, der eine Temperatur von ca. 21,9°C hatte und ca. 1,0 mol-% Kohlendioxid, 0,9 mol-% Methylnitrit, 91,2 mol-% Methanol, 4,9 mol-% Dimethylcarbonat, 1,4 mol-% Wasser und 0,6 mol-% Leichtsieder enthielt, wurde in das Puffergefäß H (vgl. Fig. 10) geleitet. Der gasförmige Kopfstrom (Abgas) (vgl. Strom (t) in Fig. 11), im Mittel 17,5 g/h, hatte eine Temperatur von 17,5°C und enthielt ca. 8,3 mol-% Kohlenmonoxid, 73,5 mol-% Kohlendioxid, 0,1 mol-% Sauerstoff, 3,6 mol-% Methanol, 4,8 mol-% Stickstoff, 4,9 mol-% Methan und 4,9 mol-% Wasserstoff.

Ca. 1448,2 g/h wurden aus dem Puffergefäß L dem oberen Bereich des Methylnitrit-Desorbers (vgl. Apparatur 8a in Fig. 11) zugeführt, in dessen unteren Bereich der schon beschriebene Strom (f4) (vgl. Fig. 11) eingeleitet wurde. Bei diesem Methylnitrit-Desorber handelte es sich um eine mit Füllkörpern (Glasringe der Abmessung 4 x 4 mm) befüllte Kolonne (Abmessungen 51 x 2000 mm, Wandstärke 1,6 mm), die bei einem Druck von 3200 mbar und einer Temperatur von ca. 26°C betrieben wurde. Der flüssige Bodenablauf (vgl. Strom (x6) in Fig. 11), im Mittel 1485,6 g/h, der eine Temperatur von ca. 15°C hatte und ca. 0,7 mol-% Kohlendioxid, 0,4 mol-% Methylnitrit, 90,6 mol-% Methanol, 6,2 mol-% Dimethylcarbonat, 1,4 mol-% Wasser und 0,7 mol-% Leichtsieder enthielt, wurde in das Puffergefäß G (vgl. Fig. 11) geleitet. Der gasförmige Kopfstrom (vgl. Strom (f5) in Fig. 11), der schon oben beschrieben wurde, wurde der Methylnitrit-Synthese (vgl. Apparatur 3 in Fig. 10) zugeführt.

## Beispiel 4

### Katalysatorherstellung

Als Katalysator wurde der in Beispiel 3 beschriebene, verdünnt mit Glaskörpern, eingesetzt.

### Herstellung von Dimethylcarbonat

In einen aus dem Werkstoff 1.4571 gefertigten Rohrreaktor von ca. 3 m Länge (Abmessung des Reaktionsrohrs: 21 x 2900 mm, Wandstärke 2 mm) wurden 970 ml des wie oben beschrieben verdünnten Katalysators eingebracht,

wobei die Katalysator-Füllhöhe ca. 2800 mm betrug. Der Reaktor war mit einem von einer Kühlflüssigkeit durchströmten Mantel versehen. Die durchschnittliche Temperatur des Kühlmittels betrug 70°C.

Man ließ bei 3130 mbar durchschnittlich 1878,8 g/h eines vermittels eines vorgeschalteten Rohrbündelwärmeaustauschers auf ca. 62°C temperierten Gemisches strömen, das durchschnittlich 13,2 mol-% Kohlenmonoxid, 43,1 mol-% Kohlendioxid, 25,8 mol-% Methylnitrit, 6,6 mol-% Methanol, 1,0 mol-% Dimethylcarbonat, 0,4 mol-% Stickstoffmonoxid, 0,1 mol-% Wasser, 1000 ppm Chlorwasserstoff und 9,7 mol-% Leichtsieder (Ameisensäuremethylester, Formaldehyddimethylacetal, Methylchlorid, Stickstoff, Methan und Wasserstoff) enthielt (vgl. Strom (d2) in Fig. 13). Im kontinuierlichen Betrieb setzte sich dieses Gasgemisch aus dem am Kopf des Methylnitrit-Reaktors entströmenden Gas (vgl. Strom (d), (d1) in Fig. 13), ca. 125,5 g/h frisch zudosiertem Kohlenmonoxid (vgl. Strom (a) in Fig. 13) und ca. 1,2 g/h frisch zudosiertem Chlorwasserstoff (vgl. Strom (y) in Fig. 13) zusammen. Der den Reaktor verlassende Gasstrom (vgl. Strom (e) in Fig. 13) besaß eine Temperatur von ca. 70°C. Dieser Gasstrom wurde seitlich in einen mit Glas-Raschigringen (4 x 4 mm) befüllten Wäscher/Kondensator (Abmessung: 2000 x 33,7 mm, Wandstärke 1,6 mm) eingeleitet, in dem eine Teilkondensation erfolgte. Am Kopf dieser Apparatur war ein mantelseitig kondensierender Rohrbündelwärmeaustauscher angebracht, der aus 7 Rohren der Abmessung 1000 x 10 mm bestand (Wandstärke 1 mm) und durch Kühlung mit Kaltwasser (Temperatur < 15°C) so einreguliert wurde, daß das hinter diesem Kondensator austretende Gas (vgl. Strom (f) in Fig. 13) eine Temperatur von ca. 30°C besaß. Bei einem sich dabei einstellenden Druck von 3010 mbar enthielt dieses Gas im Mittel 3,0 mol -% Kohlenmonoxid, 48,9 mol-% Kohlendioxid, 5,8 mol-% Methylnitrit, 5,4 mol-% Methanol, 1,9 mol-% Dimethylcarbonat, 23,7 mol-% Stickstoffmonoxid, 0,1 mol-% $H_2O$ und 11,2 mol-% der oben genannten Leichtsieder. Der Bodenstrom (vgl. Strom (h) in Fig. 13), im Mittel 366,8 g/h, lief bei einer Temperatur von ca. 63°C ab, enthielt 13,2 mol-% Methanol, 85,6 mol-% Dimethylcarbonat, 0,7 mol-% Oxalsäuredimethylester sowie 0,2 mol-% Wasser und wurde in das Puffergefäß A (vgl. Fig. 13) geleitet.

Dem Rücklauf des Kondensators wurde ein flüssiger Strom von im Mittel 16,7 g/h (vgl. Strom (f7) in Fig. 13), entnommen und dem Puffergefäß G (vgl. Fig. 11), dessen Inhalt magnetisch gerührt wurde, zugeführt. Er besaß eine Temperatur von ca. 30°C und enthielt im Mittel 60,4 mol-% Methanol, 37,5 mol-% Dimethylcarbonat, 1,3 mol-% Wasser und 0,7 mol-% Leichtsieder.

Ca. 3,5 Gew.-% des am Kopf des Wäscher/Kondensators anfallenden Gasstroms (vgl. Strom (f) in Fig. 13) wurden kontinuierlich ausgeschleust un dem unteren Bereich des Leichtsieder-Wäschers (vgl. Apparatur 8c in Fig. 11) zugeführt, um der Anreicherung gasförmiger Nebenprodukte innerhalb des Kreisprozesses vorzubeugen (vgl. Strom (f2) in Fig. 13). Das verbleibende Gas wurde über einen Wärmeaustauscher sowie einen Kolbenverdichter auf einen Druck von ca. 3250 mbar und eine Temperatur von ca. 42°C gebracht (vgl. Ströme (f3), (f4) in Fig. 13) und in den unteren Bereich des Methylnitrit-Desorbers (vgl. Apparatur 8a in Fig. 11) eingeleitet. Der am Kopf dieses Methylnitrit-Desorbers gewonnene Gasstrom (vgl. Strom (f5) in Fig. 13) besaß eine Temperatur von ca. 26°C und enthielt im Mittel 3,0 mol-% Kohlenmonoxid, 49,8 mol-% Kohlendioxid, 6,4 mol-% Methylnitritt, 5,0 mol-% Methanol, 0,5 mol-% Dimethylcarbonat, 24,0 mol-% Stickstoffmonoxid und 11,2 mol-% der oben genannten Leichtsieder.

Dieser Strom wurde mit durchschnittlich zusätzlichen 6,2 g/h Stickstoffmonoxid (vgl. Strom (r) in Fig. 13) und 12,7 g/h Kohlendioxid (vgl. Ströme (s), (s1) in Fig. 13) vereinigt (vgl. Strom (f6) in Fig. 13) und mit einem Strom von ca. 97,5 g/h (vgl. Strom (b3) in Fig. 13) aus dem Puffergefäß H (vgl. Fig. 13) sowie ca. 69,4 g/h Sauerstoff (vgl. Strom (c2) in Fig. 13) in den unteren Teil des Methylnitrit-Reaktors (vgl. Apparatur 3 in Fig. 13) so eingespeist, daß eine möglichst schlagartige und vollständige Vermischung aller Reaktanten gegeben war, ohne daß dabei sicherheitstechnisch kritische Konzentrationsbereiche zündfähiger Komponenten durchschritten wurden.

Dies geschah im Falle des hier beschriebenen Beispiels auf die Weise, daß innerhalb eines ersten Statikmischerelementes die Vermischung des Kreisgases mit den frisch zudosierten Gasen Stickstoffmonoxid (vgl. Strom (r), (s1) in Fig. 13) und Kohlendioxid (vgl. Strom (s) in Fig. 13) erfolgte, gleich dahinter über eine Einstoffdüse der im wesentlichen Methanol enthaltende Strom aus dem Puffergefäß H (vgl. Fig. 13) eingesprüht wurde und unmittelbar nach der Methanol-Zugabe und noch vor dem nachfolgenden Statikmischerelement, in dem alle dann vorhandenen Reaktionskomponenten intensiv vermischt wurden, Sauerstoff (vgl. Strom (c2) in Fig. 13) zugespeist wurde.

In den oberen Teil des Reaktors wurde dabei ein Strom von im Mittel 390,1 g/h (vgl. Strom (b2) in Fig. 13) aus dem Puffergefäß H (vgl. Fig. 13) gepumpt.

Bei dem Methylnitrit-Reaktor (Abmessungen: 7000 x 51 mm, Wandstärke 1,6 mm) handelte es sich um ein Füllkörper (Glasringe der Abmessung 4 x 4 mm) enthaltendes Reaktionsgefäß, das ein inneres freies Volumen von ca. 12,6 l besaß.

Am Kopf dieser Apparatur war ein mantelseitig kondensierender Rohrbündelwärmeaustauscher angebracht, der aus 7 Rohren der Abmessung 10 x 1000 mm bestand (Wandstärke 1 mm) und dessen Kühlleistung durch Kühlung mit Kaltwasser (Temperatur < 15°C) so einreguliert wurde, daß das hinter diesem Konensator austretende Gas (vgl. Strom (d) in Fig. 13) eine Temperatur von ca. 34°C besaß. Es stand im Mittel unter einem Druck von ca. 3140 mbar und wurde kontinuierlich in den Dimethylcarbonat-Reaktor zurückgeführt (siehe oben).

Der flüssige Bodenstrom (vgl. Strom (g) in Fig. 13), im Mittel 229,1 g/h, lief bei einer Temperatur von ca. 51°C ab, enthielt ca. 38,7 mol-% Methanol, 6,0 mol-% Dimethylcarbonat, 53,9 mol-% Wasser sowie 1,1 mol-% Salpetersäure und wurde nach Neutralisation der darin enthaltenen Salpetersäure mit ca. 6,6 g/h 50 %iger Natronlauge (vgl. Strom

(q) in Fig. 13) in das Puffergefäß B (vgl. Fig. 13) geleitet (vgl. Strom (g1) in Fig. 13).

Ca. 367,9 g/h von dem im Puffergefäß A und ca. 78,0 g/h von dem im Puffergefäß F (vgl. Fig. 13) befindlichen Gemisch wurden seitlich in eine kontinuierlich arbeitende Druckkolonne (Apparatur 4 in Fig. 13) eingespeist, die in einem Druckbereich von 10072 (Boden) bis 10039 mbar (Kopf) betrieben wurde. Bei dieser Druckkolonne handelte es sich um eine mit Füllkörpern (Glasringe der Abmessung 4 x 4 mm) befüllte Destillationskolonne (Abmessungen 33,7 x 3000 mm, Wandstärke 1,6 mm), in der ca. 10 theoretische Trennstufen realisiert waren und die bei einem Rücklaufverhältniss von 1,97 : 1 arbeitete. Den unteren Abschluß der genannten Druckkolonne bildete ein Kolonnenschuß mit Mantelbeheizung. Am obereren Ende der Druckkolonne befand sich ein liegender Doppelrohrkondensator, der mit Kühlwasser betrieben wurde.

Das am Boden ablaufende flüssige Gemisch (vgl. Strom (i) in Fig. 13), im Mittel 379,9 g/h, hatte eine Temperatur von ca. 185°C. Es enthielt 99,2 mol-% Dimethylcarbonat, 0,8 mol-% Oxalsäuredimethylester und wurde in das Puffergefäß D (vgl. Fig. 13) geleitet.

Das am Kopf gewonnene Kondensat (vgl. Strom (k) in Fig. 13), im Mittel 66,0 g/h, besaß eine Temperatur von ca. 120°C und enthielt im Mittel ca. 69,1 mol-% Methanol, 22,4 mol-% Dimethylcarbonat, 0,5 mol-% Wasser sowie 7,9 mol-% Leichtsieder. Es wurde dem Puffergefäß C (vgl. Fig. 13) zugeführt, dessen Inhalt magnetisch gerührt wurde, und in das außerdem der Strom (m) (vgl. Fig. 13), im Mittel 183,5 g/h, der der Abwasser-Destillation (vgl. Apparat 6 in Fig. 13) entstammte, geleitet wurde. Der Inhalt dieses Puffergefäßes wurde durch eine Temperaturregelung auf 70°C und durch eine Druckregelung auf 10 bar gehalten.

Aus dem Puffergefäß C (vgl. Fig. 13) wurde retentatseitig ein flüssiger Strom von ca. 249,5 g/h (vgl. Strom (k1) in Fig. 13) seitlich in eine kontinuierlich arbeitende Pervaporationsanlage (vgl. Apparatur 7 in Fig. 13) eingespeist, in der eine Trennung in ein flüssiges Permeat (vgl. Strom (n) in Fig. 13), im Mittel 167,4 g/h, dessen Temperatur ca. -7°C betrug, das ca. 98,0 mol-% Methanol, 1,7 mol-% Dimethylcarbonat und 0,3 mol-% Wasser enthielt und das in das Puffergefäß E (vgl. Fig. 13) übergeführt wurde, sowie in ein flüssiges Retentat (vgl. Strom (1) in Fig. 13), im Mittel 82,1 g/h, das ca. 33,1 mol-% Methanol, 58,4 mol-% Dimethylcarbonat sowie 8,5 mol-% Leichtsieder enthielt und das mit einer Temperatur von ca. 63°C in das Puffergefäß F (vgl. Fig. 13) geleitet wurde, erfolgte. Der Inhalt dieses Puffergefäßes wurde durch eine Temperaturregelung auf 70°C und durch eine Druckregelung auf 1300 mbar gehalten. Der dabei anfallende gasförmige Strom von im Mittel 4,1 g/h, der ca. 0,2 mol-% Kohlendioxid, 52,9 mol-% Methanol, 26,1 mol-% Dimethylcarbonat und 20,7 mol-% Leichtsieder enthielt (vgl. Strom (z) in Fig. 13) wurde gegebenenfalls, zum Beispiel mit dem Ziel der Wiedergewinnung darin enthaltener Wertstoffe, weiter aufgearbeitet (im vorliegenden Beispiel nicht beschrieben).

Bei der Pervaporationsanlage handelte es sich um drei in Serie geschaltete Zellen mit Membranen des Typs AERK 300 der Firma GFT, deren Membranfläche jeweils 200 $cm^2$ betrug. Die Einheit wurde auf der Permeatseite mit einem Druck von 20 mbar und einer Temperatur von -7°C und auf der Retentatseite mit einem Druck von 1300 mbar und einer Temperatur von 70°C betrieben.

Ca. 379,9 g/h des im Puffergefäß D (vgl. Fig. 13) befindlichen Stoffgemisches wurden nach Abkühlen auf ca. 51°C vermittels eines zwischengeschalteten einfachen Rohrbündelwärmeaustauschers, dessen Kühlleistung automatisch so geregelt wurde, daß die austretende Flüssigkeit die gewünschte Temperatur hatte, seitlich in eine bei Normaldruck betriebene kontinuierlich arbeitende Kolonne eingespeist (vgl. Strom (i1) in Fig. 13). Bei dieser Kolonne handelte es sich um eine mit Füllkörpern (Glasringe der Abmessung 4 x 4 mm) befüllte Destillationskolonne (Abmessungen 33,7 x 1500 mm, Wandstärke 1,6 mm), in der ca. 14 theoretische Trennstufen realisiert waren und die bei einem Rücklaufverhältnis von 0,2 : 1 arbeitete. Den unteren Abschluß der Kolonne bildete ein Kolonnenschuß mit Mantelbeheizung. Am oberen Ende der Kolonne befand sich ein liegender Doppelrohrkondensator, der mit Kühlwasser betrieben wurde.

Als Kopfprodukt der Destillation (vgl. Strom (o) in Fig. 13) wurden im Mittel 372,2 g/h Dimethylcarbonat mit einer Reinheit > 99,9 % (GC) gewonnen. Im Bodenablauf, im Mittel 7,6 g/h, befanden sich 56,1 mol-% Dimethylcarbonat und 43,9 mol-% Oxalsäuredimethylester.

Von dem im Puffergefäß B (vgl. Fig. 13) befindlichen neutralisierten Bodenablauf des Methylnitrit-Reaktors (vgl. Apparatur 3 in Fig. 13) wurden ca. 235,6 g/h (vgl. Strom (g1) in Fig. 13) und von dem im Puffergefäß J (vgl. Fig. 13) befindlichen Bodenablauf des Leichtsieder-Wäschers (vgl. Apparatur 8c in Fig. 11) wurden ca. 61,8 g/h (vgl. Strom (u) in Fig.13) jeweils seitlich in den mittleren bzw. den oberen Bereich einer bei Normaldruck betriebenen Kolonne eingespeist. Bei dieser Kolonne handelte es sich um eine mit Füllkörpern (Glasringe der Abmessung 4 x 4 mm) befüllte Destillationskolonne (Abmessungen 42 x 3000 mm, Wandstärke 1,6 mm), in der ca. 20 theoretische Trennstufen realisiert waren und die bei einem Rücklaufverhältnis von 49,8:1 arbeitete. Den unteren Abschluß der Kolonne bildete ein Kolonnenschuß mit Mantelbeheizung. Am oberen Ende der Kolonne befand sich ein liegender Doppelrohrkondensator, der mit Kühlwasser betrieben wurde und dem ein Abgasstrom von im Mittel 10,1 g/h entnommen wurde, der ca. 18,5 mol-% Kohlendioxid, 38,1 mol-% Methylnitrit, 17,9 mol-% Methanol, 3,2 mol-% Dimethylcarbonat und 22,2 mol-% Leichtsieder enthielt.

Als Kopfprodukt der Destillation (vgl. Strom (w) in Fig. 13) wurden im Mittel 13,6 g/h eines Gemisches erhalten, das durchschnittlich 0,5 mol-% Methylnitrit, 82,7 mol-% Methanol, 13,5 mol-% Dimethylcarbonat und 3,2 mol-% Leichtsieder enthielt und das in das Puffergefäß I (vgl. Fig. 13) geleitet wurde (vgl. Strom (w) in Fig. 13), dessen Inhalt gegebenenfalls kontinuierlich oder diskontinuierlich mit dem Ziel der Wiedergewinnung darin enthaltener Wertstoffe einer weiteren Aufarbeitung zugeführt wurde. Im Bodenablauf (vgl. Strom (p) in Fig. 13), im Mittel 90,2 g/h, befanden sich 98,1 mol-% Wasser sowie 1,9 mol-% Natriumnitrat.

Dem oberen Teil der Kolonne wurde ein flüssiger Seitenstrom von im Mittel 183,5 g/h entnommen, der eine Temperatur von ca. 66°C besaß und ca. 90,5 mol-% Methanol, 9,3 mol-% Dimethylcarbonat und 0,2 mol-% Wasser enthielt, und der dem Puffergefäß C (vgl. Fig. 13) zugeführt wurde.

Bei dem Leichtsieder-Wäscher (vgl. Apparatur 8c in Fig. 11), dem im unteren Teil der oben beschriebene Strom (f2) (vgl. Fig. 11) zugeführt wurde, handelte es sich um eine mit Füllkörpern (Glasringe der Abmessung 4 x 4 mm) befüllte Kolonne (Abmessungen 33,7 x 2000 mm, Wandstärke 1,6 mm), die bei einem Druck von 3000 mbar und einer Temperatur von 0°C betrieben wurde und dem im oberen Teil im Mittel 50,0 g/h frisches Methanol, das eine Temperatur von ca. 15°C besaß, zugeführt wurden (vgl. Ströme (b), (x2) in Fig. 11).

Die dieser Kolonne entnommenen Ströme, die eine Temperatur von 0°C besaßen, bestanden aus dem Bodenablauf, im Mittel 61,8 g/h (vgl. Strom (u) in Fig. 11), der in das Puffergefäß J (vgl. Fig. 13) geleitet wurde und der ca. 1,6 mol-% Kohlendioxid, 4,2 mol -% Methylnitrit, 90,7 mol-% Methanol, 1,4 mol-% Dimethylcarbonat und 2,0 mol-% Leichtsieder enthielt, sowie dem gasförmigen Kopfstrom, im Mittel 40,5 g/h (vgl. Strom (x5) in Fig. 11), der ca. 3,6 mol-% Kohlenmonoxid, 56,4 mol-% Kohlendioxid, 1,3 mol-% Methanol, 28,6 mol-% Stickstoffmonoxid und 10,1 mol-% Leichtsieder enthielt und der in den unteren Teil des Methylnitrit-Nachreaktors (vgl. Apparatur 8b in Fig 11), zusammen mit ca. 2,5 g/h Sauerstoff (vgl. Strom (c1) in Fig. 11), eingeleitet wurde.

Bei diesem Methylnitrit-Nachreaktor handelte es sich um eine mit Füllkörpern (Glasringe der Abmessung 4 x 4 mm) befüllte Kolonne (Abmessungene 33,7 x 3000 mm, Wandstärke 1,6 mm), die bei einem Druck von 3126 mbar und einer Temperatur von ca. 22°C betrieben wurde und der im oberen Teil im Mittel 240,9 g/h frisches Methanol mit einer Temperatur von ca. 15°C (vgl. Ströme (b), (x1) in Fig. 11) sowie im Mittel 167,4 g/h des flüssigen Gemisches aus dem Puffergefäß E (vgl. Fig. 13), das vermittels eines vorgeschalteten Rohrbündelwärmeaustauschers auf ca. 15°C aufgeheizt wurde (vgl. Ströme (x3), (x4) in Fig. 11), zugeführt wurden. In den mittleren Bereich der Kolonne wurden im Mittel 1516,9 g/h (vgl. Strom (x10) in Fig. 11) aus dem Puffergefäß G (vgl. Fig. 11) dessen Inhalt magnetisch gerührt wurde, eingespeist.

Der dem Mittelteil der Kolonne entnommene flüssige Strom (vgl. Strom (x9) in Fig. 11), im Mittel 1462,9 g/h, der eine Temperatur von 21,9°C besaß und der ca. 1,0 mol-% Kohlendioxid, 0,9 mol-% Methylnitrit, 90,9 mol-% Methanol, 5,3 mol-% Dimethylcarbonat, 1,4 mol-% Wasser und 0,6 mol-% Leichtsieder enthielt, wurde in das Puffergefäß L (vgl. Fig. 11) geleitet. Der flüssige Bodenablauf (vgl. Strom (b1) in Fig. 11), im Mittel 487,6 g/h, der eine Temperatur von ca. 21,9°C hatte und ca. 1,0 mol-% Kohlendioxid, 0,9 mol-% Methylnitrit, 90,9 mol-% Methanol, 5,3 mol-% Dimethylcarbonat, 1,4 mol-% Wasser und 0,6 mol-% Leichtsieder enthielt, wurde in das Puffergefäß H (vgl. Fig. 13) geleitet. Der gasförmige Kopfstrom (Abgas) (vgl. Strom (t) in Fig. 11), im Mittel 17,8 g/h, hatte eine Temperatur von 17,5°C und enthielt ca. 8,1 mol-% Kohlenmonoxid, 73,9 mol-% Kohlendioxid, 0,1 mol-% Sauerstoff, 3,6 mol-% Methanol, 4,7 mol-% Stickstoff, 4,8 mol-% Methan und 4,8 mol-% Wasserstoff.

Ca. 1462,9 g/h wurden aus dem Puffergefäß L dem oberen Bereich des Methylnitrit-Desorbers (vgl. Apparatur 8a in Fig. 11) zugeführt, in dessen unteren Bereich der schon beschriebene Strom (f4) (vgl. Fig. 11) eingeleitet wurde. Bei diesem Methylnitrit-Desorber handelte es sich um eine mit Füllkörpern (Glasringe der Abmessung 4 x 4 mm) befüllte Kolonne (Abmessungen 51 x 2000 mm, Wandstärke 1,6 mm), die bei einem Druck von 3200 mbar und einer Temperatur von ca. 26°C betrieben wurde. Der flüssige Bodenablauf (vgl. Strom (x6) in Fig. 11), im Mittel 1500,2 g/h, der eine Temperatur von ca. 15°C hatte und ca. 0,7 mol-% Kohlendioxid, 0,4 mol-% Methylnitrit, 90,4 mol-% Methanol, 6,4 mol-% Dimethylcarbonat, 1,4 mol-% Wasser und 0,7 mol-% Leichtsieder enthielt, wurde in das Puffergefäß G (vgl. Fig. 11) geleitet. Der gasförmige Kopfstrom (vgl. Strom (f5) in Fig. 11), der schon oben beschrieben wurde, wurde der Methylnitrit-Synthese (vgl. Apparatur 3 in Fig. 13) zugeführt.

## Patentansprüche

1. Verfahren zur kontinuierlichen Herstellung von Dimethylcarbonat aus Kohlenmonoxid und Methylnitrit und Recyclisieren des dabei entstehenden Stickoxids zur erneuten Bildung von Methylnitrit, dadurch gekennzeichnet, daß man

   (a) Kohlenmonoxid und Methylnitrit in der Gasphase in Gegenwart eines heterogenen, ein Platinmetall enthaltenden Katalysators, bevorzugt eines Palladium enthaltenden Trägerkatalysators, und eines Inertgases im Temperaturbereich von 50 bis 170°C, vorzugsweise von 70 bis 150°C, und im Druckbereich von 1 bis 5 bar, vorzugsweise 2-4 bar, zur Reaktion bringt, wobei dem Gasgemisch als Aktivator Halogenwasserstoff, Halogen, Chlorameisensäuremethylester und/oder andere unter den Reaktionsbedingungen aktivierend wirkendes Halogen enthaltende Substanzen in einer Konzentration von 0-3000 ppm, bevorzugt 10-1000 ppm zugesetzt wird,

   (b) das bei (a) anfallende Gemisch in gasförmige und flüssige Reaktionsprodukte trennt, einen Teil von 0-7

Gew.-%, bevorzugt 0,1-5 Gew.-% des Gasstromes ausspeist, die darin enthaltenden Leichtsieder abtrennt und gegebenenfalls einer weiteren Aufarbeitung zuführt, das darin enthaltende Stickstoffmonoxid mit Sauerstoff und Methanol zu Methylnitrit umsetzt, abtrennt und in das Verfahren zurückführt und die verbleibenden akkumulierten, inerten Gase aus dem Verfahren ausschleust,

(c) die gasförmigen Produkte zur erneuten Bildung des Methylnitrits mit Methanol, Sauerstoff sowie gegebenenfalls frisch zugesetztem Stickoxid oder Stickoxidäquivalenten umsetzt, wobei das Gasgemisch, welches das neu gebildete Methylnitrit enthält, abgeleitet und wiederum der Herstellung von Dimethylcarbonat zugeführt wird, und Wasser sowie gegebenenfalls entstehende weitere flüssige Nebenprodukte ebenfalls abgeleitet und, bevorzugt nach einer nachgeschalteten Rückgewinnung darin enthaltener Wertstoffe, ausgeschleust werden und

(d) die flüssigen Produkte von (b) einer destillativen Auftrennung unterwirft, in der das gesamte Produktgemisch zunächst einer ersten Destillation unterworfen wird, die bei einem Druck von 1 bis 25 bar, vorzugsweise von 1 bis 12 bar, durchgeführt wird, und dann entweder

(e1) das Kopfprodukt aus der ersten Destillation einer weiteren bei einem Druck von 200 bis 1500 mbar durchgeführten Destillation zuführt, in der als Bodenprodukt ein methanolreicher Ablauf erhalten wird, der zur Herstellung von Dimethylcarbonat, bevorzugt in den Teilschritt zur erneuten Bildung des Methylnitrits, zurückgeführt wird, und in der ein Kopfprodukt anfällt, das, gegebenenfalls gemeinsam mit anderen Rückströmen, wiederum in die erste Destillation zurückgeführt wird, oder

(e2) das Kopfprodukt aus der ersten Destillation einer Pervaporation oder einer Dampfpermeation, die auf der Retentatseite bei einer Temperatur von 20 bis 150°C und einem Druck von 0,2 bis 10 bar und auf der Permeatseite bei einer Temperatur von -30 bis +30°C und einem Druck von 0,5-500 mbar betrieben wird, zuführt, in der als Permeat ein methanolreicher Abstrom erhalten wird, der zur Herstellung von Dimethylcarbonat, bevorzugt in den Teilschritt zur Regenerierung des Methylnitrits, zurückgeführt wird, und in der ein Retentat anfällt, das, gegebenenfalls gemeinsam mit anderen Rückströmen, wiederum in die erste Destillation zurückgeführt wird, und

(f) reines Dimethylcarbonat durch Destillation des als Bodenlauf der ersten Destillation anfallenden Gemisches gewinnt.

2. Verfahren nach Anspruch 1, dadurch gekennzeichnet, daß als Katalysatoren eine oder mehrere auf Trägersubstanzen, bevorzugt auf Aluminiumoxiden, Aktivkohlen, Metallphosphaten, Zeolithen, Alumosilikaten und Heteropolysäuren, besonders bevorzugt auf Aluminiumoxiden und Aktivkohlen aufgebrachte, Palladium enthaltende Verbindungen, denen gegebenenfalls weitere Promotoren zugesetzt wurden, verwendet werden.

3. Verfahren nach Anspruch 1, dadurch gekennzeichnet, daß als Inertgas Kohlendioxid, Stickstoff oder Argon, bevorzugt Stickstoff oder Kohlendioxid, besonders bevorzugt Kohlendioxid verwendet wird.

4. Verfahren nach Anspruch 1, dadurch gekennzeichnet, daß zur Trennung des bei (a) anfallenden Gemisches ein als Wäscher/Kondensator ausgebildeter Apparat eingesetzt wird.

5. Verfahren nach Anspruch 1, dadurch gekennzeichnet, daß die Einspeisung der bei (b) abgetrennten gasförmigen Reaktionsprodukte, des Sauerstoffs, des frisch zugesetzten Inertgases, des Stickoxids bzw. Stickoxidäquivalents und mindestens eines Teil des Methanols in die zur erneuten Bildung des Methylnitrits vorgesehenen Apparatur unter Verwendung eines Statikmischers und/oder einer oder mehrerer Einstoff- oder Zweistoffdüsen, bevorzugt unter Verwendung einer Kombination aus einem Statikmischer und einer oder mehrerer Einstoff- oder Zweistoffdüsen, erfolgt.

6. Verfahren nach Anspruch 1, dadurch gekennzeichnet, daß das als Bodenablauf bei der erneuten Bildung des Methylnitrits gemäß (c) anfallende Gemisch, bevorzugt nach Neutralisation darin enthaltener saurer Komponenten, einer Destillation zugeführt wird, bei der als erneuter Bodenablauf wäßrige Abfälle und bei der als Kopfprodukt Wertstoffe, bevorzugt Methanol und Dimethylcarbonat enthaltende Kondensate, erhalten werden, die in das Gesamtverfahren, bevorzugt in den Reaktor zur erneuten Bildung des Methylnitrits, in die Druckdestillation gemäß (d), in die Destillation gemäß (e1) oder in die Pervaporation bzw. Dampfpermeation gemäß (e2) zurückgeführt werden.

7. Verfahren nach Anspruch 2, dadurch gekennzeichnet, daß Aluminiumoxide oder A-Kohlen, bevorzugt Aluminiumoxide als Träger eingesetzt werden.

8. Verfahren nach Anspruch 1, dadurch gekennzeichnet, daß als Aktivatoren Chorwasserstoff und/oder Chlor und/oder Chlorameisensäuremethylester eingesetzt wird (werden).

## Claims

1. Process for the continuous preparation of dimethyl carbonate from carbon monoxide and methyl nitrite and for recycling the nitrogen oxide thereby formed for renewed formation of methyl nitrite, characterized in that

   (a) carbon monoxide and methyl nitrite are reacted in the gas phase in the presence of a heterogeneous catalyst comprising a platinum metal, preferably a supported catalyst comprising palladium, and an inert gas in the temperature range from 50 to 170°C, preferably from 70 to 150°C, and in the pressure range from 1 to 5 bar, preferably 2-4 bar, wherein hydrogen halide, halogen, methyl chloroformate and/or other halogen-containing substances which have an activating effect under the reaction conditions are added as activator in a concentration of 0-3000 ppm, preferably 10-1000 ppm, to the gas mixture,
   (b) the mixture obtained in (a) is separated into gaseous and liquid reaction products, a portion of 0.7% by weight, preferably 0.1-5% by weight, of the gas stream is withdrawn, the low boilers present therein are separated off and supplied, if desired, to a subsequent reprocessing stage, the nitrogen monoxide present therein is reacted with oxygen and methanol to form methyl nitrite, which is separated off and recycled to the process, and the remaining accumulated, inert gases are removed from the process,
   (c) the gaseous products are reacted with methanol, oxygen and if appropriate freshly added nitrogen oxide or nitrogen oxide equivalents for renewed formation of the methyl nitrite, the gas mixture which contains the newly formed methyl nitrite being led off and recycled again to the preparation of dimethyl carbonate, and water and any other liquid by-products formed also being led off and removed from the circulation, preferably after subsequent recovery of the useful substances contained therein, and
   (d) the liquid products from (b) are subjected to separation by distillation, in which the entire product mixture is initially subjected to a first distillation, which is carried out under a pressure of 1 to 25 bar, preferably 1 to 12 bar, and then either
   (e1) the top product from the first distillation is fed to another distillation carried out under a pressure of 200 to 1500 mbar, in which a methanol-rich discharge is obtained as the bottom product, which is recycled to the preparation of dimethyl carbonate, preferably to the component step for renewed formation of the methyl nitrite, and in which a top product is obtained which is recycled again to the first distillation, if appropriate together with other return streams, or
   (e2) the top product from the first distillation is fed to a pervaporation or a vapour permeation, which is operated on the retained material side at a temperature of 20 to 150°C under a pressure of 0.2 to 10 bar and on the permeate side at a temperature of -30 to +30°C under a pressure of 0.5-500 mbar, in which a methanol-rich outflow is obtained as the permeate, which is recycled to the preparation of dimethyl carbonate, preferably to the component step for regeneration of the methyl nitrite, and in which a retained material is obtained which is recycled to the first distillation, if appropriate together with other return streams, and
   (f) pure dimethyl carbonate is obtained by distillation of the mixture obtained as the bottom running of the first distillation.

2. Process according to Claim 1, characterized in that one or more palladium-containing compounds which have been applied to support substances, preferably to aluminium oxides, active charcoals, metal phosphates, zeolites, alumosilicates and heteropolyacids, particularly preferably to aluminium oxides and active charcoals, and to which further promoters have been added, if appropriate, are used as the catalysts.

3. Process according to Claim 1, characterized in that carbon dioxide, nitrogen or argon, preferably nitrogen or carbon dioxide, particularly preferably carbon dioxide, is used as the inert gas.

4. Process according to Claim 1, characterized in that an apparatus constructed as a scrubber/condenser is employed for separation of the mixture obtained in (a).

5. Process according to Claim 1, characterized in that the gaseous reaction products separated off at (b), the oxygen, the freshly added inert gas, the nitrogen oxide or nitrogen oxide equivalent and at least some of the methanol are fed into the apparatus intended for renewed formation of methyl nitrite using a static mixer and/or one or more one-component or two-component nozzles, preferably using a combination of a static mixer and one or more one-component or two-component nozzles.

6. Process according to Claim 1, characterized in that the mixture obtained as the bottom discharge during renewed formation of methyl nitrite according to (c) is fed to a distillation, preferably after neutralization of acid components contained therein, in which aqueous waste products are obtained as a renewed bottom discharge and in which useful substances, preferably condensates containing methanol and dimethyl carbonate, are obtained as the top product and are recycled to the overall process, preferably into the reactor for renewed formation of methyl nitrite or into the pressure distillation according to (d), into the distillation according to (e1) or into the pervaporation or vapour permeation according to (e2).

7. Process according to Claim 2, characterized in that aluminium oxides or active charcoals, preferably aluminium oxides, are employed as supports.

8. Process according to Claim 1, characterized in that hydrogen chloride and/or chlorine and/or methyl chloroformate are (is) employed as activator(s).

**Revendications**

1. Procédé pour la préparation en continu de carbonate de diméthyle à partir de monoxyde de carbone et de nitrite de méthyle et recyclage de l'oxyde d'azote qui se forme en l'occurrence pour une formation renouvelée du nitrite de méthyle, caractérisé en ce que

(a) on amène à réagir du monoxyde de carbone et du nitrite de méthyle en phase gazeuse en présence d'un catalyseur hétérogène contenant un métal du groupe du platine, de préférence un catalyseur déposé sur un support contenant du palladium et d'un gaz inerte dans le domaine de température de 50 à 170°C, de préférence de 70 à 150°C et dans le domaine de pression de 1 à 5 bar, de préférence de 2 à 4 bar, en ajoutant au mélange gazeux à titre d'activateur, de l'acide halogénhydrique, un halogène, l'ester méthylique de l'acide chloroformique et/ou d'autres substances halogénées ayant un effet d'activation dans les conditions réactionnelles, en une concentration de 0-3000 ppm, de préférence de 10-1000 ppm,

(b) on sépare en produits réactionnels gazeux et liquides le mélange obtenu sous (a); on évacue une partie de 0-7% en poids, de préférence de 0,1 à 7% en poids du courant de gaz; on sépare les composants à bas point d'ébullition qui y sont contenus et on les achemine éventuellement à un traitement ultérieur; on fait réagir, avec de l'oxygène et du méthanol, le monoxyde d'azote qui y est contenu pour obtenir du nitrite de méthyle, on le sépare et on le recycle dans le procédé et on écluse hors du procédé les gaz inertes subsistants qui se sont accumulés,

(c) on fait réagir les produits gazeux pour une formation renouvelée du nitrite de méthyle, avec du méthanol, de l'oxygène et éventuellement de l'oxyde d'azote ou des équivalents d'oxyde d'azote fraîchement ajoutés, le mélange de gaz qui contient le nitrite de méthyle nouvellement formé étant dérivé et recyclé à la préparation du carbonate de diméthyle, et l'eau ainsi que d'autres sous-produits liquides qui se sort éventuellement formés étant également dérivés et éclusés au dehors de préférence après une récupération montée à la suite des substances valables qui y sont contenues, et,

(d) on soumet les produits liquides de (b) à une séparation par distillation dans laquelle on soumet le mélange total obtenu sous forme de produit, d'abord à une première distillation que l'on effectue sous une pression de 1 à 25 bar, de préférence de 1 à 12 bar, et ensuite

(e1) soit on achemine le produit de tête provenant de la première distillation à une distillation ultérieure réalisée sous une pression de 200 à 1500 mbar dans laquelle on obtient, comme produit de bas de colonne, un écoulement riche en méthanol, que l'on recycle pour la préparation du carbonate de diméthyle, de préférence dans l'étape partielle pour la formation renouvelée du nitrite de méthyle, et dans laquelle on obtient un produit de tête qui est à nouveau recyclé à la première distillation, éventuellement de manière conjointe avec d'autres courants de retour,

(e2) soit on achemine le produit de tête provenant de la première distillation à une pervaporation ou à une perméation à la vapeur que l'on met en oeuvre, côté rétentat, à une température de 20 à 150°C et sous une pression de 0,2 à 10 bar et, côté perméat, à une température de -30 à +30°C et sous une pression de 0,5-500 mbar, dans laquelle on obtient un écoulement riche en méthanol sous forme de perméat que l'on recycle à la

préparation du carbonate de diméthyle, de préférence dans l'étape partielle pour la régénération du nitrite de méthyle, et dans laquelle on obtient un rétentat que l'on recycle à nouveau dans la première distillation, éventuellement de manière conjointe avec d'autres courants de retour, et

(f) on obtient du carbonate de diméthyle pur par distillation du mélange obtenu sous forme de courant de bas de colonne de la première distillation.

**2.** Procédé selon la revendication 1, caractérisé en ce qu'on utilise comme catalyseurs, un ou plusieurs composés contenant du palladium appliqué sur des substances de support, de préférence sur des oxydes d'aluminium, sur des charbons actifs, sur des phosphates métalliques, sur des zéolithes, sur des alumosilicates et sur des hétéropolyacides, de manière particulièrement préférée, sur des oxydes d'aluminium et sur des charbons actifs, composés auxquels on a éventuellement ajouté d'autres promoteurs.

**3.** Procédé selon la revendication 1, caractérisé en ce qu'on utilise, comme gaz inerte, du dioxyde de carbone, de l'azote ou de l'argon, de préférence de l'azote ou du dioxyde de carbone, de manière particulièrement préférée du dioxyde de carbone.

**4.** Procédé selon la revendication 1, caractérisé en ce que, pour la séparation du mélange obtenu sous (a), on met en oeuvre un appareil réalisé sous forme d'un laveur/condenseur.

**5.** Procédé selon la revendication 1, caractérisé en ce que l'introduction des produits réactionnels gazeux séparés sous (b), de l'oxygène, du gaz inerte fraîchement ajouté, de l'oxyde d'azote, respectivement de l'équivalent d'oxyde d'azote et d'au moins une partie du méthanol dans l'appareil prévu pour la formation renouvelée du nitrite de méthyle a lieu en utilisant un mélangeur statique et/ou une ou plusieurs tuyères unitaires ou binaires, de préférence en utilisant une combinaison constituée par un mélangeur statique et par une ou plusieurs tuyères unitaires ou binaires.

**6.** Procédé selon la revendication 1, caractérisé en ce qu'on achemine à une distillation le mélange obtenu selon (c) sous forme d'écoulement de bas de colonne lors de la formation renouvelée du nitrite de méthyle, de préférence après neutralisation des composants acides qui y sont contenus, distillation au cours de laquelle on obtient des eaux usées sous forme d'écoulement de bas de colonne renouvelé et au cours de laquelle on obtient comme produit de tête de colonne des substances valables, de préférence des condensats contenant du méthanol et du carbonate de diméthyle, que l'on recycle dans le procédé global, de préférence dans le réacteur pour la formation renouvelée du nitrite de méthyle, dans la distillation sous pression, selon (d), dans la distillation selon (e1) ou dans la pervaporation respectivement dans la perméation à la vapeur selon (e2).

**7.** Procédé selon la revendication 2, caractérisé en ce qu'on met en oeuvre comme support, des oxydes d'aluminium ou des charbons A, de préférence des oxydes d'aluminium.

**8.** Procédé selon la revendication 1, caractérisé en ce qu'on met en oeuvre comme activateurs, de l'acide chlorhydrique et/ou du chlore et/ou de l'ester méthylique de l'acide chloroformique.

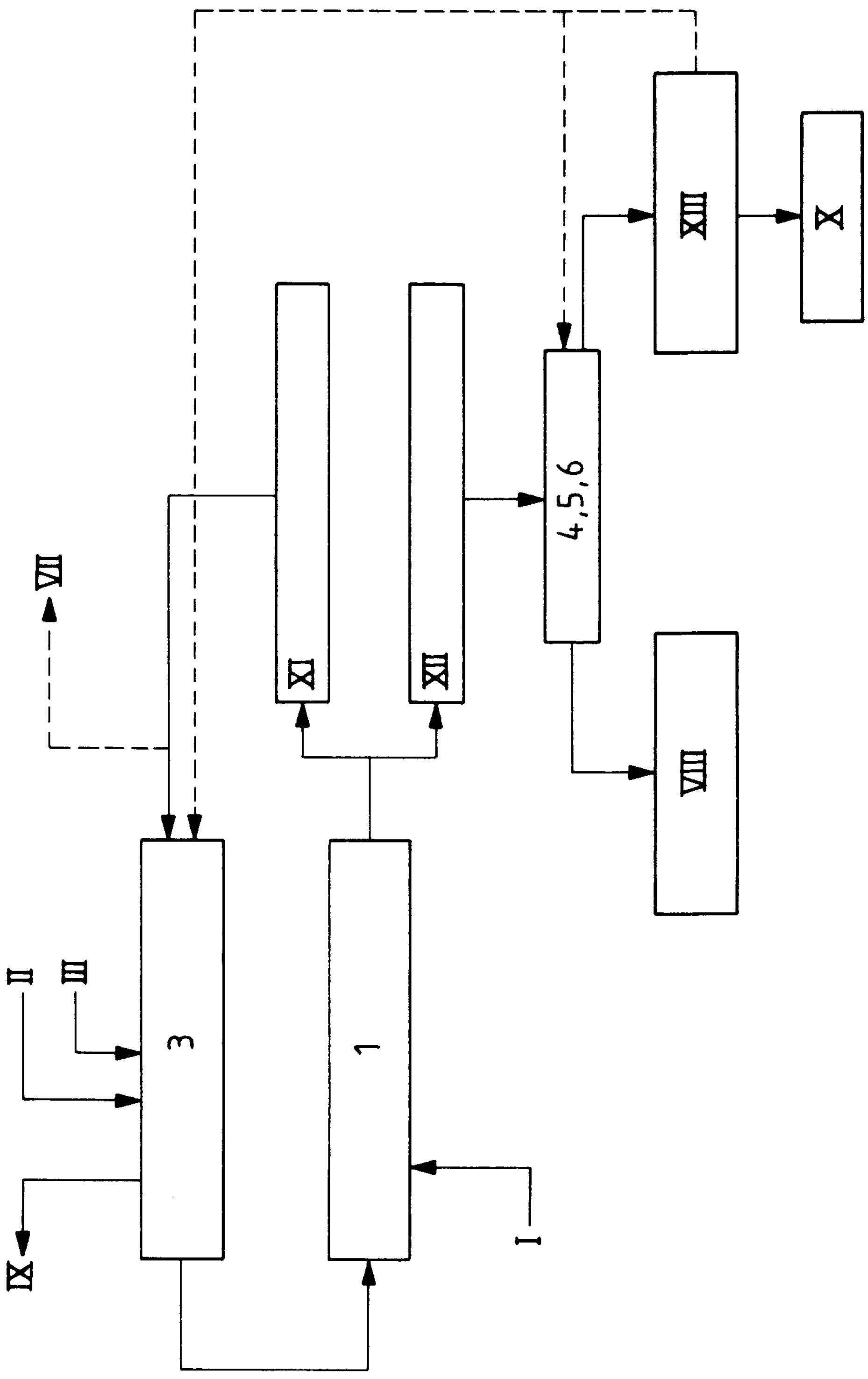


Fig.1

Fig. 2

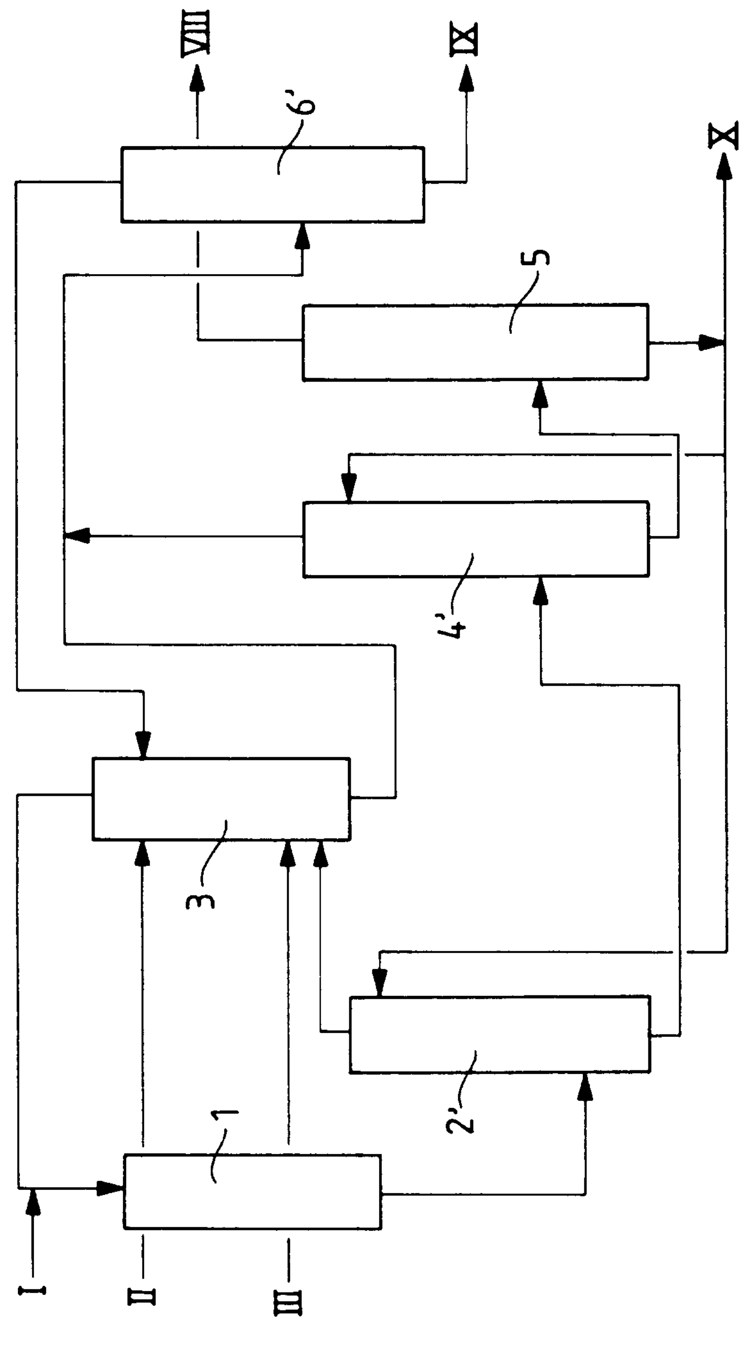

Fig. 3

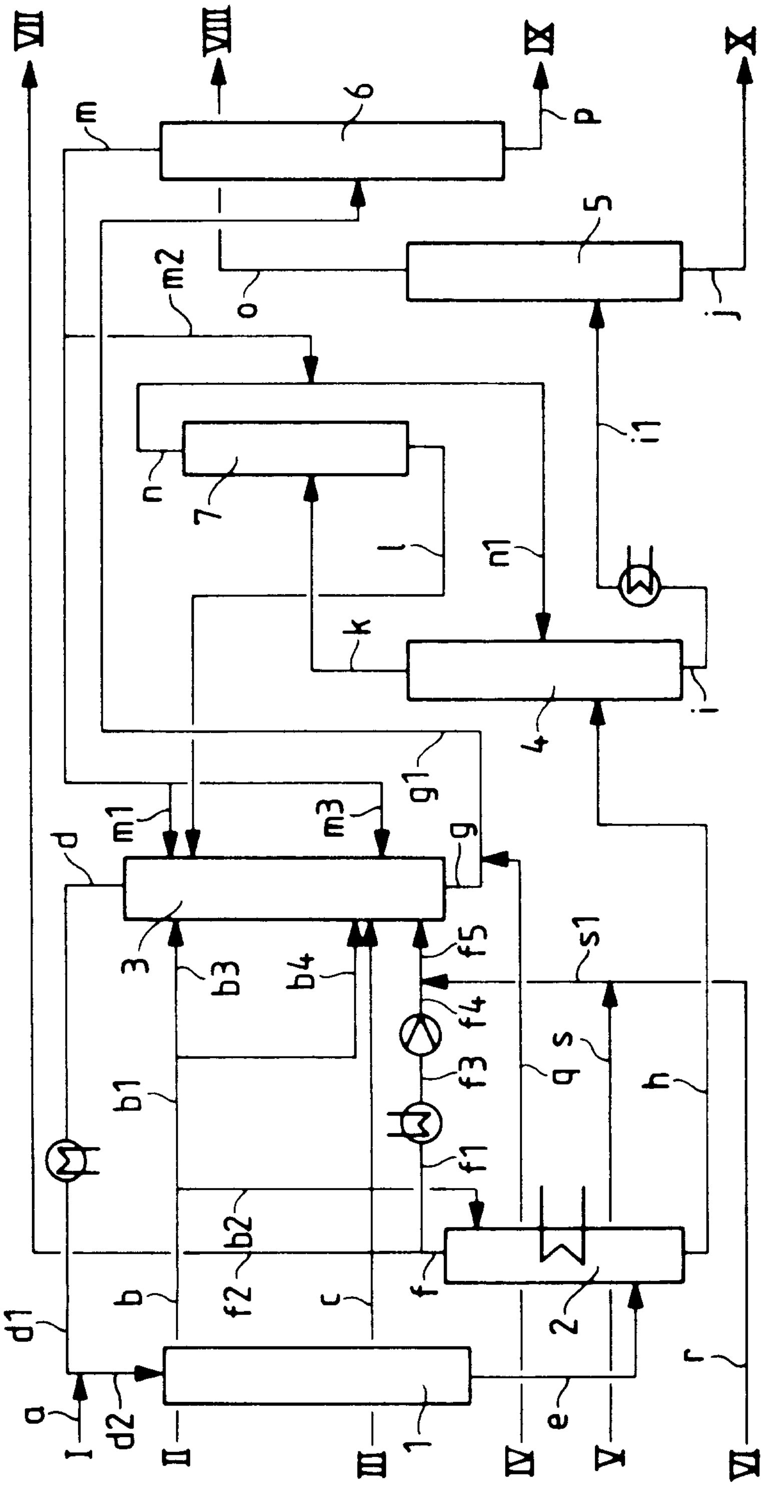

I
II
III
IV
V
VI
VII
VIII
IX
X
1
2
3
4
5
6
7
a
b
b1
b2
b3
b4
c
d
d1
d2
e
f
f1
f2
f3
f4
f5
g
g1
h
i
i1
j
k
l
m
m1
m2
m3
n
n1
o
p
q
r
s
s1

Fig. 4

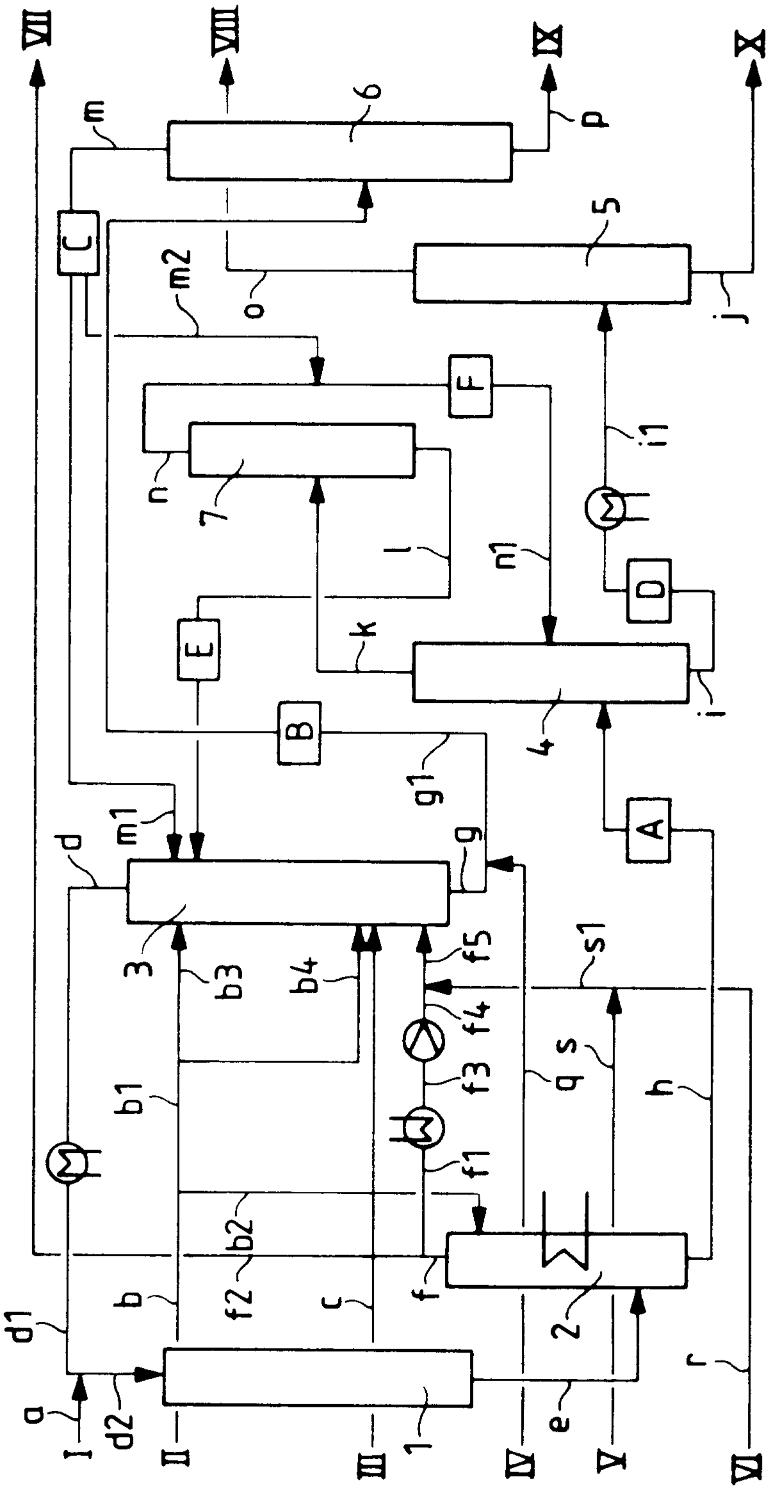
I
II
III
IV
V
VI
VII
VIII
IX
X
A
B
C
D
E
F
1
2
3
4
5
6
7
a
b
b1
b2
b3
b4
c
d
d1
d2
e
f
f1
f2
f3
f4
f5
g
g1
h
i
i1
j
k
l
m
m1
m2
n
n1
o
p
q
r
s
s1

Fig. 5

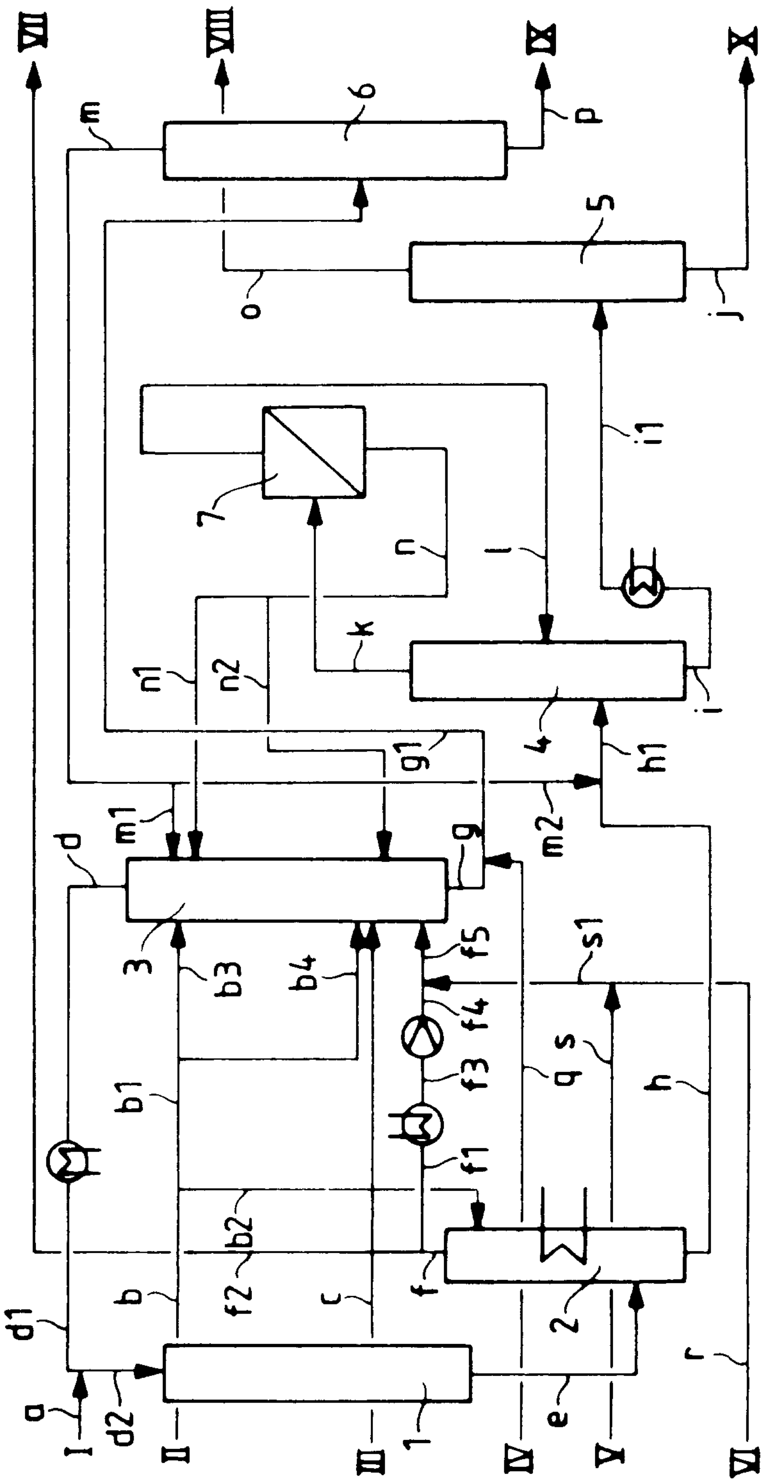
I
II
III
IV
V
VI
VII
VIII
IX
X
a
b
b1
b2
b3
b4
c
d
d1
d2
e
f
f1
f2
f3
f4
f5
g
g1
h
h1
i
i1
j
k
l
m
m1
m2
n
n1
n2
o
p
q
r
s
s1
1
2
3
4
5
6
7

Fig. 6

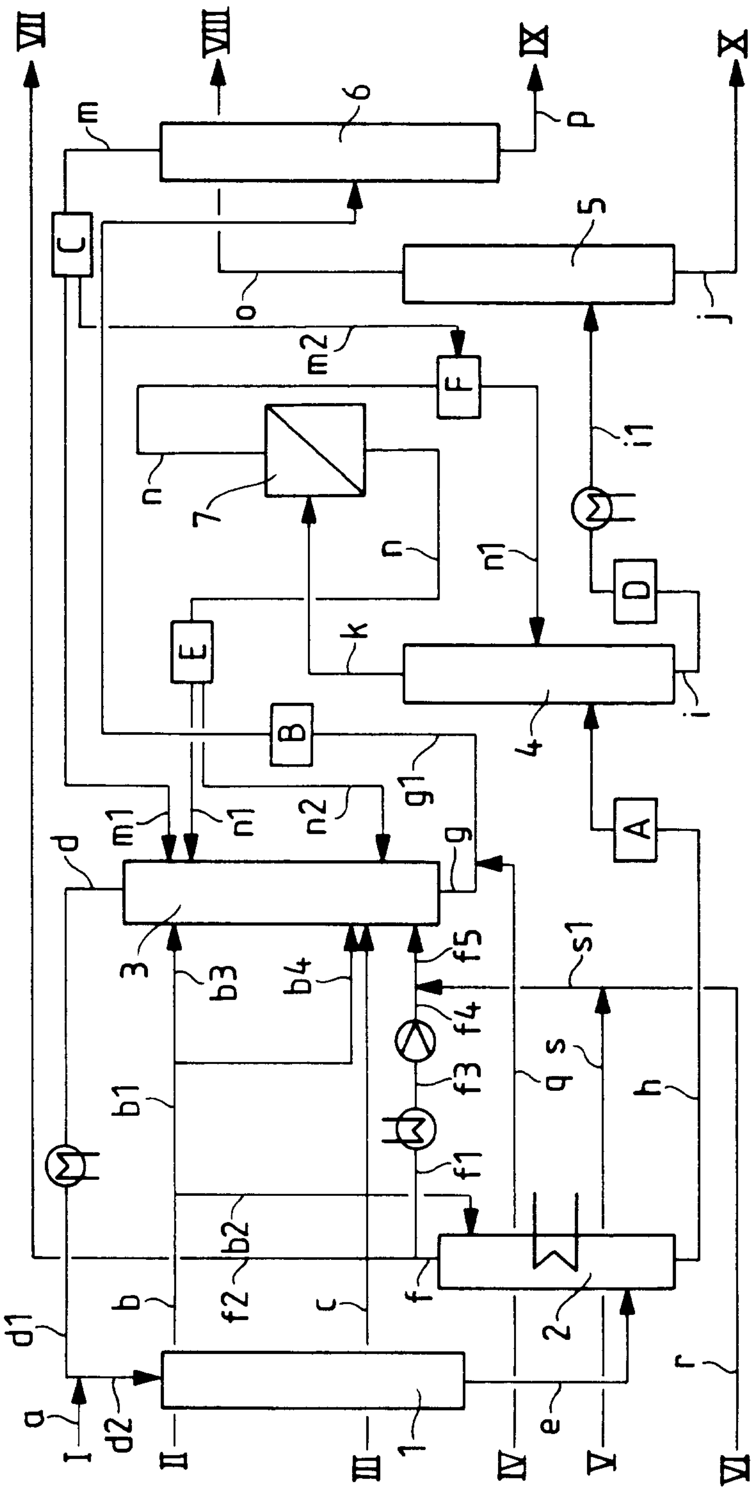
I
II
III
IV
V
VI
VII
VIII
IX
X
A
B
C
D
E
F
1
2
3
4
5
6
7
a
b
b1
b2
b3
b4
c
d
d1
d2
e
f
f1
f2
f3
f4
f5
g
g1
h
i
i1
j
k
m
m1
m2
n
n1
n2
o
p
q
r
s
s1

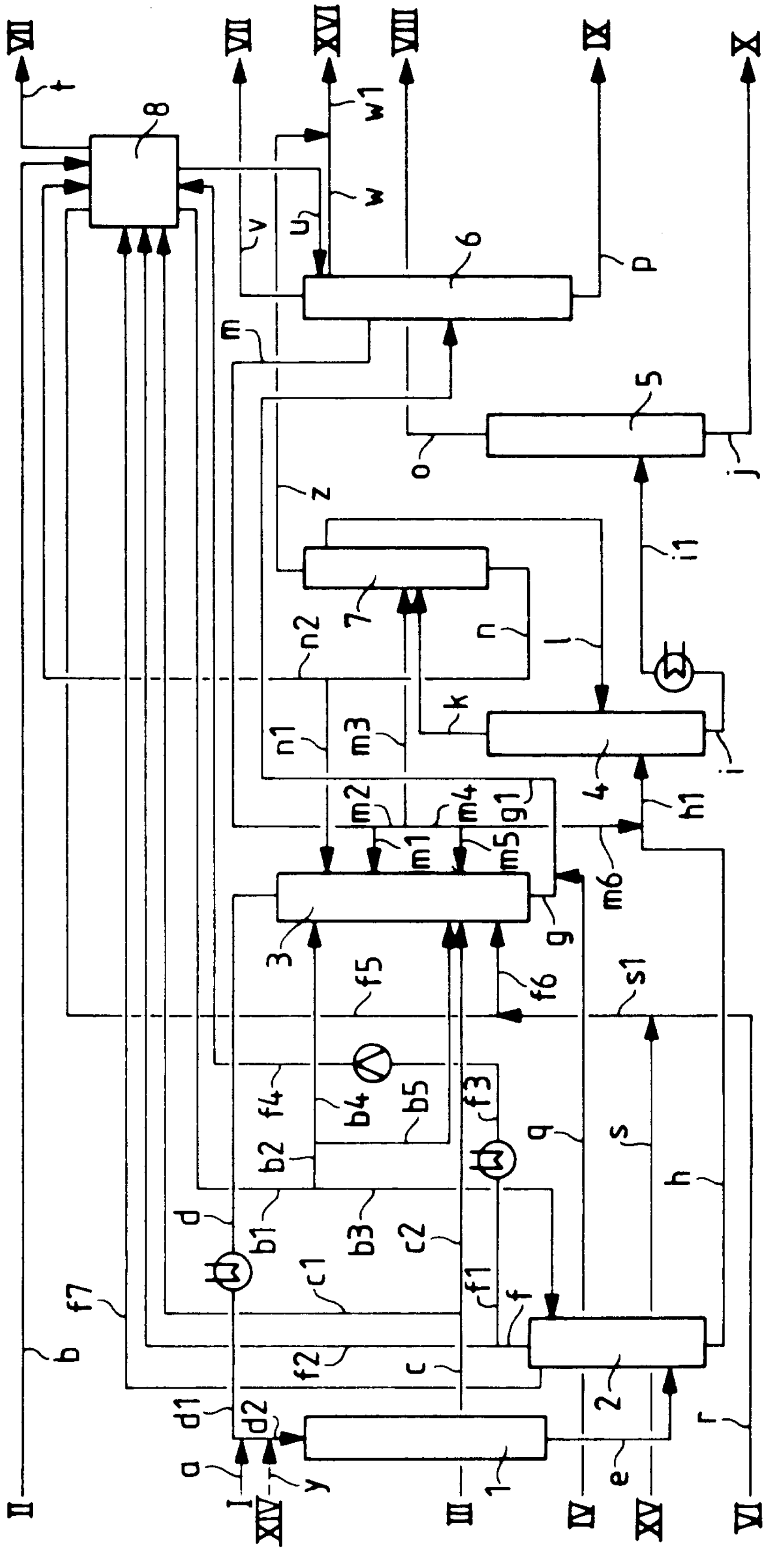

I
II
III
IV
VI
VII
VIII
IX
X
XIV
XV
XVI
1
2
3
4
5
6
7
8
a
b
b1
b2
b3
b4
b5
c
c1
c2
d
d1
d2
e
f
f1
f2
f3
f4
f5
f6
f7
g
g1
h
h1
i
i1
j
k
l
m
m1
m2
m3
m4
m5
m6
n
n1
n2
o
p
q
r
s
s1
t
u
v
w
w1
y
z

Fig.7

Fig. 8

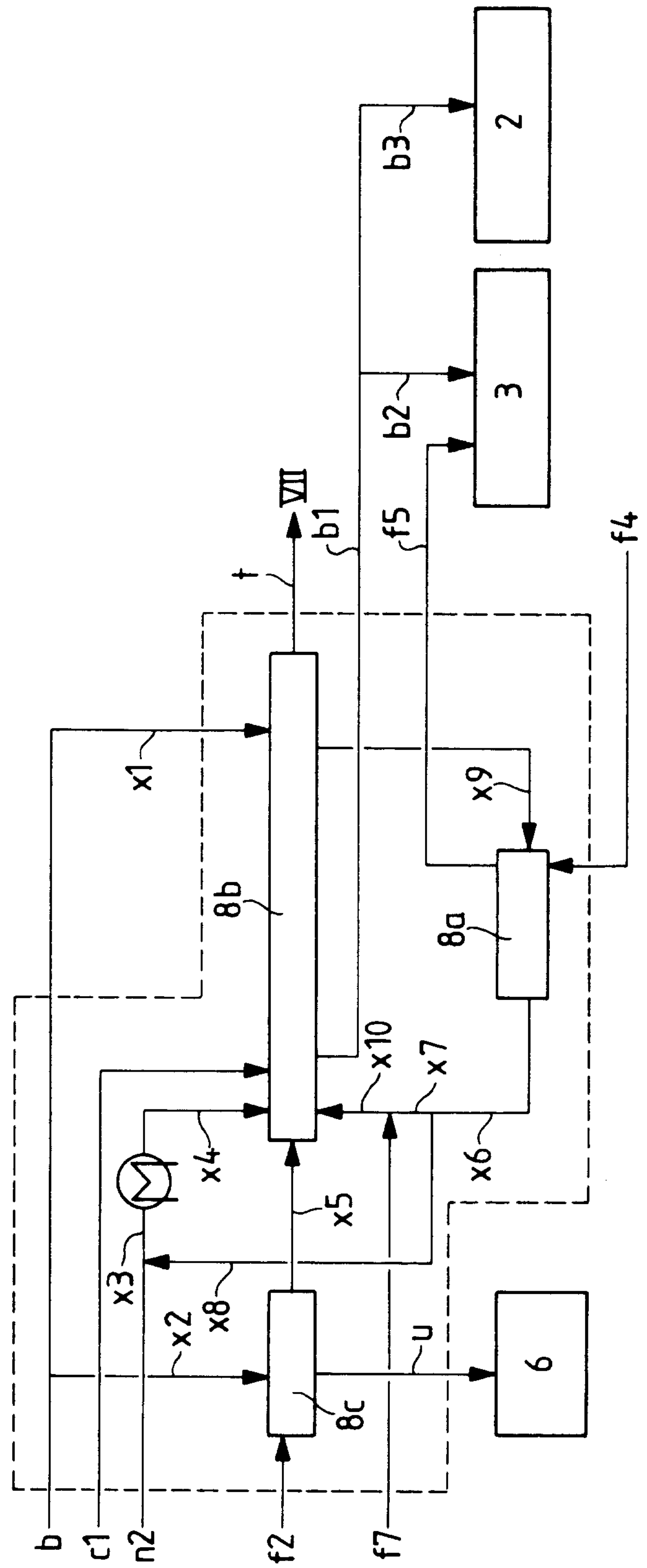
b
c1
n2
f2
f7
x1
x2
x3
x4
x5
x6
x7
x8
x9
x10
8a
8b
8c
t
VII
b1
b2
b3
f4
f5
u
2
3
6

Fig. 9

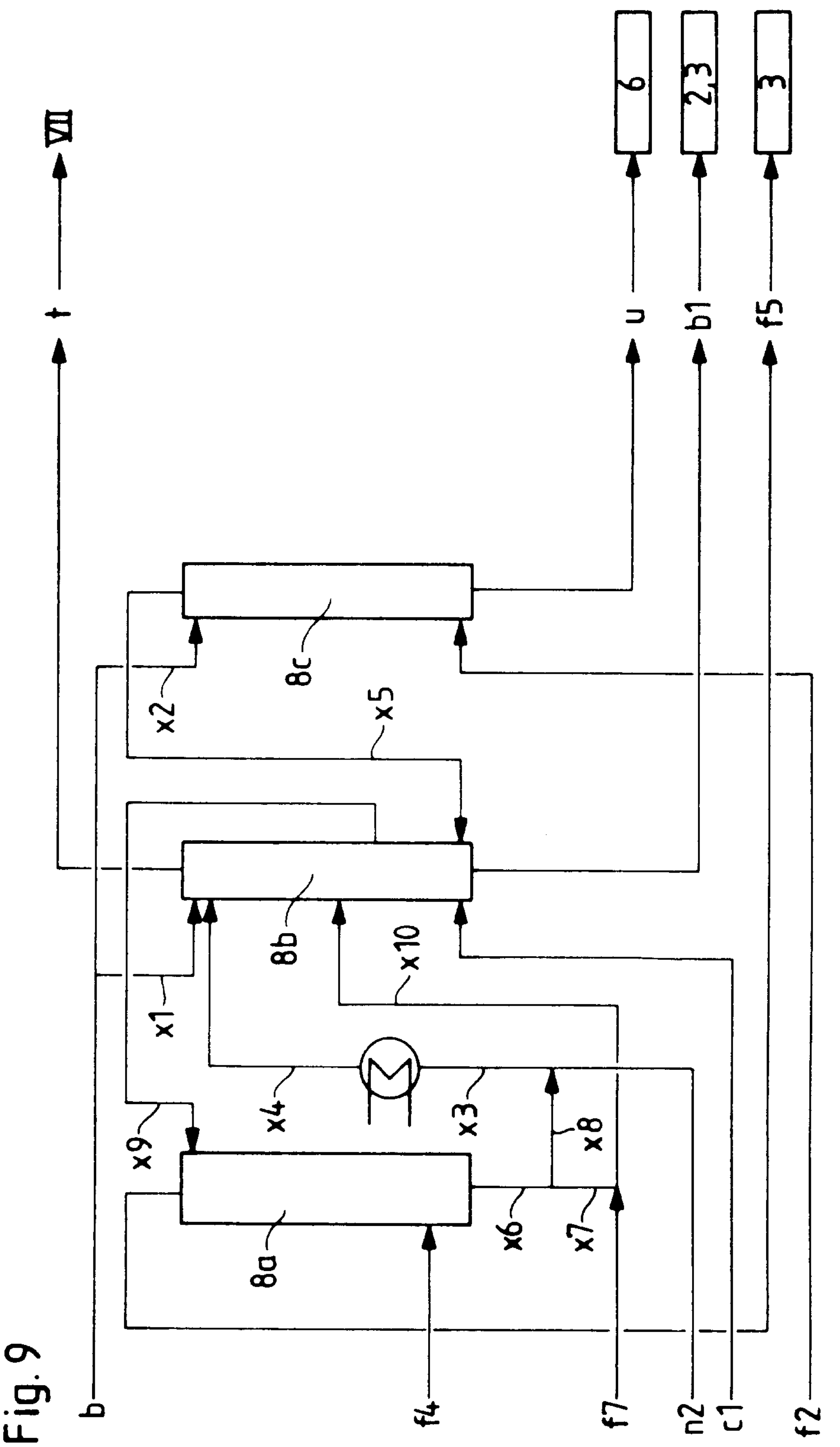

b
f4
f7
n2
c1
f2
8a
8b
8c
x1
x2
x3
x4
x5
x6
x7
x8
x9
x10
t
VII
u
b1
f5
6
2,3
3

Fig.10

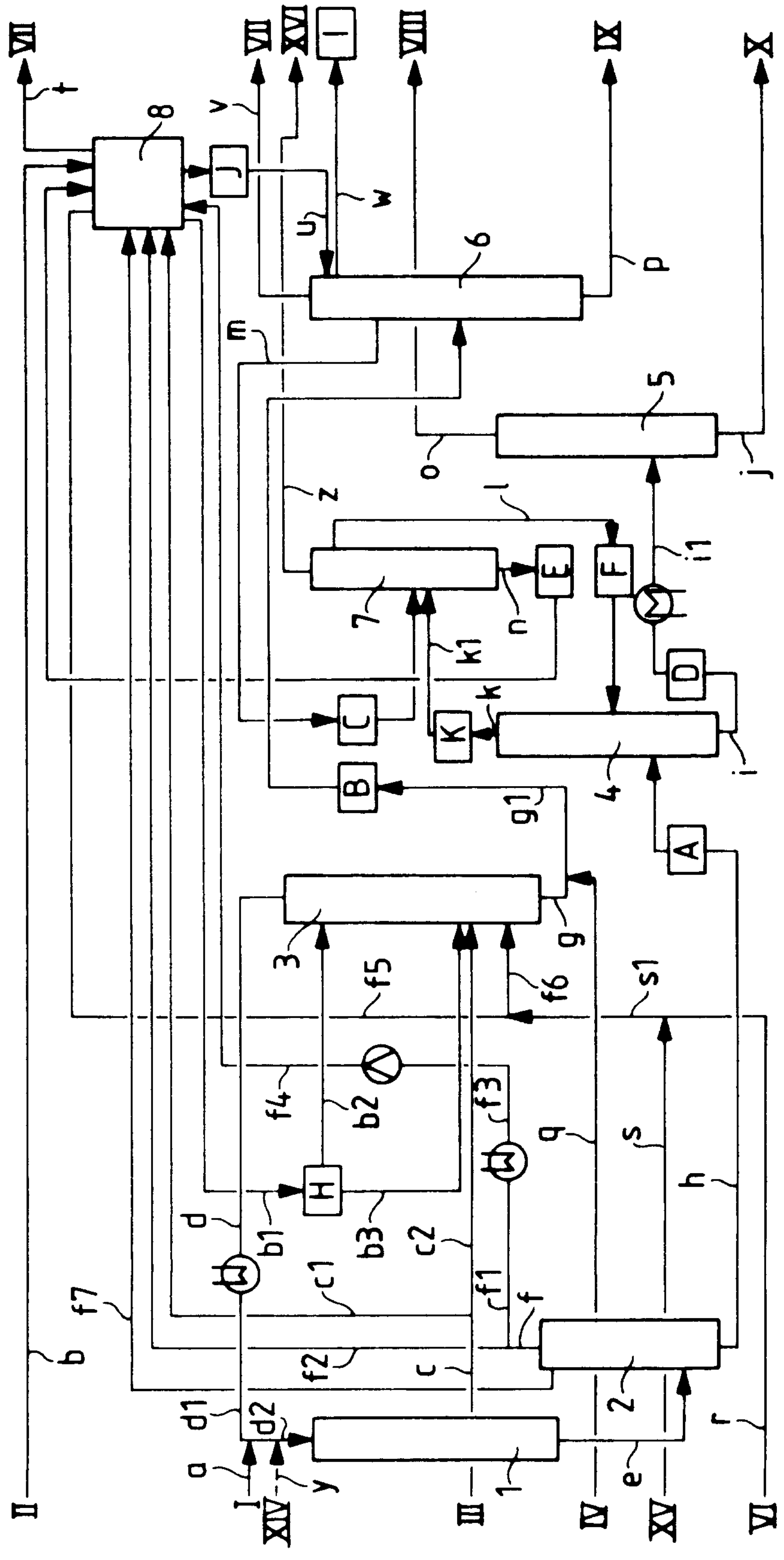
I
II
III
IV
VI
VII
VIII
IX
X
XIV
XV
XVI
1
2
3
4
5
6
7
8
A
B
C
D
E
F
H
J
K
a
b
b1
b2
b3
c
c1
c2
d
d1
d2
e
f
f1
f2
f3
f4
f5
f6
f7
g
g1
h
i
i1
j
k
k1
l
m
n
o
p
q
r
s
s1
t
u
v
w
y
z

Fig.11

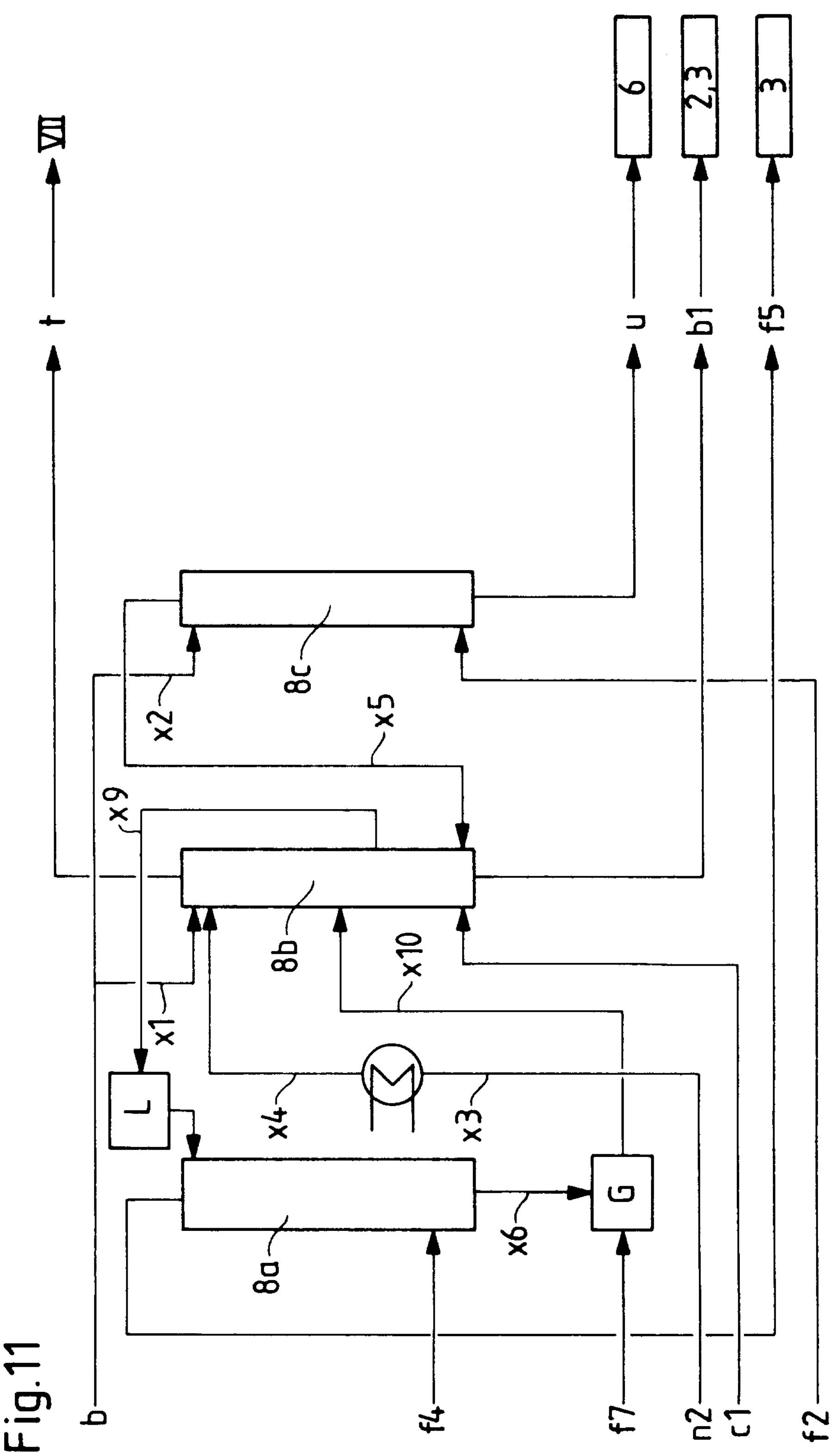

Fig. 12

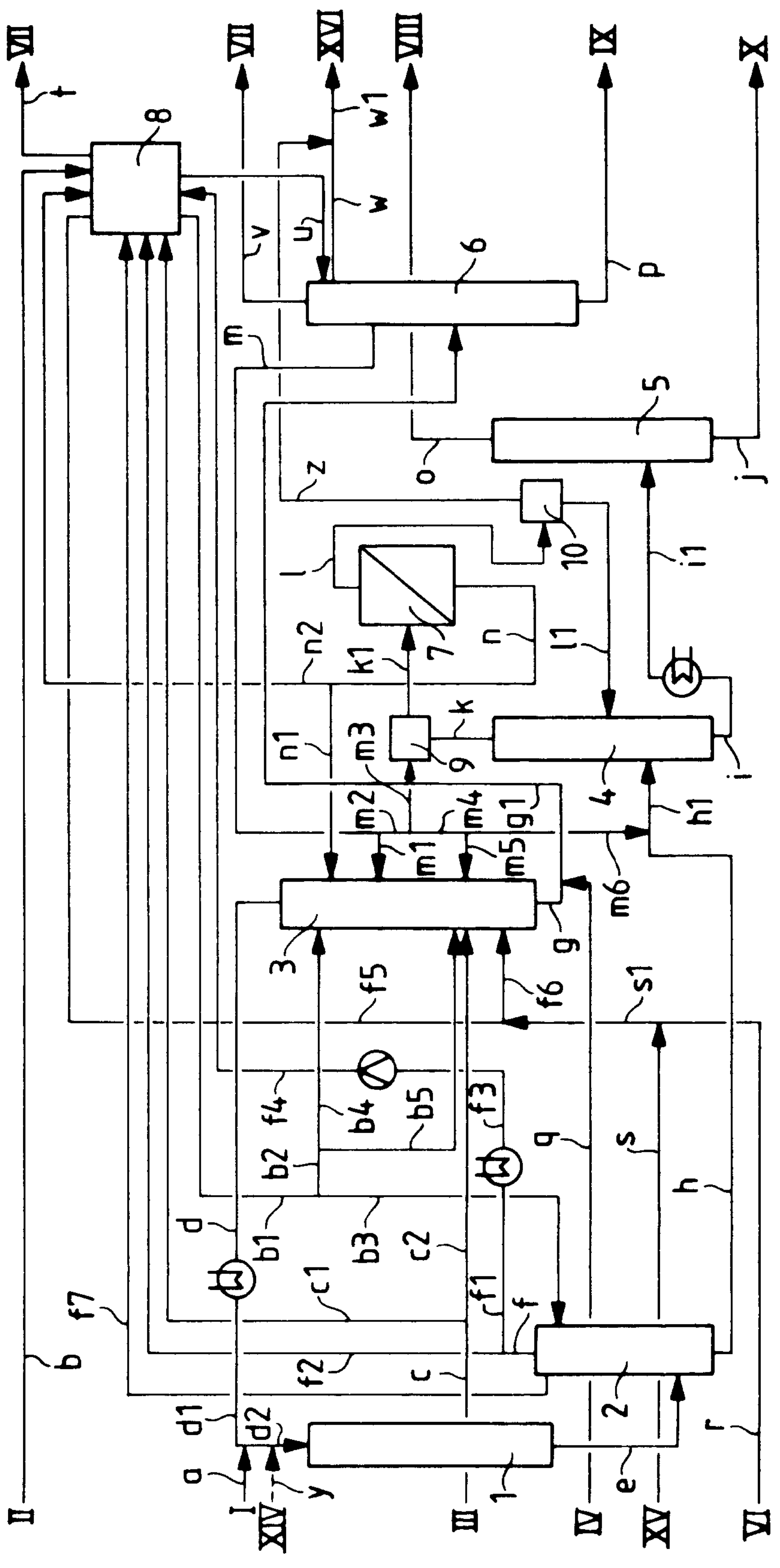
I
II
III
IV
VI
VII
VIII
IX
X
XIV
XV
XVI
1
2
3
4
5
6
7
8
9
10
a
b
c
c1
c2
d
d1
d2
e
f
f1
f2
f3
f4
f5
f6
f7
b1
b2
b3
b4
b5
g
g1
h
h1
i
i1
j
k
k1
l
l1
m
m1
m2
m3
m4
m5
m6
n
n1
n2
o
p
q
r
s
s1
t
u
v
w
w1
y
z

Fig.13

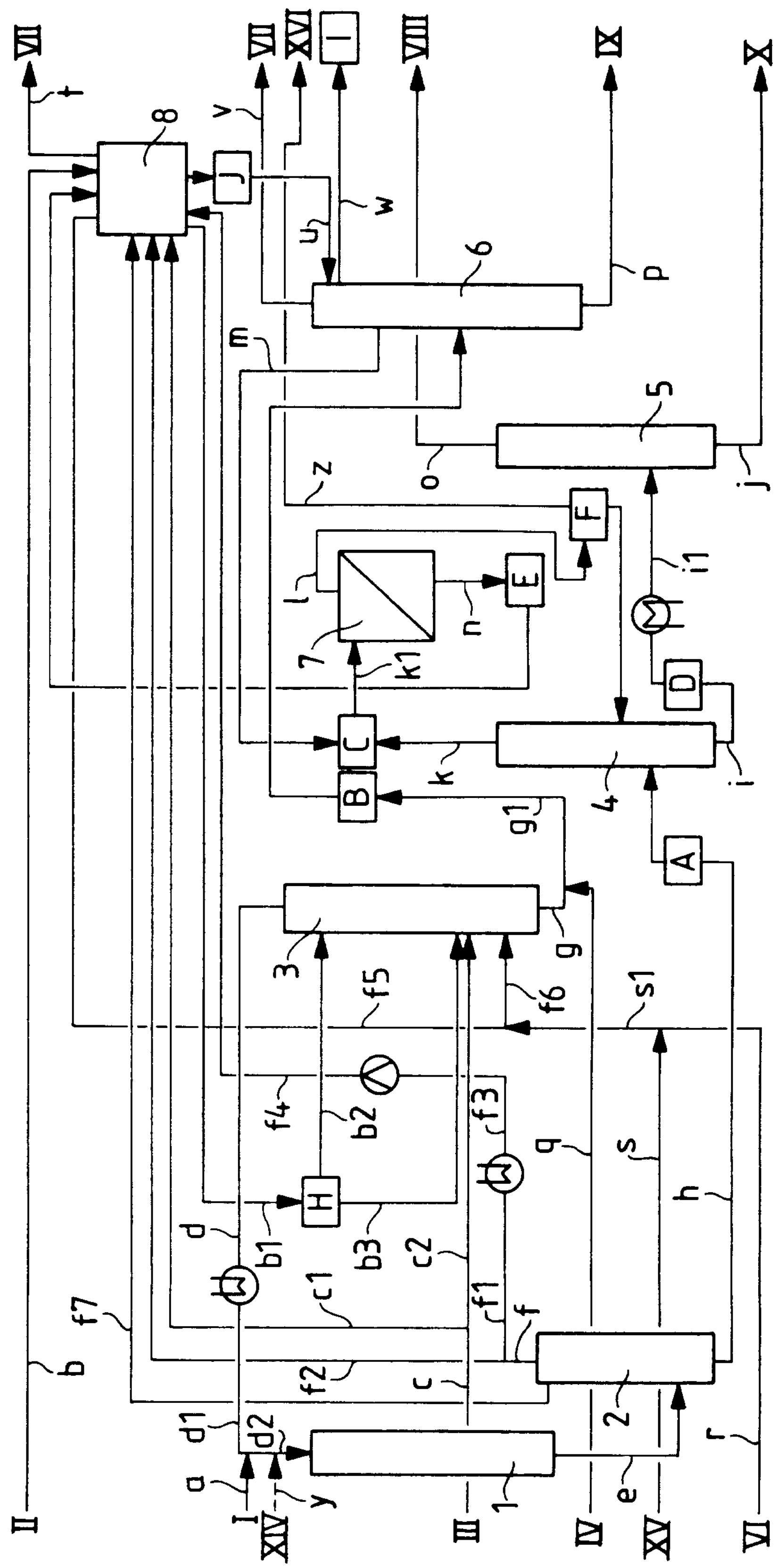

1
2
3
4
5
6
7
8
A
B
C
D
E
F
H
J
I
II
III
IV
VI
VII
VIII
IX
X
XIV
XV
XVI
a
b
b1
b2
b3
c
c1
c2
d
d1
d2
e
f
f1
f2
f3
f4
f5
f6
f7
g
g1
h
i
i1
j
k
k1
l
m
n
o
p
q
r
s
s1
t
u
v
w
y
z